(19) Europäisches Patentamt
European Patent Office
Office européen des brevets

(11) EP 4 722 363 A1

(12) EUROPEAN PATENT APPLICATION
published in accordance with Art. 153(4) EPC

(43) Date of publication:
**08.04.2026 Bulletin 2026/15**

(21) Application number: **24810449.9**

(22) Date of filing: **23.05.2024**

(51) International Patent Classification (IPC):
***C12N 15/113*** (2010.01) ***A61K 47/54*** (2017.01)
***A61P 19/06*** (2006.01) ***A61K 31/713*** (2006.01)
***C07D 211/58*** (2006.01) ***C07C 229/16*** (2006.01)
***C07D 211/28*** (2006.01)

(52) Cooperative Patent Classification (CPC):
**A61K 31/713; A61K 47/54; A61P 19/06; C07C 229/16; C07D 211/28; C07D 211/58; C12N 15/113**

(86) International application number:
**PCT/CN2024/094888**

(87) International publication number:
**WO 2024/240212 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.05.2023 CN 202310592983**

(71) Applicant: **YUN HO BIO CO., LTD**
**Shanghai 201206 (CN)**

(72) Inventors:
- **WU, Yun**
  **Shanghai 201206 (CN)**
- **ZHU, Yun**
  **Shanghai 201206 (CN)**
- **LIU, Yang**
  **Shanghai 201206 (CN)**
- **MAO, Song**
  **Shanghai 201206 (CN)**
- **DONG, Xianglei**
  **Shanghai 201206 (CN)**
- **WANG, Yuhua**
  **Shanghai 201206 (CN)**

(74) Representative: **Simmons & Simmons LLP (Munich)**
**Lehel Carré**
**Gewürzmühlstraße 11**
**80538 Munich (DE)**

Remarks:
The complete document including Reference Table(s) and the Sequence Listing(s) can be downloaded from the EPO website

# (54) RNAI AGENT FOR INHIBITING ANGPTL3 EXPRESSION AND USE THEREOF

(57) An RNAi agent for inhibiting ANGPTL3 gene expression in cells, a pharmaceutical composition thereof, and therapeutic use thereof.

EP 4 722 363 A1

## Description

### Technical Filed

The present invention relates to RNAi agents that inhibit the expression of the ANGPTL3 gene in cells and pharmaceutical compositions comprising the same. The invention also relates to therapeutic uses of the RNAi agents.

### Background

Angiopoietin-like 3 (ANGPTL3) is a member of the angiopoietin-like protein family involved in regulating lipid metabolism. The protein encoded by the ANGPTL3 gene is produced exclusively in the liver and secreted into the circulatory system. In the liver, ANGPTL3 is produced entirely by hepatocytes, and its expression is regulated by the oxysterol-activated Liver X Receptor (LXR). Studies have shown that the biological effects of ANGPTL3 are manifested in two aspects: regulating angiogenesis and lipid metabolism. Its induction effect on angiogenesis is weak, while its regulatory effect on lipid metabolism is more significant, modulating lipid metabolism by inhibiting the activities of lipoprotein lipase (LPL) and endothelial lipase (EL). Dysregulation of lipid metabolism can lead to elevated blood lipids, such as triglycerides or cholesterol levels, which are significant risk factors for metabolic diseases, such as hypertension, cardiovascular diseases, diabetes, and other pathological conditions.

Currently, only one monoclonal antibody drug targeting ANGPTL3, Evinacumab (developed by Regeneron Pharmaceuticals), has been approved for the treatment of patients aged 12 years and older with homozygous familial hypercholesterolemia (HoFH). There are no siRNA drugs targeting the same gene approved for marketing worldwide.

### Summary

One aspect of the present invention provides an RNAi agent comprising a sense strand and an antisense strand that form a duplex region, wherein the RNAi agent inhibits the expression of ANGPTL3 in a cell, and wherein the antisense strand is no more than 23 nucleotides in length and comprises a nucleotide sequence selected from any one of SEQ ID NOs: 13, 14, 27, and 28.

In some embodiments, the sense strand is no more than 21 nucleotides in length and comprises a nucleotide sequence selected from any one of SEQ ID NOs: 5, 7, 8, 19, 20, and 22.

In some embodiments, the RNAi agent provided by the present invention for inhibiting the expression of ANGPTL3 in a cell comprises a sense strand and an antisense strand that form a duplex region, the antisense strand is no more than 23 nucleotides in length, and the sense strand is no more than 21 nucleotides in length, wherein:

the sense strand comprises or consists of a sequence set forth in SEQ ID NO: 5, and the antisense strand comprises or consists of a sequence set forth in SEQ ID NO: 13;
the sense strand comprises or consists of a sequence set forth in SEQ ID NO: 5, and the antisense strand comprises or consists of a sequence set forth in SEQ ID NO: 14;
the sense strand comprises or consists of a sequence set forth in SEQ ID NO: 7, and the antisense strand comprises or consists of a sequence set forth in SEQ ID NO: 13;
the sense strand comprises or consists of a sequence set forth in SEQ ID NO: 8, and the antisense strand comprises or consists of a sequence set forth in SEQ ID NO: 13;
the sense strand comprises or consists of a sequence set forth in SEQ ID NO: 19, and the antisense strand comprises or consists of a sequence set forth in SEQ ID NO: 27;
the sense strand comprises or consists of a sequence set forth in SEQ ID NO: 19, and the antisense strand comprises or consists of a sequence set forth in SEQ ID NO: 28;
the sense strand comprises or consists of a sequence set forth in SEQ ID NO: 20, and the antisense strand comprises or consists of a sequence set forth in SEQ ID NO: 27;
the sense strand comprises or consists of a sequence set forth in SEQ ID NO: 20, and the antisense strand comprises or consists of a sequence set forth in SEQ ID NO: 28;
the sense strand comprises or consists of a sequence set forth in SEQ ID NO: 22, and the antisense strand comprises or consists of a sequence set forth in SEQ ID NO: 28;
the sense strand comprises or consists of a sequence set forth in SEQ ID NO: 5, and the antisense strand comprises or consists of a sequence set forth in SEQ ID NO: 18; or
the sense strand comprises or consists of a sequence set forth in SEQ ID NO: 19, and the antisense strand comprises or consists of a sequence set forth in SEQ ID NO: 32.

In some embodiments, the RNAi agent further comprises a ligand that targets a hepatocyte; preferably, the ligand

comprises a galactose moiety, a galactosamine moiety, or an N-acetylgalactosamine moiety; more preferably, the ligand is a trivalent or tetravalent N-acetylgalactosamine moiety.

In some embodiments, the ligand comprises a structure of formula (I):

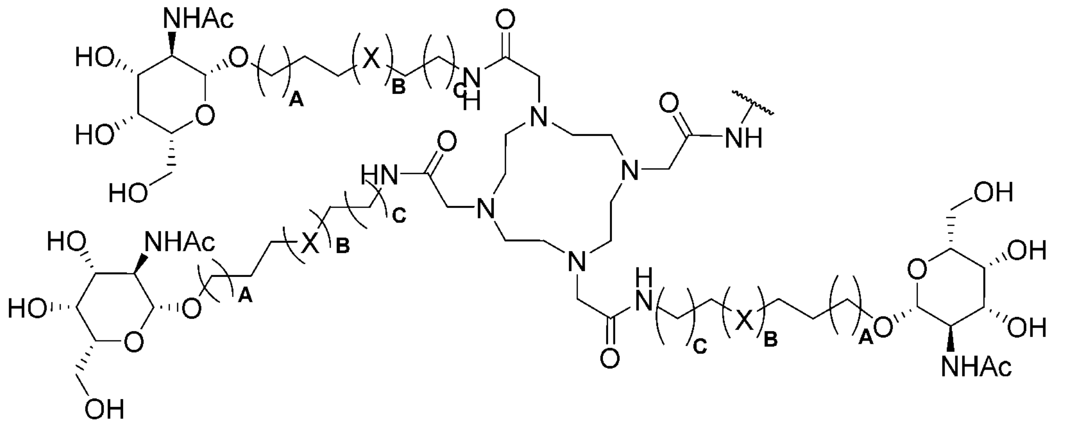

(I)

wherein,

X is -C(O)-NH-, -NH-C(O)-, -OCH2-CH2O-, -S-, or -S-S-;
A or C is independently 0 or an integer from 1 to 5;
B is 0 or an integer from 1 to 3;
the wave line represents a site to which other part of the RNAi agent is attached, wherein the ligand is connected to 5' and/or 3'-end of the sense strand and/or antisense strand; preferably, the ligand is connected to 5' and/or 3'-end of the sense strand; more preferably, the ligand is connected to 3'-end of the sense strand.

In some embodiments, the ligand has a structure of formula (II) or formula (III):

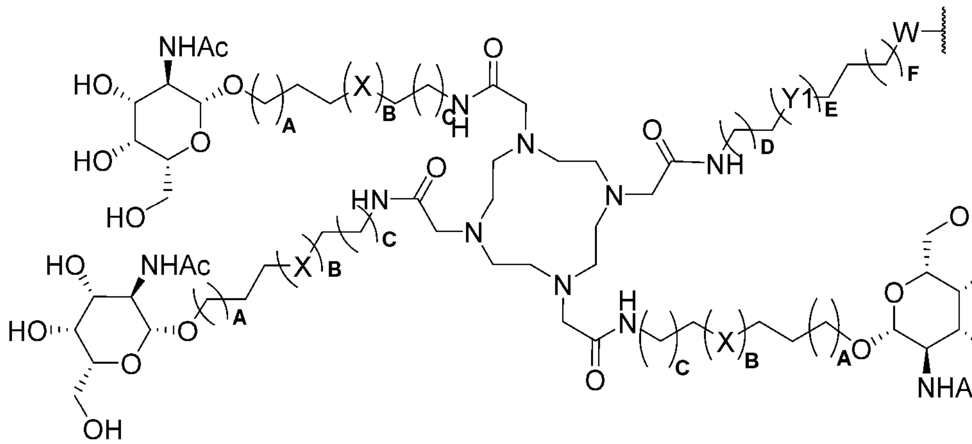

(II)

wherein,

X is -C(O)-NH-, -NH-C(O)-, -OCH2-CH2O-, -S-, or -S-S-;
Y1 is -C(O)-$NR_1$-, -NH-C(O)-, -$OCH_2$-$CH_2O$-, -S-S-,

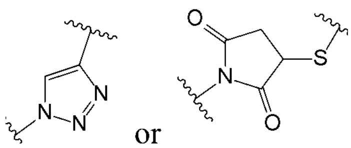

or ,

wherein $R_1$ is an alkyl group having 1-6 carbons or hydrogen;
W is -NH-, -O- or

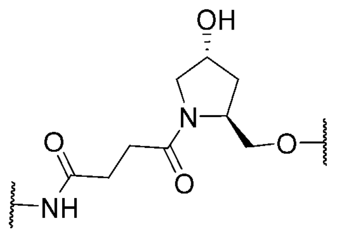

,

wherein the wave line on the right represents a site to which the sense strand or antisense strand is attached; the wave line connected to W in formula (II) represents a site to which the sense strand or antisense strand is attached; A, C, or F is 0 or an integer from 1 to 5; B or E is 0 or an integer from 1 to 3; D is an integer from 1 to 6;

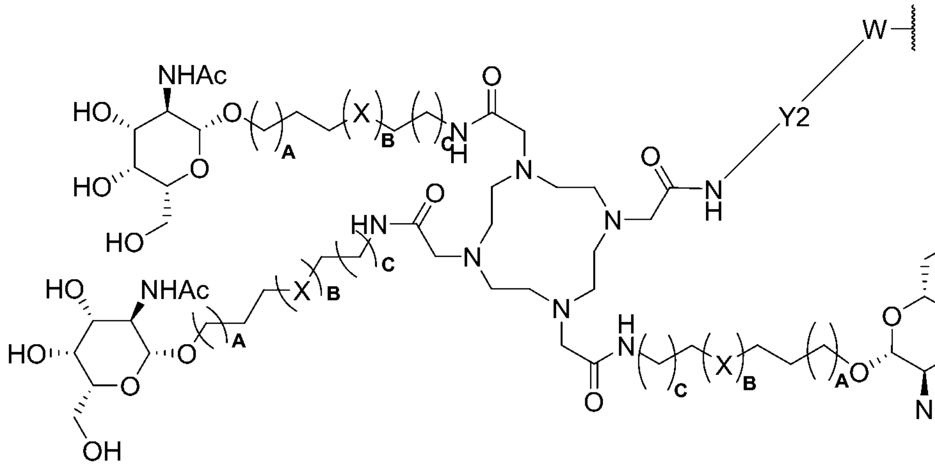

(III)

wherein,
X is -C(O)-NH-, -NH-C(O)-, $-OCH_2-CH_2O-$, -S-, or -S-S-;
Y2 is $-(CH_2)p-(O-CH_2-CH_2)q-(CH_2)j-Z_3-$, wherein $Z_3$ is O, NH or C(O), p is an integer from 1 to 3, q is an integer from 3 to 10; j is 0 or 1;
W is absent, or is

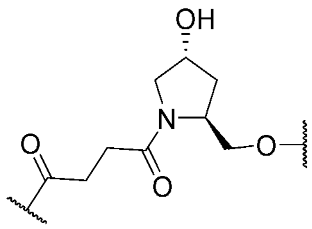

or

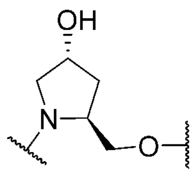

,

wherein the wave line on the right represents a site to which the sense strand or antisense strand is attached; the wave line connected to W in formula (III) represents a site to which the sense strand or antisense strand is attached; A or C is 0 or an integer from 1 to 5; B is 0 or an integer from 1 to 3.

In some embodiments, the ligand has a structure of formula (IV) or formula (V):

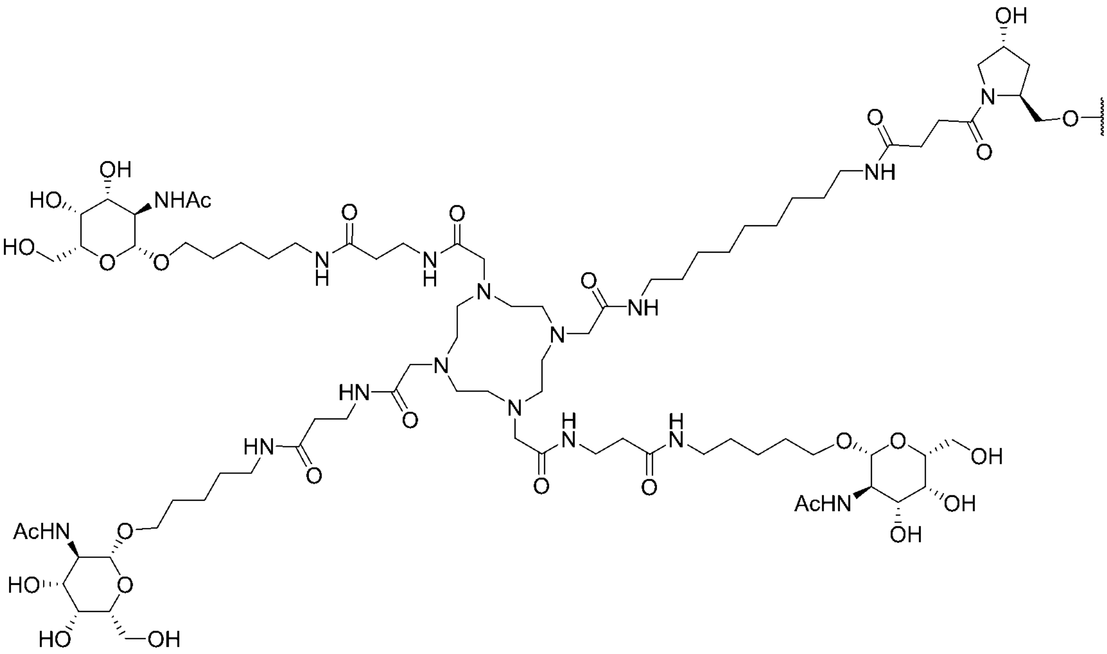

(IV)

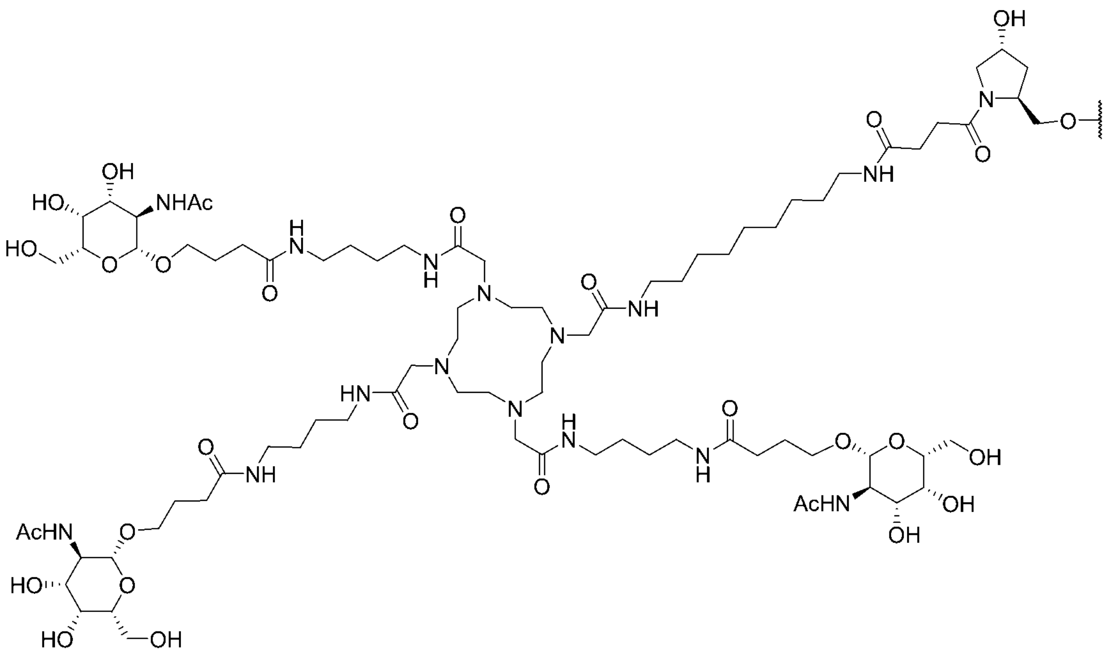

(V)

the wave line represents a site to which the sense strand or antisense strand is attached, wherein the ligand is connected to 5' and/or 3'-end of the sense strand; preferably, the ligand is connected to 3'-end of the sense strand; wherein the targeting ligand is connected to the sense strand or antisense strand via a phosphate ester group, a thiophosphate ester group or a phosphonate ester group.

In some embodiments, the RNAi agent comprises a sense strand and an antisense strand that form a duplex region, the antisense strand is no more than 23 nucleotides in length, and the sense strand is no more than 21 nucleotides in length, wherein:

the sense strand comprises or consists of a sequence set forth in SEQ ID NO: 33, and the antisense strand comprises or consists of a sequence set forth in SEQ ID NO: 13;

the sense strand comprises or consists of a sequence set forth in SEQ ID NO: 34, and the antisense strand comprises or consists of a sequence set forth in SEQ ID NO: 13;

the sense strand comprises or consists of a sequence set forth in SEQ ID NO: 38, and the antisense strand comprises or consists of a sequence set forth in SEQ ID NO: 13;

the sense strand comprises or consists of a sequence set forth in SEQ ID NO: 39, and the antisense strand comprises or consists of a sequence set forth in SEQ ID NO: 13;
the sense strand comprises or consists of a sequence set forth in SEQ ID NO: 35, and the antisense strand comprises or consists of a sequence set forth in SEQ ID NO: 27;
the sense strand comprises or consists of a sequence set forth in SEQ ID NO: 36, and the antisense strand comprises or consists of a sequence set forth in SEQ ID NO: 27;
the sense strand comprises or consists of a sequence set forth in SEQ ID NO: 40, and the antisense strand comprises or consists of a sequence set forth in SEQ ID NO: 27;
the sense strand comprises or consists of a sequence set forth in SEQ ID NO: 41, and the antisense strand comprises or consists of a sequence set forth in SEQ ID NO: 27;
the sense strand comprises or consists of a sequence set forth in SEQ ID NO: 35, and the antisense strand comprises or consists of a sequence set forth in SEQ ID NO: 28;
the sense strand comprises or consists of a sequence set forth in SEQ ID NO: 36, and the antisense strand comprises or consists of a sequence set forth in SEQ ID NO: 28;
the sense strand comprises or consists of a sequence set forth in SEQ ID NO: 37, and the antisense strand comprises or consists of a sequence set forth in SEQ ID NO: 28;
the sense strand comprises or consists of a sequence set forth in SEQ ID NO: 40, and the antisense strand comprises or consists of a sequence set forth in SEQ ID NO: 28;
the sense strand comprises or consists of a sequence set forth in SEQ ID NO: 41, and the antisense strand comprises or consists of a sequence set forth in SEQ ID NO: 28; or
the sense strand comprises or consists of a sequence set forth in SEQ ID NO: 42, and the antisense strand comprises or consists of a sequence set forth in SEQ ID NO: 28.

In some particularly preferred embodiments, the present invention provides a double-stranded RNAi agent for inhibiting the expression of ANGPTL3 in a cell, wherein the double-stranded RNAi agent comprises a sense strand and an antisense strand that form a duplex region, wherein the sense strand comprises SEQ ID NO: 19: 5'-mG*mA*mGmAfAmGfAfAfCmUmAmCmAmUmAmUmAmAmA-3' and the sense strand is no more than 21 nucleotides in length, the antisense strand comprises SEQ ID NO: 27: 5'-mU*fU*mUmAfUmAfUnGmUmAmGmUmUfCmUfUmCmUmC*-mA*mG-3' and is no more than 23 nucleotides in length,

wherein mA, mU, mG, and mC are 2'-OMe A, 2'-OMe U, 2'-OMe G, and 2'-OMe C, respectively; fA, fU, fG, and fC are 2'-F A, 2'-F U, 2'-F G, and 2'-F C, respectively; * is a phosphorothioate linkage;
wherein 3'-end of the sense strand is conjugated to a ligand that targets a hepatocyte; preferably, the ligand comprises a galactose moiety, a galactosamine moiety, or an N-acetylgalactosamine moiety; more preferably, the ligand is a trivalent or tetravalent N-acetylgalactosamine moiety.

In these particularly preferred embodiments, the ligand preferably has a structure of formula (IV):

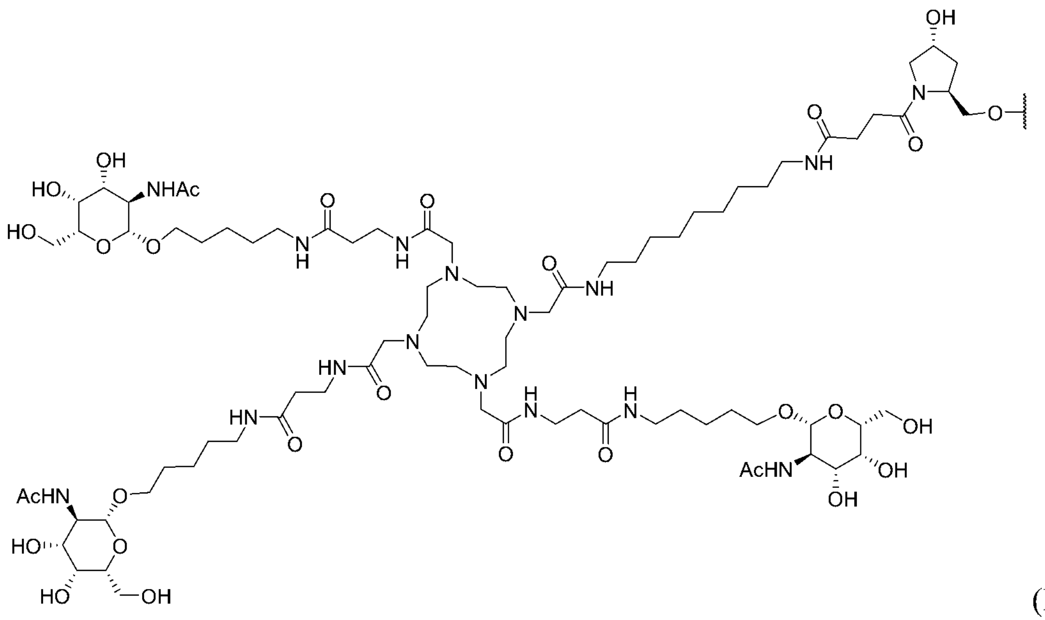

(IV),

and is connected to the sense strand via a phosphate ester group or a thiophosphate ester group.

In these particularly preferred embodiments, more preferably, the sense strand comprises SEQ ID NO: 40: 5'-mG*mA*mGmAfAmGfAfAfCmUmAmCmAmUmAmUmAmAmA-YHZY12001-3' and the sense strand is no more than 21 nucleotides in length, the antisense strand comprises SEQ ID NO: 27: 5'-mU*fU*mUmAfUmAfUmGmUmAmGmUmUfCmUfUmCmUmC*mA*mG-3' and is no more than 23 nucleotides in length,

wherein mA, mU, mG, and mC are 2'-OMe A, 2'-OMe U, 2'-OMe G, and 2'-OMe C, respectively; fA, fU, fG, and fC are 2'-F A, 2'-F U, 2'-F G, and 2'-F C, respectively; * is a phosphorothioate linkage,

wherein YHZY12001 has a structure of formula (IV) and is conjugated to 3'-end of the sense strand as follows:

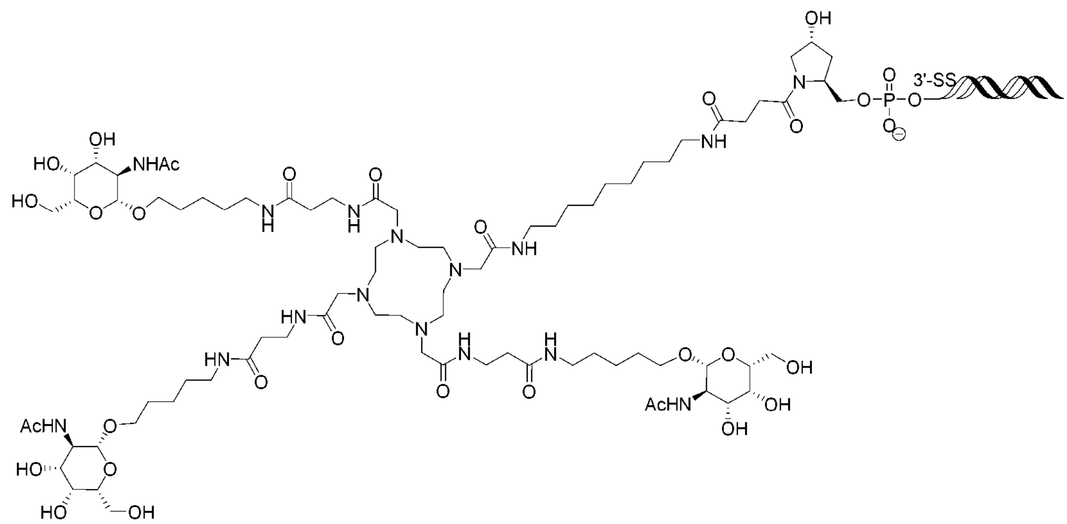

.

A further aspect of the present invention provides a pharmaceutical composition comprising any RNAi agent of the present invention and a pharmaceutically acceptable carrier; preferably, the pharmaceutical composition is formulated for intravenous or subcutaneous injection.

Another aspect of the present invention provides the use of any RNAi agent of the present invention in the manufacture of a medicament for:

(i) reducing the expression level of ANGPTL3 in a cell;
(ii) preventing or treating a disease caused by an elevated ANGPTL3 expression level; or
(iii) preventing or treating a metabolic disease, wherein the metabolic disease is, for example, hypertriglyceridemia, obesity, hyperlipidemia, abnormal lipid and/or cholesterol metabolism, atherosclerosis, Type 2 diabetes mellitus, cardiovascular disease, coronary artery disease, non-alcoholic steatohepatitis, non-alcoholic fatty liver disease, homozygous or heterozygous familial hypercholesterolemia, statin-resistant hypercholesterolemia.

Accordingly, the present invention also provides a method for reducing the expression level of ANGPTL3 in a cell, the method comprising administering a therapeutically effective amount of any RNAi agent of the present invention to a subject in need thereof. The present invention also provides a method for preventing or treating a disease caused by elevated ANGPTL3 expression level, the method comprising administering a therapeutically effective amount of any RNAi agent of the present invention to a subject in need thereof. The present invention also provides a method for preventing or treating a metabolic disease, the method comprising administering a therapeutically effective amount of any RNAi agent of the present invention to a subject in need thereof, wherein the metabolic disease is, for example, one or more of hypertriglyceridemia, obesity, hyperlipidemia, abnormal lipid and/or cholesterol metabolism, atherosclerosis, Type 2 diabetes mellitus, cardiovascular disease, coronary artery disease, non-alcoholic steatohepatitis, non-alcoholic fatty liver disease, homozygous or heterozygous familial hypercholesterolemia, statin-resistant hypercholesterolemia.

Yet another aspect of the present invention provides an RNAi agent comprising a sense strand and an antisense strand that form a duplex region, wherein in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and

in the 5' to 3' direction, the nucleotides at positions 7 and 9 of the sense strand are 2'-fluoro-modified nucleotides, one or two of the nucleotides at positions 5, 8, and 11 of the sense strand are 2'-fluoro-modified nucleotides, while the nucleotides

at remaining positions of the sense strand are all 2'-methoxy-modified nucleotides.

In some embodiments, the antisense strand of the RNAi agent of the present invention comprises at least one phosphorothioate internucleotide linkage; preferably, the phosphorothioate internucleotide linkage is present at one or more of the following locations: between nucleotide positions 1 and 2 at 5'-end of the antisense strand; between nucleotide positions 2 and 3 at 5'-end of the antisense strand; between nucleotide positions 1 and 2 at 3'-end of the antisense strand; and between nucleotide positions 2 and 3 at 3'-end of the antisense strand.

In some embodiments, the sense strand comprises at least one phosphorothioate internucleotide linkage; preferably, the phosphorothioate internucleotide linkage is present (i) between nucleotide positions 1 and 2 at 5'-end of the sense strand; and/or (ii) between nucleotide positions 2 and 3 at 5'-end of the sense strand.

In some embodiments, the duplex region is 17 to 23 base pairs in length, preferably 18 to 21 base pairs, more preferably 19 base pairs.

In some embodiments, each of the sense strand and the antisense strand is 17 to 23 nucleotides in length, preferably 19 to 21 nucleotides.

In some embodiments, the RNAi agent comprises one or two blunt ends, preferably one blunt end.

In some embodiments, the RNAi agent comprises one or two overhangs, preferably one overhang, each overhang comprising 1 to 4 unpaired nucleotides, preferably 2 unpaired nucleotides.

In some embodiments, the overhang is located at 3'-end of the sense strand, 3'-end of the antisense strand, or simultaneously at 3'-end of the sense strand and 3'-end of the antisense strand; preferably, the overhang is located at 3'-end of the antisense strand, and more preferably, the RNAi agent has one blunt end.

Other aspects of the invention will be apparent from the detailed description of the specification that follows.

## Brief Description of the Figures

Fig. 1 depicts the levels of ANGPTL3 protein, triglyceride, and total cholesterol in the plasma of hANGPTL3 mice administered with the RNAi agent of the present invention, as a function of time.

Fig. 2 shows that the serum ANGPTL3 protein level and triglyceride level were significantly reduced in aged cynomolgus monkey with hyperlipidemia administered with the RNAi agent of the present invention.

## Detailed Description of the Invention

Definition

As used herein, "ANGPTL3" refers to angiopoietin-like 3 protein or its encoding gene. As used herein, "ANGPTL3 mRNA" refers to ANGPTL3 messenger RNA, including but not limited to allelic variants and splice variants. The NCBI Reference Sequence number for human ANGPTL3 mRNA is NM _014495.4."

As used herein, the term "RNAi agent" refers to a reagent comprising an RNA molecule that, when introduced into a cell, is capable of downregulating the expression of a target gene (in this case, the ANGPTL3 gene) through an RNA interference mechanism. The terms "RNAi agent of the present invention," "RNAi agent described herein," or similar expressions encompass both chemically modified RNAi agents of the present invention, irrespective of sequence and target gene, and RNAi agents of the present invention with specific sequences used for interfering with the ANGPTL3 gene. RNA interference refers to a process whereby a nucleic acid molecule induces cleavage and degradation of a target RNA molecule (e.g., an mRNA molecule) in a sequence-specific manner, such as through the RNA-induced silencing complex (RISC) pathway. RNAi agents herein include siRNA, shRNA, and DNA/RNA hybrid molecules, also sometimes collectively referred to as double-stranded RNA (dsRNA), which comprise two antiparallel continuous nucleotide strands sufficiently complementary to hybridize and form a double-stranded region. "Hybridization" refers to the pairing of complementary polynucleotides, typically through hydrogen bonding between complementary bases in the two polynucleotides (e.g., Watson-Crick hydrogen bonding, Wobble hydrogen bonding, Hoogsteen hydrogen bonding, or reverse Hoogsteen hydrogen bonding). The "double-stranded region" refers to an area within two complementary or substantially complementary polynucleotides that form base pairs through hybridization, thereby creating a duplex between the two polynucleotide strands.

The term "antisense strand" refers to the strand in a dsRNA that contains a region substantially complementary to a target sequence. The term "sense strand" refers to the strand in a dsRNA that contains a region substantially complementary to the antisense strand region as defined herein. The term "region substantially complementary" denotes a region that is either perfectly complementary or imperfectly complementary. When the complementary region is imperfectly complementary to the target sequence, mismatches may occur in internal or terminal regions of the molecule. Typically, the most tolerated mismatches are located in terminal regions, for example, within 5, 4, 3, or 2 nucleotides at 5'

and/or 3' termini of the dsRNA.

"siRNA" refers to a nucleic acid that forms a double-stranded RNA capable of reducing or inhibiting the expression of a target gene when present in the same cell as the target gene. An siRNA is typically approximately 15 to approximately 30 base pairs in length, most typically approximately 19 to 25 base pairs in length, for example, 19, 20, 21, 22, 23, 24, or 25 nucleotide pairs in length.

"shRNA" refers to short hairpin RNA, which comprises two short inverted repeats and an intervening stem-loop structure connecting them. The stem-loop may comprise at least one unpaired nucleotide, for example, at least 2, at least 3, at least 4, at least 5, at least 6, at least 7, at least 8, at least 9, at least 10, at least 20, at least 23, or more unpaired nucleotides. The stem-loop may be 10 or fewer nucleotides in length. The stem-loop may comprise 8 or fewer unpaired nucleotides. The stem-loop may comprise 4 to 10 unpaired nucleotides. The stem-loop may be 4 to 8 nucleotides in length.

The two substantially complementary strands of a dsRNA need not be, but may be, covalently linked. The maximum number of base pairs is the number of nucleotides in the shortest strand of the dsRNA minus any overhangs present in the duplex. In addition to the duplex structure, a dsRNA may comprise one or more nucleotide overhangs. An overhanging nucleotide refers to one or more unpaired nucleotides extending beyond the double-stranded region at the terminus of a strand. Nucleotide overhangs typically occur when the 3' end of one strand extends beyond the 5' end of the other strand, or when the 5' end of one strand extends beyond the 3' end of the other strand. For example, at least one strand comprises a 3' overhang of at least 1 nucleotide, e.g., 1 to 4 nucleotide overhangs. In another example, at least one strand comprises a 5' overhang of at least 1 nucleotide, e.g., 1 to 4 nucleotide overhangs. In other embodiments, both the 3' terminus and the 5' terminus of one strand of the dsRNA comprise an overhang of at least 1 nucleotide.

As used herein, the terms "blunt end" or "blunt terminus" with respect to dsRNA refer to the absence of unpaired nucleotides or nucleotide analogs at a given terminus of the dsRNA, i.e., no nucleotide overhang. One end or both ends of the dsRNA may be blunt. Where both ends of the dsRNA are blunt, the dsRNA is referred to as blunt-ended. It is to be expressly understood that a "blunt-ended" dsRNA is one having blunt termini at both ends, meaning no nucleotide overhang exists at either end of the molecule. In most cases, such molecules are double-stranded throughout their entire length. As used herein, the term "nucleotide overhang" refers to at least one unpaired nucleotide projecting from the duplex structure of the dsRNA. For example, a nucleotide overhang exists when the 3' terminus of one strand of the dsRNA extends beyond the 5' terminus of the other strand, or vice versa. The overhang may comprise or consist of nucleotides/nucleosides analogs, including deoxyribonucleotides/deoxyribonucleosides. Overhangs may be present on the sense strand, antisense strand, or any combination thereof. Furthermore, the overhanging nucleotides may reside at the 5' terminus, 3' terminus, or both termini of the antisense strand or sense strand of the dsRNA.

The dsRNA molecule may comprise chemical modifications to ribonucleotides, including modifications to the ribose, base, or backbone components of ribonucleic acid, as described herein or known in the art. Any such modifications, when used in double-stranded ribonucleic acid molecules (e.g., siRNA, shRNA, etc.), are encompassed by the term "dsRNA" for purposes of this disclosure. A "modified" nucleotide refers to a nucleotide that independently has a modified sugar moiety, a modified internucleoside linkage, and/or a modified nucleobase. Accordingly, the term "modified nucleotide" includes substitutions, additions, or removals of functional groups or atoms to the internucleoside linkage, sugar moiety, or nucleobase. Modifications suitable for the present invention include all types of modifications disclosed herein or known in the art. For example, the modified nucleotide is selected from the group consisting of: 2'-deoxythymidine (dT) nucleotide, 2'-O-methyl-modified nucleotide, 2'-fluoro-modified nucleotide, 2'-deoxy-modified nucleotide, unlocked nucleotide, conformationally constrained nucleotide, constrained ethyl nucleotide, 2'-amino-modified nucleotide, 2'-O-allyl-modified nucleotide, 2'-C-alkyl-modified nucleotide, 2'-methoxyethyl-modified nucleotide, abasic nucleotide, inverted abasic nucleotide, inverted nucleotide, morpholino nucleotide, phosphoramidate, tetrahydropyran-modified nucleotide, 1,5-anhydrohexitol-modified nucleotide, cyclohexenyl-modified nucleotide, nucleotide comprising phosphorothioate group, nucleotide comprising methylphosphonate group, nucleotide comprising 5'-phosphate, and nucleotide comprising 5'-phosphate mimic; preferably selected from 2'-O-methyl-modified nucleotide, 2'-fluoro-modified nucleotide, and nucleotide comprising phosphorothioate internucleoside linkage.

The term "ligand" refers to a cell or tissue targeting agent that binds to a specified cell type (such as hepatocytes), including, for example, lectins, glycoproteins, lipids, or proteins (e.g., antibodies). Exemplary targeting agents include, but are not limited to, thyrotropin, melanotropin, lectins, glycoproteins, surfactant protein A, mucin carbohydrates, multivalent lactose, multivalent galactose, N-acetylgalactosamine (GalNAc), multivalent (e.g., bivalent or trivalent) GalNAc, N-acetylglucosamine, multivalent mannose, multivalent fucose, glycosylated poly-amino acids, multivalent galactose, transferrin, bisphosphonates, polyglutamate, polyaspartate, cholesterol, steroids, bile acids, folate, vitamin B12, biotin, RGD peptides, and RGD peptide mimetics. In preferred embodiments, the ligand is a carbohydrate, such as a monosaccharide, disaccharide, trisaccharide, tetrasaccharide, or polysaccharide. For example, the ligand may be a derivative comprising GalNAc. In preferred embodiments, the ligand comprises one or more N-acetylgalactosamine derivatives attached via a bivalent or trivalent branched linker, such as L96.

The term "therapeutically effective amount" refers to an amount of an RNAi agent or composition of the present invention that is effective to produce a desired therapeutic effect in at least a subpopulation of cells in an animal at a

reasonable benefit/risk ratio applicable to any medical treatment.

The term "pharmaceutically acceptable" refers to compounds, materials, compositions, and/or dosage forms that are, within the scope of sound medical judgment, suitable for contact with tissues of humans and animals without excessive toxicity, irritation, allergic reactions, or other problems or complications and commensurate with a reasonable benefit/risk ratio.

The term "pharmaceutically acceptable carrier" refers to a pharmaceutically acceptable material, composition, or vehicle that participates in carrying or transporting an RNAi agent from one organ or part of the body to another organ or part of the body, such as a liquid or solid filler, diluent, excipient, manufacturing aid, or solvent encapsulating material. Each carrier must be "acceptable" in the sense of being compatible with other ingredients of the composition and innocuous to the patient.

The term "treatment" encompasses prevention, therapy, and cure. The patient receiving such treatment is typically any animal in need, including primates (particularly humans) and other mammals such as horses, cattle, swine, sheep, poultry, and companion animals.

## Modified RNAi Agents

One aspect of the present invention provides a modified RNAi agent comprising a sense strand and an antisense strand that form a duplex region, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

In some embodiments, the present invention provides a modified RNAi agent comprising a sense strand and an antisense strand that form a duplex region, wherein, in the 5' to 3' direction, the nucleotides at positions 2, 5, 7, 12, and 14 of the antisense strand are 2'-fluoro-modified nucleotides, while the nucleotides at remaining positions of the antisense strand are all 2'-methoxy-modified nucleotides.

In some embodiments, the present invention provides a modified RNAi agent comprising a sense strand and an antisense strand that form a duplex region, wherein, in the 5' to 3' direction, the nucleotides at positions 2, 5, 7, 14, and 16 of the antisense strand are 2'-fluoro-modified nucleotides, while the nucleotides at remaining positions of the antisense strand are all 2'-methoxy-modified nucleotides.

In some embodiments, the present invention provides a modified RNAi agent comprising a sense strand and an antisense strand that form a duplex region, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and wherein the antisense strand comprises at least one phosphorothioate internucleotide linkage. In a preferred embodiment, the phosphorothioate internucleotide linkage is present at one or more of the following locations: between nucleotide positions 1 and 2 at 5'-end of the antisense strand; between nucleotide positions 2 and 3 at 5'-end of the antisense strand; between nucleotide positions 1 and 2 at 3'-end of the antisense strand; and between nucleotide positions 2 and 3 at 3'-end of the antisense strand. In a preferred embodiment, the phosphorothioate internucleotide linkage is present between nucleotide positions 1 and 2 at 5'-end of the antisense strand; between nucleotide positions 2 and 3 at 5'-end of the antisense strand; between nucleotide positions 1 and 2 at 3'-end of the antisense strand; and between nucleotide positions 2 and 3 at 3'-end of the antisense strand.

The duplex region of the RNAi agent should be of sufficient length to allow the RNAi agent to enter the RNA interference pathway, for example, by engaging the Dicer enzyme and/or the RISC complex. In some embodiments, the duplex region of the RNAi agent of the present invention has a length of about 17 to about 23 base pairs. For example, suitable duplex lengths are about 17 to about 22 base pairs, about 17 to about 21 base pairs, about 17 to about 20 base pairs, about 17 to about 19 base pairs, about 17 to about 18 base pairs, about 18 to about 23 base pairs, about 18 to about 22 base pairs, about 18 to about 21 base pairs, about 18 to about 20 base pairs, about 19 to about 23 base pairs, about 19 to about 22 base pairs, about 19 to about 21 base pairs, about 20 to about 23 base pairs, about 20 to about 22 base pairs, or about 21 to about 23 base pairs. In certain embodiments, the duplex region has a length of about 18 to about 21 base pairs. In other embodiments, the duplex region has a length of about 19 base pairs.

Accordingly, in some embodiments, the present invention provides a modified RNAi agent comprising a sense strand and an antisense strand that form a duplex region having a length of about 17 to about 23 base pairs, preferably about 18 to about 21 base pairs, more preferably about 19 base pairs, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

In some embodiments, the present invention provides a modified RNAi agent comprising a sense strand and an antisense strand that form a duplex region having a length of about 17 to about 23 base pairs, preferably about 18 to about

21 base pairs, more preferably about 19 base pairs, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and wherein the antisense strand comprises at least one phosphorothioate internucleotide linkage.

In some embodiments, the present invention provides a modified RNAi agent comprising a sense strand and an antisense strand that form a duplex region having a length of about 17 to about 23 base pairs, preferably about 18 to about 21 base pairs, more preferably about 19 base pairs, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and wherein the antisense strand comprises at least one phosphorothioate internucleotide linkage, wherein the phosphorothioate internucleotide linkage is present between nucleotide positions 1 and 2 at 5'-end of the antisense strand; between nucleotide positions 2 and 3 at 5'-end of the antisense strand; between nucleotide positions 1 and 2 at 3'-end of the antisense strand; and between nucleotide positions 2 and 3 at 3'-end of the antisense strand.

In some embodiments, the sense strand and the antisense strand in the modified RNAi agent provided by the present invention each independently have a length of about 17 to about 23 nucleotides, for example, about 18 to about 23 nucleotides, about 19 to about 23 nucleotides, about 20 to about 23 nucleotides, about 21 to about 23 nucleotides, about 17 to about 22 nucleotides, about 17 to about 21 nucleotides, about 17 to about 20 nucleotides, about 17 to about 19 nucleotides, about 18 to about 22 nucleotides, about 18 to about 21 nucleotides, about 18 to about 20 nucleotides, about 19 to about 22 nucleotides, about 19 to about 21 nucleotides, or about 20 to about 22 nucleotides. In certain embodiments, the sense strand and the antisense strand each independently have a length of about 17, about 18, about 19, about 20, about 21, about 22, or about 23 nucleotides.

In some embodiments, the sense strand and antisense strand are of identical length but form a duplex region shorter than the full length of the strands, such that the RNAi agent comprises two nucleotide overhangs. For example, in one embodiment, the RNAi agent comprises (i) a sense strand and an antisense strand each having a length of 21 nucleotides, (ii) a duplex region having a length of 19 base pairs, and (iii) a single-nucleotide overhang at each of 3'-end of the sense strand and 3'-end of the antisense strand. In another embodiment, the RNAi agent comprises (i) a sense strand and an antisense strand each having a length of 23 nucleotides, (ii) a duplex region having a length of 21 base pairs, and (iii) a single-nucleotide overhang at each of 3'-end of the sense strand and 3'-end of the antisense strand.

In other embodiments, the sense strand and antisense strand are of identical length and form a duplex region along their entire length, such that no nucleotide overhangs are present at either end of the duplex molecule. In one such embodiment, the RNAi agent is blunt-ended and comprises (i) a sense strand and an antisense strand each having a length of 21 nucleotides, and (ii) a duplex region having a length of 21 base pairs. In another such embodiment, the RNAi agent is blunt-ended and comprises (i) a sense strand and an antisense strand each having a length of 23 nucleotides, and (ii) a duplex region having a length of 23 base pairs. In another such embodiment, the RNAi agent is blunt-ended and comprises (i) a sense strand and an antisense strand each having a length of 19 nucleotides, and (ii) a duplex region having a length of 19 base pairs.

In other embodiments, the sense strand or antisense strand is longer than the other strand, and the two strands form a duplex region of length equal to that of the shorter strand, such that the RNAi agent comprises at least one nucleotide overhang. For example, in some embodiments, the sense strand is 1 to 4 nucleotides longer than the antisense strand, and the duplex region formed by both strands equals the length of the antisense strand, such that the sense strand forms an overhang comprising 1 to 4 unpaired nucleotides. In other embodiments, the antisense strand is 1 to 4 nucleotides longer than the sense strand, and the duplex region formed by both strands equals the length of the sense strand, such that the antisense strand forms an overhang comprising 1 to 4 unpaired nucleotides. In some embodiments, the nucleotide overhang has a length of 1, 2, 3, or 4 nucleotides. In a specific embodiment, the overhang comprises 2 nucleotides. In certain embodiments, the overhang comprises a single nucleotide.

The overhanging nucleotides may be ribonucleotides or modified nucleotides as described herein. In some embodiments, the overhanging nucleotides are 2'-modified nucleotides (e.g., 2'-fluoro-modified nucleotides, 2'-O-methyl-modified nucleotides) or combinations thereof. For example, in one embodiment, the overhanging nucleotides are deoxyribonucleotides, such as deoxythymidine. In another embodiment, the overhanging nucleotides are selected from: 2'-O-methyl-modified nucleotides, 2'-fluoro-modified nucleotides, 2'-methoxyethyl-modified nucleotides, abasic nucleotides, inverted abasic nucleotides, inverted nucleotides, or combinations thereof. In other embodiments, the overhang comprises a 5'-uridine-uridine-3' (5'-UU-3') dinucleotide. In such embodiments, the UU dinucleotide may comprise ribonucleotides or modified nucleotides, such as 2'-modified nucleotides. In other embodiments, the overhang comprises a 5'-deoxythymidine-deoxythymidine-3' (5'-dTdT-3') dinucleotide. When a nucleotide overhang is present on the antisense strand, nucleotides in the overhang may be complementary to the target gene sequence, may form mismatches with the target gene sequence, or may comprise other sequences (such as UU, TT, AA, GG, etc.).

The nucleotide overhang may be located at the 5'-end or 3'-end of either strand or both strands. For example, in one embodiment, the RNAi agent comprises nucleotide overhangs at both 5'-end and 3'-end of the antisense strand. In another embodiment, the RNAi agent comprises nucleotide overhangs at both 5'-end and 3'-end of the sense strand. In some embodiments, the RNAi agent comprises nucleotide overhangs at the 5'-end of the sense strand and the 5'-end of the antisense strand. In other embodiments, the RNAi agent comprises nucleotide overhangs at the 3'-end of the sense strand and the 3'-end of the antisense strand. In some embodiments, the RNAi agent comprises only a nucleotide overhang at the 5'-end of the sense strand. In some embodiments, the RNAi agent comprises only a nucleotide overhang at the 3'-end of the sense strand. In some embodiments, the RNAi agent comprises only a nucleotide overhang at the 3'-end of the antisense strand. In some embodiments, the RNAi agent comprises only a nucleotide overhang at the 5'-end of the antisense strand. In some embodiments, the RNAi agent comprises only a nucleotide overhang at the 5'-end of the sense strand.

The RNAi agent may comprise a nucleotide overhang at one end and a blunt end at the opposite end of the double-stranded RNA molecule. "Blunt end" means that the sense strand and antisense strand are fully base-paired at the molecular terminus, with no unpaired nucleotides extending beyond the duplex region. In some embodiments, the RNAi agent comprises a nucleotide overhang at the 3'-end of the sense strand, and blunt ends at the 5'-end of the sense strand and the 3'-end of the antisense strand. In other embodiments, the RNAi agent comprises a nucleotide overhang at the 3'-end of the antisense strand, and blunt ends at the 5'-end of the antisense strand and the 3'-end of the sense strand.

Specifically, by way of example, in one embodiment, the RNAi agent comprises: (i) a sense strand of 19 nucleotides in length, (ii) an antisense strand of 21 nucleotides in length, wherein the strands form a duplex region equal to the length of the sense strand. In another embodiment, the RNAi agent comprises: (i) a sense strand of 21 nucleotides in length, (ii) an antisense strand of 23 nucleotides in length, wherein the strands form a duplex region equal to the length of the sense strand.

In some embodiments, in the 5' to 3' direction, the nucleotides at positions 7 and 9 of the sense strand are 2'-fluoro-modified nucleotides, one or two of the nucleotides at positions 5, 8, and 11 of the sense strand are 2'-fluoro-modified nucleotides, while the nucleotides at remaining positions of the sense strand are all 2'-methoxy-modified nucleotides.

For example, in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides; or, in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides.

In a preferred embodiment, the sense strand comprises at least one phosphorothioate internucleotide linkage. In a preferred embodiment, the phosphorothioate internucleotide linkage is present between nucleotide positions 1 and 2 at 5'-end of the sense strand; and/or between nucleotide positions 2 and 3 at 5'-end of the sense strand. In a more preferred embodiment, the phosphorothioate internucleotide linkage is present between nucleotide positions 1 and 2 at 5'-end of the sense strand; and between nucleotide positions 2 and 3 at 5'-end of the sense strand.

Accordingly, in some embodiments, in the 5' to 3' direction, the nucleotides at positions 7 and 9 of the sense strand are 2'-fluoro-modified nucleotides, one or two of the nucleotides at positions 5, 8, and 11 of the sense strand are 2'-fluoro-modified nucleotides, while the nucleotides at remaining positions of the sense strand are all 2'-methoxy-modified nucleotides; and wherein the sense strand comprises at least one phosphorothioate internucleotide linkage, and the phosphorothioate internucleotide linkage is present between nucleotide positions 1 and 2 at 5'-end of the sense strand; and/or between nucleotide positions 2 and 3 at 5'-end of the sense strand.

In some embodiments, in the 5' to 3' direction, the nucleotides at positions 7 and 9 of the sense strand are 2'-fluoro-modified nucleotides, one or two of the nucleotides at positions 5, 8, and 11 of the sense strand are 2'-fluoro-modified nucleotides, while the nucleotides at remaining positions of the sense strand are all 2'-methoxy-modified nucleotides; and wherein the sense strand comprises at least one phosphorothioate internucleotide linkage, and the phosphorothioate internucleotide linkage is present between nucleotide positions 1 and 2 at 5'-end of the sense strand; and between nucleotide positions 2 and 3 at 5'-end of the sense strand.

In some embodiments, in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides; or, in the 5' to 3' direction, the nucleotides at positions 7, 9, and 11 of the sense strand are 2'-fluoro-modified nucleotides; and, the nucleotides at the

remaining positions of the sense strand are all 2'-methoxy-modified nucleotides; and wherein the sense strand comprises at least one phosphorothioate internucleotide linkage, and the phosphorothioate internucleotide linkage is present between nucleotide positions 1 and 2 at 5'-end of the sense strand; and between nucleotide positions 2 and 3 at 5'-end of the sense strand.

In a preferred embodiment, the present invention provides a modified RNAi agent comprising a sense strand and an antisense strand that form a duplex region, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and wherein, in the 5' to 3' direction, the nucleotides at positions 7 and 9 of the sense strand are 2'-fluoro-modified nucleotides, one or two of the nucleotides at positions 5, 8, and 11 of the sense strand are 2'-fluoro-modified nucleotides, while the nucleotides at remaining positions of the sense strand are all 2'-methoxy-modified nucleotides.

In a preferred embodiment, the present invention provides a modified RNAi agent comprising a sense strand and an antisense strand that form a duplex region, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and wherein, in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides; or, in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides.

In a preferred embodiment, the present invention provides a modified RNAi agent comprising a sense strand and an antisense strand that form a duplex region, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and wherein, in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides; or, in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides; and wherein the antisense strand and the sense strand each have at least one phosphorothioate internucleotide linkage. In a preferred embodiment, the phosphorothioate internucleotide linkage of the antisense strand is present in one or more of the following: between nucleotide positions 1 and 2 at 5'-end of the antisense strand; between nucleotide positions 2 and 3 at 5'-end of the antisense strand; between nucleotide positions 1 and 2 at 3'-end of the antisense strand; and between nucleotide positions 2 and 3 at 3'-end of the antisense strand; and the phosphorothioate internucleotide linkage of the sense strand is present between nucleotide positions 1 and 2 at 5'-end of the sense strand; and/or between nucleotide positions 2 and 3 at 5'-end of the sense strand.

In a preferred embodiment, the present invention provides a modified RNAi agent comprising a sense strand and an antisense strand that form a duplex region, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and wherein, in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides; or, in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides; and wherein the antisense strand and the sense strand each have at least one phosphorothioate internucleotide linkage, wherein the phosphorothioate internucleotide linkage of the antisense strand is present between nucleotide positions 1 and 2 at 5'-end of the antisense strand; between nucleotide positions 2 and 3 at 5'-end of the antisense strand; between nucleotide

positions 1 and 2 at 3'-end of the antisense strand; and between nucleotide positions 2 and 3 at 3'-end of the antisense strand; and the phosphorothioate internucleotide linkage of the sense strand is present between nucleotide positions 1 and 2 at 5'-end of the sense strand; and between nucleotide positions 2 and 3 at 5'-end of the sense strand.

In a preferred embodiment, the present invention provides a modified RNAi agent comprising a sense strand and an antisense strand that form a duplex region having a length of about 17 to about 23 base pairs, preferably about 18 to about 21 base pairs, more preferably about 19 base pairs, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and wherein, in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides; or, in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides; and wherein the antisense strand and the sense strand each have at least one phosphorothioate internucleotide linkage, wherein the phosphorothioate internucleotide linkage of the antisense strand is present between nucleotide positions 1 and 2 at 5'-end of the antisense strand; between nucleotide positions 2 and 3 at 5'-end of the antisense strand; between nucleotide positions 1 and 2 at 3'-end of the antisense strand; and between nucleotide positions 2 and 3 at 3'-end of the antisense strand; and the phosphorothioate internucleotide linkage of the sense strand is present between nucleotide positions 1 and 2 at 5'-end of the sense strand; and between nucleotide positions 2 and 3 at 5'-end of the sense strand.

In a preferred embodiment, the present invention provides a modified RNAi agent comprising a sense strand and an antisense strand that form a duplex region having a length of about 17 to about 23 base pairs, preferably about 18 to about 21 base pairs, more preferably about 19 base pairs, and the sense strand and the antisense strand each independently have a length of about 17 to about 23 nucleotides, wherein the RNAi agent comprises a nucleotide overhang at 3'-end of the antisense strand and blunt ends at 5'-end of the antisense strand and 3'-end of the sense strand, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and wherein, in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides; or, in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides; and wherein the antisense strand and the sense strand each have at least one phosphorothioate internucleotide linkage, wherein the phosphorothioate internucleotide linkage of the antisense strand is present between nucleotide positions 1 and 2 at 5'-end of the antisense strand; between nucleotide positions 2 and 3 at 5'-end of the antisense strand; between nucleotide positions 1 and 2 at 3'-end of the antisense strand; and between nucleotide positions 2 and 3 at 3'-end of the antisense strand; and the phosphorothioate internucleotide linkage of the sense strand is present between nucleotide positions 1 and 2 at 5'-end of the sense strand; and between nucleotide positions 2 and 3 at 5'-end of the sense strand.

In a preferred embodiment, the present invention provides a modified RNAi agent comprising a sense strand and an antisense strand that form a duplex region having a length of about 19 base pairs, the sense strand has a length of about 19 nucleotides, and the antisense strand has a length of about 21 nucleotides, wherein the RNAi agent comprises an overhang of about two nucleotides at 3'-end of the antisense strand and blunt ends at 5'-end of the antisense strand and 3'-end of the sense strand, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and wherein, in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides; or, in the 5' to 3' direction, the nucleotides at positions 7, 9, and 11 of the sense strand are 2'-fluoro-modified nucleotides, while the nucleotides at

remaining positions of the sense strand are all 2'-methoxy-modified nucleotides; and wherein the antisense strand and the sense strand each have at least one phosphorothioate internucleotide linkage, wherein the phosphorothioate internucleotide linkage of the antisense strand is present between nucleotide positions 1 and 2 at 5'-end of the antisense strand; between nucleotide positions 2 and 3 at 5'-end of the antisense strand; between nucleotide positions 1 and 2 at 3'-end of the antisense strand; and between nucleotide positions 2 and 3 at 3'-end of the antisense strand; and the phosphorothioate internucleotide linkage of the sense strand is present between nucleotide positions 1 and 2 at 5'-end of the sense strand; and between nucleotide positions 2 and 3 at 5'-end of the sense strand.

In a preferred embodiment, the present invention provides a modified RNAi agent that inhibits the expression of the ANGPTL3 gene, comprising a sense strand and an antisense strand that form a duplex region having a length of about 19 base pairs, the sense strand has a length of about 19 nucleotides, and the antisense strand has a length of about 21 nucleotides, wherein the RNAi agent comprises an overhang of about two nucleotides at 3'-end of the antisense strand and blunt ends at 5'-end of the antisense strand and 3'-end of the sense strand, wherein, in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and wherein, in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides; or, in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides; and wherein the antisense strand and the sense strand each have at least one phosphorothioate internucleotide linkage, wherein the phosphorothioate internucleotide linkage of the antisense strand is present between nucleotide positions 1 and 2 at 5'-end of the antisense strand; between nucleotide positions 2 and 3 at 5'-end of the antisense strand; between nucleotide positions 1 and 2 at 3'-end of the antisense strand; and between nucleotide positions 2 and 3 at 3'-end of the antisense strand; and the phosphorothioate internucleotide linkage of the sense strand is present between nucleotide positions 1 and 2 at 5'-end of the sense strand; and between nucleotide positions 2 and 3 at 5'-end of the sense strand.

RNAi Agents for Inhibiting ANGPTL3 Gene Expression

Another aspect of the present invention provides an RNAi agent for inhibiting the expression of the ANGPTL3 gene, comprising a sense strand and an antisense strand that form a complementary duplex region, wherein the antisense strand is no more than 23 nucleotides in length and comprises a nucleotide sequence selected from any one of SEQ ID NOs: 13, 14, 27, and 28.

In some embodiments, the duplex region has a length of about 17 to about 23 base pairs. For example, suitable duplex lengths are about 17 to about 22 base pairs, about 17 to about 21 base pairs, about 17 to about 20 base pairs, about 17 to about 19 base pairs, about 17 to about 18 base pairs, about 18 to about 23 base pairs, about 18 to about 22 base pairs, about 18 to about 21 base pairs, about 18 to about 20 base pairs, about 19 to about 23 base pairs, about 19 to about 22 base pairs, about 19 to about 21 base pairs, about 20 to about 23 base pairs, about 20 to about 22 base pairs, or about 21 to about 23 base pairs. In certain embodiments, the duplex region has a length of about 18 to about 21 base pairs. In other embodiments, the duplex region has a length of about 19 base pairs.

Accordingly, in some embodiments of the present invention, an RNAi agent for inhibiting the expression of the ANGPTL3 gene is provided, comprising a sense strand and an antisense strand that form a complementary duplex region having a length of about 17 to about 23 base pairs, and wherein the antisense strand is no more than 23 nucleotides in length and comprises a nucleotide sequence selected from any one of SEQ ID NOs: 13, 14, 27, and 28.

In some embodiments of the present invention, an RNAi agent for inhibiting the expression of the ANGPTL3 gene is provided, comprising a sense strand and an antisense strand that form a complementary duplex region having a length of about 18 to about 21 base pairs, and wherein the antisense strand is no more than 23 nucleotides in length and comprises a nucleotide sequence selected from any one of SEQ ID NOs: 13, 14, 27, and 28.

In some embodiments of the present invention, an RNAi agent for inhibiting the expression of the ANGPTL3 gene is provided, comprising a sense strand and an antisense strand that form a complementary duplex region having a length of about 19 base pairs, and wherein the antisense strand is no more than 23 nucleotides in length and comprises a nucleotide sequence selected from any one of SEQ ID NOs: 13, 14, 27, and 28.

In some embodiments, the sense strand and the antisense strand in the RNAi agent of the present invention each independently have a length of about 17 to about 23 nucleotides, for example, about 18 to about 23 nucleotides, about 19 to about 23 nucleotides, about 20 to about 23 nucleotides, about 21 to about 23 nucleotides, about 17 to about 22 nucleotides,

about 17 to about 21 nucleotides, about 17 to about 20 nucleotides, about 17 to about 19 nucleotides, about 18 to about 22 nucleotides, about 18 to about 21 nucleotides, about 18 to about 20 nucleotides, about 19 to about 22 nucleotides, about 19 to about 21 nucleotides, or about 20 to about 22 nucleotides. In certain embodiments, the sense strand and the antisense strand each independently have a length of about 17, about 18, about 19, about 20, about 21, about 22, or about 23 nucleotides.

In some embodiments, the sense strand and antisense strand are of identical length but form a duplex region shorter than the full length of the strands, such that the RNAi agent for inhibiting ANGPTL3 gene expression comprises two nucleotide overhangs. For example, in one embodiment, the RNAi agent for inhibiting ANGPTL3 gene expression comprises (i) a sense strand and an antisense strand each having a length of 21 nucleotides, (ii) a duplex region having a length of 19 base pairs, and (iii) a single-nucleotide overhang at each of 3'-end of the sense strand and 3'-end of the antisense strand. In another embodiment, the RNAi agent for inhibiting ANGPTL3 gene expression comprises (i) a sense strand and an antisense strand each having a length of 23 nucleotides, (ii) a duplex region having a length of 21 base pairs, and (iii) a single-nucleotide overhang at each of 3'-end of the sense strand and 3'-end of the antisense strand.

In other embodiments, the sense strand and antisense strand are of identical length and form a duplex region along their entire length, such that no nucleotide overhangs are present at either end of the duplex molecule. In one such embodiment, the RNAi agent for inhibiting ANGPTL3 gene expression is blunt-ended and comprises (i) a sense strand and an antisense strand each having a length of 21 nucleotides, and (ii) a duplex region having a length of 21 base pairs. In another such embodiment, the RNAi agent for inhibiting ANGPTL3 gene expression is blunt-ended and comprises (i) a sense strand and an antisense strand each having a length of 23 nucleotides, and (ii) a duplex region having a length of 23 base pairs. In another such embodiment, the RNAi agent for inhibiting ANGPTL3 gene expression is blunt-ended and comprises (i) a sense strand and an antisense strand each having a length of 19 nucleotides, and (ii) a duplex region having a length of 19 base pairs.

In other embodiments, the sense strand or antisense strand is longer than the other strand, and the two strands form a duplex region of length equal to that of the shorter strand, such that the RNAi agent for inhibiting ANGPTL3 gene expression comprises at least one nucleotide overhang. For example, in some embodiments, the sense strand is 1 to 4 nucleotides longer than the antisense strand, and the duplex region formed by both strands equals the length of the sense strand, such that the sense strand forms an overhang having 1 to 4 unpaired nucleotides. In other embodiments, the antisense strand is 1 to 4 nucleotides longer than the sense strand, and the duplex region formed by both strands equals the length of the sense strand, such that the antisense strand forms an overhang having 1 to 4 unpaired nucleotides. In some embodiments, the nucleotide overhang has a length of 1, 2, 3, or 4 nucleotides. In a particular embodiment, the overhang comprises 2 nucleotides. In certain embodiments, the overhang comprises a single nucleotide.

The overhanging nucleotides may be ribonucleotides or modified nucleotides as described herein. In some embodiments, the overhanging nucleotides are 2'-modified nucleotides (e.g., 2'-fluoro-modified nucleotides, 2'-O-methyl-modified nucleotides) or combinations thereof. For example, in one embodiment, the overhanging nucleotides are deoxyribonucleotides, such as deoxythymidine. In another embodiment, the overhanging nucleotides are selected from: 2'-O-methyl-modified nucleotides, 2'-fluoro-modified nucleotides, 2'-methoxyethyl-modified nucleotides, abasic nucleotides, inverted abasic nucleotides, inverted nucleotides, or combinations thereof. In other embodiments, the overhang comprises a 5'-uridine-uridine-3' (5'-UU-3') dinucleotide. In such embodiments, the UU dinucleotide may comprise ribonucleotides or modified nucleotides, such as 2'-modified nucleotides. In other embodiments, the overhang comprises a 5'-deoxythymidine-deoxythymidine-3' (5'-dTdT-3') dinucleotide. When a nucleotide overhang is present on the antisense strand, nucleotides in the overhang may be complementary to the target gene sequence, may form mismatches with the target gene sequence, or may comprise other sequences (such as UU, TT, AA, GG, etc.).

The nucleotide overhang may be located at the 5'-end or 3'-end of either strand or both strands. For example, in one embodiment, the RNAi agent for inhibiting ANGPTL3 gene expression comprises nucleotide overhangs at both 5'-end and 3'-end of the antisense strand. In another embodiment, the RNAi agent for inhibiting ANGPTL3 gene expression comprises nucleotide overhangs at both 5'-end and 3'-end of the sense strand. In some embodiments, the RNAi agent for inhibiting ANGPTL3 gene expression comprises nucleotide overhangs at 5'-end of the sense strand and 5'-end of the antisense strand. In other embodiments, the RNAi agent for inhibiting ANGPTL3 gene expression comprises nucleotide overhangs at 3'-end of the sense strand and 3'-end of the antisense strand. In some embodiments, the RNAi agent for inhibiting ANGPTL3 gene expression comprises only a nucleotide overhang at the 5'-end of the sense strand. In some embodiments, the RNAi agent for inhibiting ANGPTL3 gene expression comprises only a nucleotide overhang at the 3'-end of the sense strand. In some embodiments, the RNAi agent for inhibiting ANGPTL3 gene expression comprises only a nucleotide overhang at the 3'-end of the antisense strand. In some embodiments, the RNAi agent for inhibiting ANGPTL3 gene expression comprises only a nucleotide overhang at the 5'-end of the antisense strand. In some embodiments, the RNAi agent for inhibiting ANGPTL3 gene expression comprises only a nucleotide overhang at the 5'-end of the sense strand.

The RNAi agent for inhibiting ANGPTL3 gene expression may comprise a nucleotide overhang at one end and a blunt end at the opposite end of the double-stranded RNA molecule. "Blunt end" means that the sense strand and

antisense strand are fully base-paired at the molecular terminus, with no unpaired nucleotides extending beyond the duplex region. In some embodiments, the RNAi agent for inhibiting ANGPTL3 gene expression comprises a nucleotide overhang at 3'-end of the sense strand and blunt ends at 5'-end of the sense strand and 3'-end of the antisense strand. In other embodiments, the RNAi agent for inhibiting ANGPTL3 gene expression comprises a nucleotide overhang at 3'-end of the antisense strand and blunt ends at 5'-end of the antisense strand and 3'-end of the sense strand.

Specifically, by way of example, in one embodiment, the RNAi agent for inhibiting ANGPTL3 gene expression comprises (i) a sense strand having a length of 19 nucleotides, (ii) an antisense strand having a length of 21 nucleotides, wherein the strands form a duplex region equal to the length of the sense strand. In another embodiment, the RNAi agent for inhibiting ANGPTL3 gene expression comprises (i) a sense strand having a length of 21 nucleotides, (ii) an antisense strand having a length of 23 nucleotides, wherein the strands form a duplex region equal to the length of the sense strand.

In some embodiments, an RNAi agent for inhibiting the expression of the ANGPTL3 gene in a cell is provided, comprising a sense strand and an antisense strand that form a duplex region, wherein the antisense strand is no more than 23 nucleotides in length and comprises a nucleotide sequence selected from any one of SEQ ID NOs: 13, 14, 27, and 28, and wherein the RNAi agent comprises one overhang and one blunt end, the overhang preferably having 2 unpaired nucleotides.

In some embodiments, an RNAi agent for inhibiting the expression of the ANGPTL3 gene in a cell is provided, comprising a sense strand and an antisense strand that form a duplex region, wherein the antisense strand is no more than 23 nucleotides in length and comprises a nucleotide sequence selected from any one of SEQ ID NOs: 13, 14, 27, and 28, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting the expression of the ANGPTL3 gene in a cell is provided, comprising a sense strand and an antisense strand that form a duplex region having a length of 19 base pairs, wherein the antisense strand is no more than 23 nucleotides in length and comprises a nucleotide sequence selected from any one of SEQ ID NOs: 13, 14, 27, and 28, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting the expression of the ANGPTL3 gene in a cell is provided, comprising a sense strand and an antisense strand that form a duplex region having a length of 19 base pairs, wherein the antisense strand is no more than 23 nucleotides in length and comprises a nucleotide sequence selected from any one of SEQ ID NOs: 13, 14, 27, and 28, wherein the sense strand has a length of 19 to 21 nucleotides, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting the expression of the ANGPTL3 gene in a cell is provided, comprising a sense strand and an antisense strand that form a duplex region having a length of 19 base pairs, wherein the antisense strand has a length of 21 nucleotides and consists of a nucleotide sequence selected from any one of SEQ ID NOs: 13, 14, 27, and 28, wherein the sense strand has a length of 19 nucleotides, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, in the RNAi agent for inhibiting the expression of the ANGPTL3 gene in a cell, in the 5' to 3' direction, the nucleotides at positions 7 and 9 of the sense strand are 2'-fluoro-modified nucleotides, one or two of the nucleotides at positions 5, 8, and 11 of the sense strand are 2'-fluoro-modified nucleotides, while the nucleotides at remaining positions of the sense strand are all 2'-methoxy-modified nucleotides.

For example, in the RNAi agent for inhibiting the expression of the ANGPTL3 gene in a cell, in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides; or, in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides.

In a preferred embodiment, the sense strand comprises at least one phosphorothioate internucleotide linkage. In a preferred embodiment, the phosphorothioate internucleotide linkage is present between nucleotide positions 1 and 2 at 5'-end of the sense strand; and/or between nucleotide positions 2 and 3 at 5'-end of the sense strand. In a more preferred embodiment, the phosphorothioate internucleotide linkage is present between nucleotide positions 1 and 2 at 5'-end of the

sense strand; and between nucleotide positions 2 and 3 at 5'-end of the sense strand.

Accordingly, in some embodiments, in the RNAi agent for inhibiting the expression of the ANGPTL3 gene in a cell, in the 5' to 3' direction, the nucleotides at positions 7 and 9 of the sense strand are 2'-fluoro-modified nucleotides, one or two of the nucleotides at positions 5, 8, and 11 of the sense strand are 2'-fluoro-modified nucleotides, while the nucleotides at remaining positions of the sense strand are all 2'-methoxy-modified nucleotides; and wherein the sense strand comprises at least one phosphorothioate internucleotide linkage, and the phosphorothioate internucleotide linkage is present between nucleotide positions 1 and 2 at 5'-end of the sense strand; and/or between nucleotide positions 2 and 3 at 5'-end of the sense strand.

In some embodiments, in the RNAi agent for inhibiting the expression of the ANGPTL3 gene in a cell, in the 5' to 3' direction, the nucleotides at positions 7 and 9 of the sense strand are 2'-fluoro-modified nucleotides, one or two of the nucleotides at positions 5, 8, and 11 of the sense strand are 2'-fluoro-modified nucleotides, while the nucleotides at remaining positions of the sense strand are all 2'-methoxy-modified nucleotides; and wherein the sense strand comprises at least one phosphorothioate internucleotide linkage, and the phosphorothioate internucleotide linkage is present between nucleotide positions 1 and 2 at 5'-end of the sense strand; and between nucleotide positions 2 and 3 at 5'-end of the sense strand.

In some embodiments, in the RNAi agent for inhibiting the expression of the ANGPTL3 gene in a cell, in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides; in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides; or, in the 5' to 3' direction, the nucleotides at positions 7, 9, and 11 of the sense strand are 2'-fluoro-modified nucleotides; and, the nucleotides at the remaining positions of the sense strand are all 2'-methoxy-modified nucleotides; and wherein the sense strand comprises at least one phosphorothioate internucleotide linkage, and the phosphorothioate internucleotide linkage is present between nucleotide positions 1 and 2 at 5'-end of the sense strand; and between nucleotide positions 2 and 3 at 5'-end of the sense strand.

In a preferred embodiment, the sense strand is no more than 21 nucleotides in length and comprises a nucleotide sequence selected from any one of SEQ ID NOs: 5, 7, 8, 19, 20, and 22. In a preferred embodiment, the sense strand has a length of 20 nucleotides and comprises a nucleotide sequence selected from any one of SEQ ID NOs: 5, 7, 8, 19, 20, and 22. In a preferred embodiment, the sense strand consists of a nucleotide sequence selected from any one of SEQ ID NOs: 5, 7, 8, 19, 20, and 22.

In some embodiments, an RNAi agent for inhibiting the expression of the ANGPTL3 gene is provided, comprising a sense strand and an antisense strand that form a complementary duplex region, wherein the antisense strand is no more than 23 nucleotides in length and comprises a nucleotide sequence selected from any one of SEQ ID NOs: 13, 14, 27, and 28, and wherein the sense strand is no more than 21 nucleotides in length and comprises a nucleotide sequence selected from any one of SEQ ID NOs: 5, 7, 8, 19, 20, and 22.

In some embodiments, an RNAi agent for inhibiting the expression of the ANGPTL3 gene is provided, comprising a sense strand and an antisense strand that form a complementary duplex region, wherein the antisense strand consists of a nucleotide sequence selected from any one of SEQ ID NOs: 13, 14, 27, and 28, and wherein the sense strand consists of a nucleotide sequence selected from any one of SEQ ID NOs: 5, 7, 8, 19, 20, and 22.

In a preferred embodiment, an RNAi agent for inhibiting the expression of the ANGPTL3 gene is provided, comprising a sense strand and an antisense strand that form a complementary duplex region, wherein the sense strand and the antisense strand are each no more than 23 nucleotides in length, and wherein:

the sense strand comprises or consists of a sequence set forth in SEQ ID NO: 5, and the antisense strand comprises or consists of a sequence set forth in SEQ ID NO: 13;
the sense strand comprises or consists of a sequence set forth in SEQ ID NO: 5, and the antisense strand comprises or consists of a sequence set forth in SEQ ID NO: 14;
the sense strand comprises or consists of a sequence set forth in SEQ ID NO: 7, and the antisense strand comprises or consists of a sequence set forth in SEQ ID NO: 13;
the sense strand comprises or consists of a sequence set forth in SEQ ID NO: 8, and the antisense strand comprises or consists of a sequence set forth in SEQ ID NO: 13;
the sense strand comprises or consists of a sequence set forth in SEQ ID NO: 19, and the antisense strand comprises or consists of a sequence set forth in SEQ ID NO: 27;
the sense strand comprises or consists of a sequence set forth in SEQ ID NO: 19, and the antisense strand comprises or consists of a sequence set forth in SEQ ID NO: 28;
the sense strand comprises or consists of a sequence set forth in SEQ ID NO: 20, and the antisense strand comprises or consists of a sequence set forth in SEQ ID NO: 27;

the sense strand comprises or consists of a sequence set forth in SEQ ID NO: 20, and the antisense strand comprises or consists of a sequence set forth in SEQ ID NO: 28;

the sense strand comprises or consists of a sequence set forth in SEQ ID NO: 22, and the antisense strand comprises or consists of a sequence set forth in SEQ ID NO: 28;

the sense strand comprises or consists of a sequence set forth in SEQ ID NO: 5, and the antisense strand comprises or consists of a sequence set forth in SEQ ID NO: 18; or

the sense strand comprises or consists of a sequence set forth in SEQ ID NO: 19, and the antisense strand comprises or consists of a sequence set forth in SEQ ID NO: 32.

In some embodiments, an RNAi agent for inhibiting the expression of the ANGPTL3 gene is provided, comprising a sense strand and an antisense strand that form a complementary duplex region, wherein the sense strand and antisense strand pair to form any one of the duplex siRNAs described herein as Duplex 2002-010, 2002-011, 2002-018, 2002-034, 2008-010, 2008-011, 2008-018, 2008-019, and 2008-035.

The RNAi agent of the present invention may comprise a ligand. As used herein, a "ligand" refers to any compound or molecule capable of interacting, directly or indirectly, with another compound or molecule. The interaction of a ligand with another compound or molecule may elicit a biological response (e.g., initiation of a signal transduction cascade, induction of receptor-mediated endocytosis) or may simply be a physical attachment. A ligand may alter one or more properties of the attached double-stranded RNA molecule, such as the pharmacodynamics, pharmacokinetics, binding, absorption, cellular distribution, cellular uptake, charge, and/or clearance of the RNA molecule.

The ANGPTL3 gene is primarily expressed in the liver. Therefore, in certain embodiments, it is desirable to specifically deliver the RNAi agents of the present invention to hepatocytes. Accordingly, in certain embodiments, the ligand targets the specific delivery of the RNAi agent to hepatocytes using various methods described in more detail below. In certain embodiments, the RNAi agent is targeted to hepatocytes with a ligand that binds the surface-expressed asialoglycoprotein receptor (ASGR) or a component thereof (e.g., ASGR1, ASGR2).

In some embodiments, the RNAi agent can be specifically targeted to the liver by using a ligand that binds or interacts with a protein expressed on the surface of hepatocytes. For example, in certain embodiments, the ligand can comprise an antigen-binding protein (e.g., an antibody or binding fragment thereof such as Fab, scFv) that specifically binds a receptor expressed on hepatocytes, such as the asialoglycoprotein receptor and the LDL receptor. In one particular embodiment, the ligand comprises an antibody or binding fragment thereof that specifically binds ASGR1 and/or ASGR2. In another embodiment, the ligand comprises a Fab fragment of an antibody that specifically binds ASGR1 and/or ASGR2. In another embodiment, the ligand comprises a single-chain variable antibody fragment (scFv fragment) of an antibody that specifically binds ASGR1 and/or ASGR2. Exemplary antibodies that specifically bind ASGR1 and binding fragments thereof, which can serve as ligands for targeting the RNAi agents of the present invention to the liver, are described in WO 2017/058944, which is incorporated herein by reference in its entirety. Other antibodies or binding fragments thereof that specifically bind ASGR1, the LDL receptor, or other liver surface-expressed proteins suitable for use as ligands in the RNAi agents of the present invention are available from commercial sources.

In certain embodiments, the ligand comprises a carbohydrate. A "carbohydrate" refers to a compound composed of one or more monosaccharide units having at least 6 carbon atoms (which can be linear, branched, or cyclic) with oxygen, nitrogen, or sulfur atoms attached to each carbon atom. Carbohydrates include, but are not limited to, sugars (e.g., monosaccharides, disaccharides, trisaccharides, tetrasaccharides, and oligosaccharides containing about 4, 5, 6, 7, 8, or 9 monosaccharide units) and polysaccharides (such as starch, glycogen, cellulose, and polysaccharide gums). In some embodiments, the carbohydrate incorporated into the ligand is selected from pentoses, hexoses, or heptoses, and disaccharides and trisaccharides including such monosaccharide units. In other embodiments, the carbohydrate incorporated into the ligand is an amino sugar, such as galactosamine, glucosamine, N-acetylgalactosamine, and N-acetylglucosamine.

In some embodiments, the ligand comprises a hexose or a hexosamine. The hexose can be selected from glucose, galactose, mannose, fucose, or fructose. The hexosamine can be selected from fructosamine, galactosamine, glucosamine, or mannosamine. In certain embodiments, the ligand comprises glucose, galactose, galactosamine, or glucosamine. In one embodiment, the ligand comprises glucose, glucosamine, or N-acetylglucosamine. In another embodiment, the ligand comprises galactose, galactosamine, or N-acetylgalactosamine. In particular embodiments, ligands comprising glucose, galactose, and N-acetylgalactosamine (GalNAc) are particularly effective in targeting RNA to hepatocytes, as these ligands bind the ASGR expressed on the surface of hepatocytes. Examples of GalNAc- or galactose-containing ligands that can be incorporated into the RNAi agents of the present invention are described in U.S. Pat. Nos. 7,491,805, 8,106,022, and 8,877,917; U.S. Patent Application Publication No. US 20030130186; and WIPO Publication No. WO 2013/166155, all of which are incorporated herein by reference in their entirety.

In certain embodiments, the ligand comprises a multivalent carbohydrate moiety. As used herein, a "multivalent carbohydrate moiety" refers to a moiety comprising two or more carbohydrate units capable of independently binding or interacting with other molecules. For example, a multivalent carbohydrate moiety includes two or more binding domains

composed of carbohydrates that can bind to two or more different molecules or two or more different sites on the same molecule. The "valency" of a carbohydrate moiety indicates the number of individual binding domains within the carbohydrate moiety. For example, the terms "monovalent," "bivalent," "trivalent," and "tetravalent" relative to a carbohydrate moiety refer to carbohydrate moieties having one, two, three, and four binding domains, respectively. A multivalent carbohydrate moiety can comprise a multivalent lactose moiety, a multivalent galactose moiety, a multivalent glucose moiety, a multivalent N-acetylgalactosamine moiety, a multivalent N-acetylglucosamine moiety, a multivalent mannose moiety, or a multivalent fucose moiety. In some embodiments, the ligand comprises a multivalent galactose moiety. In other embodiments, the ligand comprises a multivalent N-acetylgalactosamine moiety. In these and other embodiments, the multivalent carbohydrate moiety can be bivalent, trivalent, or tetravalent. In such embodiments, the multivalent carbohydrate moiety can be bi-branched or tri-branched. In one particular embodiment, the multivalent N-acetylgalactosamine moiety is trivalent or tetravalent. In another particular embodiment, the multivalent galactose moiety is trivalent or tetravalent. Exemplary trivalent and tetravalent GalNAc-containing ligands for incorporation into the RNAi agents of the present invention are described in detail below.

The ligand can be attached or conjugated to the RNA molecule of the RNAi agent directly or indirectly. For example, in some embodiments, the ligand is directly covalently attached to the sense strand or the antisense strand of the RNAi agent. In other embodiments, the ligand is covalently attached to the sense strand or the antisense strand of the RNAi agent via a linker. The ligand can be attached to a nucleobase, sugar moiety, or internucleotide linkage of the sense strand or antisense strand of the RNAi agent of the present invention.

In some embodiments, the ligand can be attached to 3' or 5'-end of the sense strand or antisense strand. In certain embodiments, the ligand is covalently attached to 5'-end of the sense strand. In such embodiments, the ligand is attached to 5'-terminal nucleotide of the sense strand. In these and other embodiments, the ligand is attached at 5'-position of 5'-terminal nucleotide of the sense strand. In other embodiments, the ligand is covalently attached to 3'-end of the sense strand. For example, in some embodiments, the ligand is attached to 3'-terminal nucleotide of the sense strand. In certain such embodiments, the ligand is attached at 3'-position of 3'-terminal nucleotide of the sense strand. In alternative embodiments, the ligand is attached near 3'-end but prior to one or more terminal nucleotides (i.e., one, two, three, or four nucleotides from the terminus). In some embodiments, the ligand is attached at the 2'-position of the sugar of 3'-terminal nucleotide of the sense strand. In other embodiments, the ligand is attached at the 2'-position of the sugar of 5'-terminal nucleotide of the sense strand.

In certain embodiments, the ligand is attached to the sense strand or antisense strand via a linker. A "linker" refers to an atom or group of atoms that covalently attaches the ligand to the polynucleotide component of the RNAi agent. The linker can have a length of about 1 to about 30 atoms, about 2 to about 28 atoms, about 3 to about 26 atoms, about 4 to about 24 atoms, about 6 to about 20 atoms, about 7 to about 20 atoms, about 8 to about 20 atoms, about 8 to about 18 atoms, and about 12 to about 18 atoms. In some embodiments, the linker can comprise a bifunctional linking moiety, typically comprising an alkyl group with two functional groups. One functional group is chosen to bind to the compound of interest (e.g., the sense strand or antisense strand of the RNAi agent), while the other functional group is chosen to bind to essentially any selected group, such as the ligands described herein. In certain embodiments, the linker comprises an oligomer of a chain structure or repeating units, such as ethylene glycol or amino acid units. Examples of functional groups commonly used in bifunctional linking moieties include, but are not limited to, electrophiles for reaction with nucleophilic groups and nucleophiles for reaction with electrophilic groups. In some embodiments, the bifunctional linking moiety includes an amino group, a hydroxyl group, a carboxylic acid, a thiol, an unsaturated bond (e.g., a double or triple bond), and the like.

Linkers suitable for attaching a ligand to the sense strand or antisense strand of the RNAi agent of the present invention include, but are not limited to, pyrrolidine, 8-amino-3,6-dioxaoctanoic acid, succinimidyl-4-(N-maleimidomethyl) cyclohexane-1-carboxylate, 6-aminohexanoic acid, substituted $C_1$-$C_{10}$ alkyl, substituted or unsubstituted $C_2$-$C_{10}$ alkenyl, or substituted or unsubstituted $C_2$-$C_{10}$ alkynyl. Preferred substituents for such linkers include, but are not limited to, hydroxyl, amino, alkoxy, carboxyl, benzyl, phenyl, nitro, thiol, thioalkoxy, halogen, alkyl, aryl, alkenyl, and alkynyl.

In certain embodiments, the linker is cleavable. A cleavable linker is one that is sufficiently stable outside the cell but is cleaved to release the two parts linked together by the linker upon entry into a target cell. In some embodiments, the cleavable linker is cleaved at least 10, 20, 30, 40, 50, 60, 70, 80, 90 times or more, or at least 100 times faster in the target cell or under a first reference condition (which can, for example, be selected to mimic or represent intracellular conditions) than in the blood of a subject.

Cleavable linkers are susceptible to cleavage agents, such as pH, redox potential, or the presence of degradative molecules. Generally, cleavage agents are more prevalent or found at higher levels or activity inside cells than in serum or blood. Examples of such cleavage agents include: redox agents, selected for specific substrates or lacking substrate specificity, including, for example, oxidases or reductases present in cells; esterases; endosomes or agents that can create an acidic environment, such as those resulting in a pH of 5 or lower; enzymes that can hydrolyze or degrade acid-cleavable linkers acting as general acids, peptidases (which can be substrate-specific), and phosphatases.

A cleavable linker can comprise a pH-sensitive moiety. Human serum has a pH of 7.4, while the average

intracellular pH is slightly lower, ranging from about 7.1-7.3. Endosomes have a more acidic pH, in the range of 5.5-6.0, and lysosomes have an even more acidic pH (around 5.0). Some linkers will have a cleavable group that is cleaved at a preferred pH, thereby releasing the RNA molecule from the ligand inside the cell, or into a desired cellular organelle. The linker can include a cleavable group that is cleaved by a specific enzyme. The type of cleavable group incorporated into the linker can depend on the cell to be targeted. For example, a liver-targeting ligand can be linked to the RNA molecule via a linker comprising an ester group. Hepatocytes are rich in esterases, so the linker is cleaved more efficiently in hepatocytes than in cell types not rich in esterases. Other cell types rich in esterases include cells of the lung, renal cortex, and testis. When targeting cells rich in peptidases, such as hepatocytes and synoviocytes, linkers containing peptide bonds can be used.

Other types of linkers suitable for attaching ligands to the sense strand or antisense strand in the RNAi agents of the present invention are known in the art, such as those described in U.S. Pat. Nos. 7,723,509, 8,017,762, 8,828,956, 8,877,917, and 9,181,551, all of which are incorporated herein by reference in their entirety.

In certain embodiments, the ligand covalently attached to the sense strand or antisense strand of the RNAi agent of the present invention comprises a GalNAc moiety, such as a multivalent GalNAc. In some embodiments, the multivalent GalNAc moiety is a trivalent GalNAc and is attached to 3'-end of the sense strand. In other embodiments, the multivalent GalNAc moiety is a trivalent GalNAc and is attached to 5'-end of the sense strand. In other embodiments, the multivalent GalNAc moiety is a tetravalent GalNAc moiety and is attached to 3'-end of the sense strand. In other embodiments, the multivalent GalNAc moiety is a tetravalent GalNAc moiety and is attached to 5'-end of the sense strand.

In some embodiments, the ligand comprised in the RNAi agent of the present invention comprises a structure of formula (I):

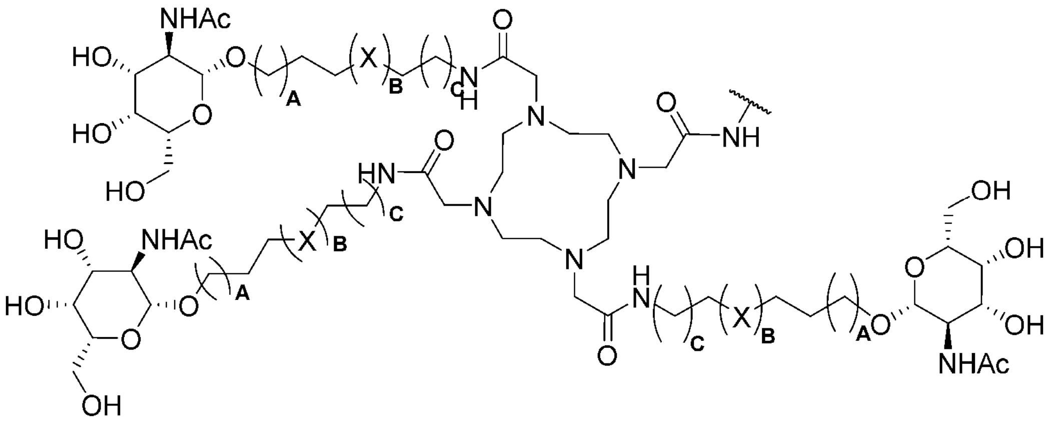

(I)

wherein,

- X is -C(O)-NH-, -NH-C(O)-, $-OCH_2-CH_2O-$, -S-, or -S-S-;
- A or C is independently 0 or an integer from 1 to 5;
- B is 0 or an integer from 1 to 3;
- the wave line represents a site to which other part of the RNAi agent is attached, wherein the ligand is connected to 5' and/or 3'-end of the sense strand and/or antisense strand; preferably, the ligand is connected to 5' and/or 3'-end of the sense strand; more preferably, the ligand is connected to 3'-end of the sense strand.

In a preferred embodiment, the ligand is covalently attached to 5' and/or 3'-end of the sense strand via a linker. In one embodiment, the linker is a PEG linker.

In preferred embodiments, the ligand has a structure of formula (II) or formula (III):

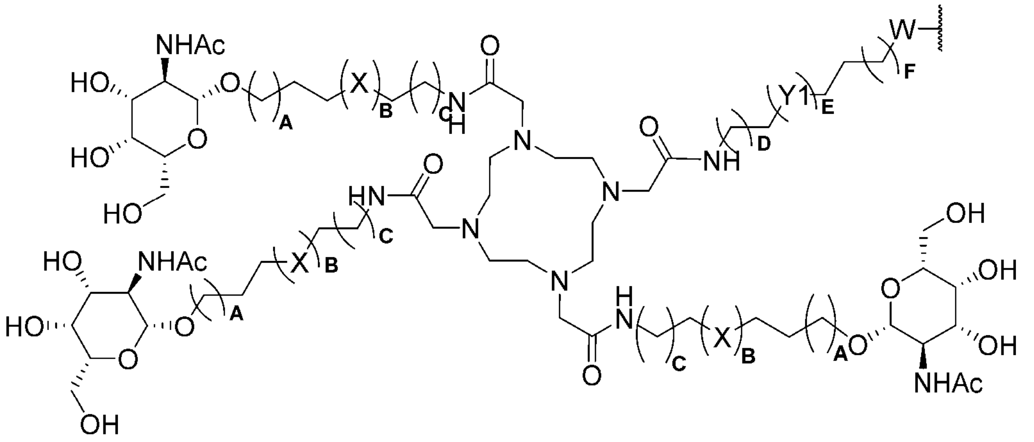

(II)

wherein,

X is -C(O)-NH-, -NH-C(O)-, $-OCH_2-CH_2O-$, -S-, or -S-S-;
Y1 is $-C(O)-NR_1-$, -NH-C(O)-, $-OCH_2-CH_2O-$, -S-S-,

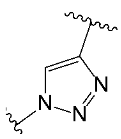

or

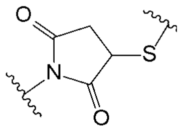

,

wherein $R_1$ is an alkyl group having 1-6 carbons or hydrogen;
W is -NH-, -O- or

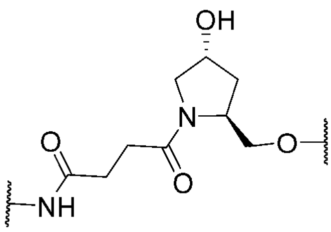

,

wherein the wave line on the right represents a site to which the sense strand or antisense strand is attached;
the wave line connected to W in formula (II) represents a site to which the sense strand or antisense strand is attached;
A, C, or F is 0 or an integer from 1 to 5;
B or E is 0 or an integer from 1 to 3;
D is an integer from 1 to 6;

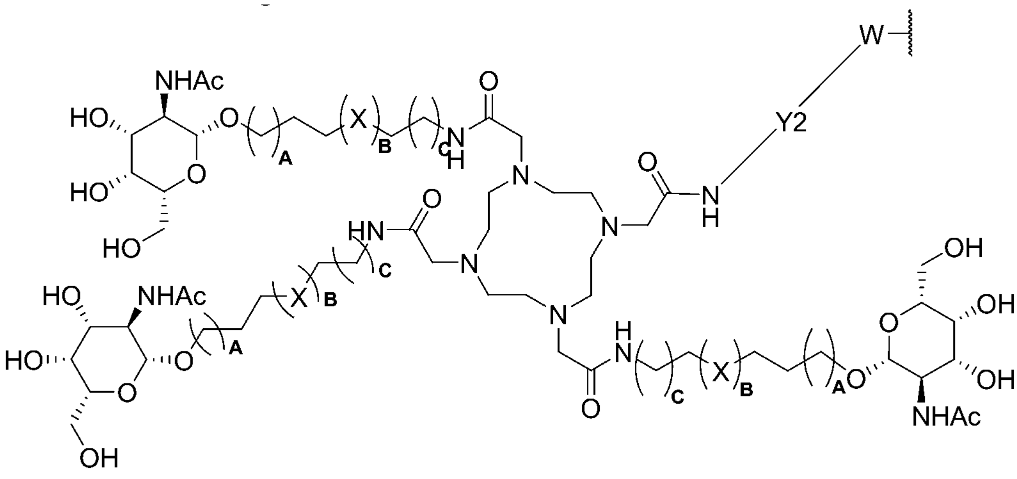

(III)

wherein,

X is -C(O)-NH-, -NH-C(O)-, $-OCH_2-CH_2O-$, -S-, or -S-S-;

Y2 is $-(CH_2)p-(O-CH_2-CH_2)q-(CH_2)j-Z_3-$, wherein $Z_3$ is O, NH or C(O), p is an integer from 1 to 3, q is an integer from 3 to 10; j is 0 or 1; W is absent, or is

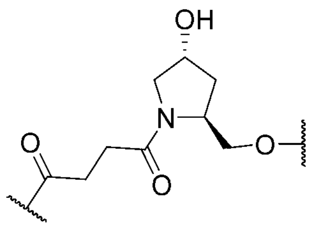

or

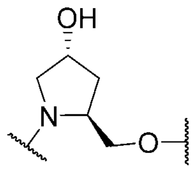

,

wherein the wave line on the right represents a site to which the sense strand or antisense strand is attached;

the wave line connected to W in formula (III) represents a site to which the sense strand or antisense strand is attached;

A or C is 0 or an integer from 1 to 5;

B is 0 or an integer from 1 to 3.

Preferably, exemplary trivalent and/or tetravalent GalNAc ligands that can be attached to the double-stranded RNA molecule in the RNAi agent of the present invention are provided in formulae (IV) or (V) below:

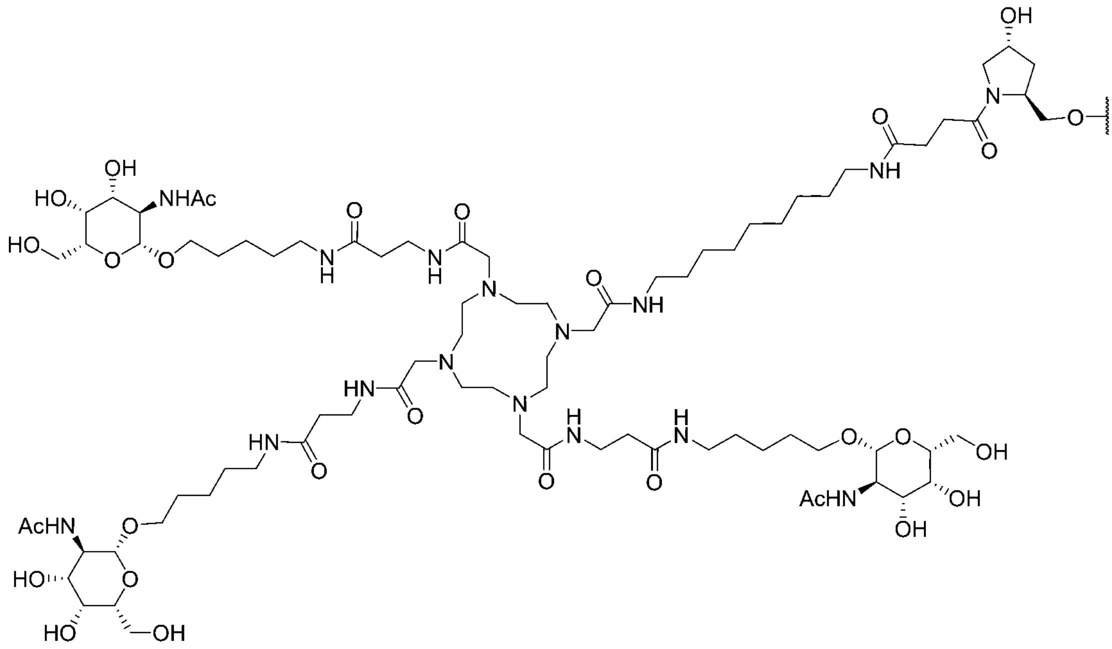

(IV)

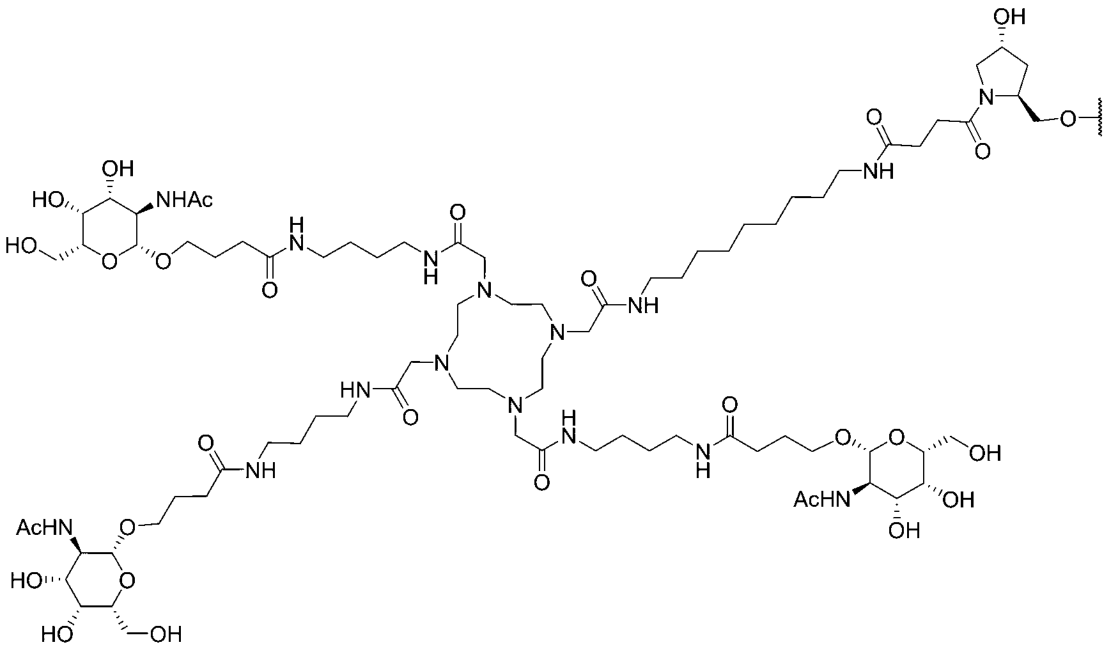

(V)

wherein the wave line represents a site to which the sense strand or antisense strand is attached; preferably, the ligand is connected to 5' and/or 3'-end of the sense strand; more preferably, the ligand is connected to 3'-end of the sense strand; wherein the targeting ligand is connected to the sense strand or antisense strand via a phosphate ester group, a thiophosphate ester group or a phosphonate ester group.

In some embodiments, the RNAi agent of the present invention is further complexed with a metal cation, preferably a monovalent metal cation such as $Na^+$, $K^+$, or $Ag^+$; a divalent metal cation such as $Ca^{2+}$, $Mg^{2+}$, $Cu^{2+}$, $Fe^{2+}$, $Zn^{2+}$, or $Mn^{2+}$; or a trivalent metal cation such as $Fe^{3+}$, $Al^{3+}$, or a lanthanide metal ion. In some embodiments, the metal cation is complexed with the nitrogen-containing heterocyclic structure in any one of formulae (II) to (V).

For example, in one embodiment, the RNAi agent provided by the present invention has a structure as shown below, wherein $K^+$ is complexed to the nitrogen-containing heterocyclic structure of the ligand, and wherein 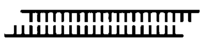 represents the duplex portion of the RNAi agent for ANGPTL3 gene expression:

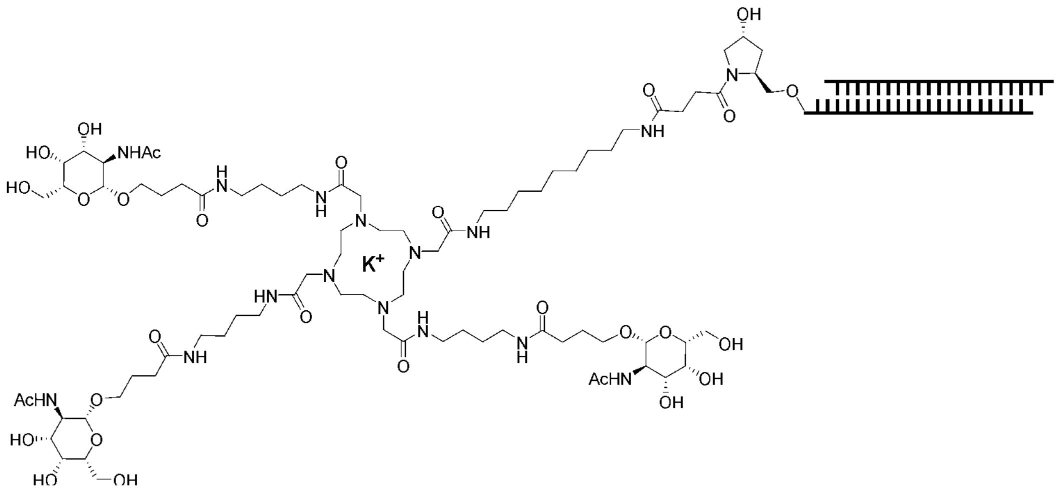

For convenience of description, the ligand represented by formula (IV) is referred to herein by the code name YHZY12001, and the ligand represented by formula (V) is referred to herein by the code name YHZY12002. RNAi agents with a ligand of formula (I), (II), or (III) attached to 5' and/or 3'-end of the sense strand are referred to herein simply as MCPP-siRNA.

In some embodiments, the present invention provides an RNAi agent for inhibiting the expression of the ANGPTL3 gene, wherein:

the sense strand comprises or consists of a sequence set forth in SEQ ID NO: 33, and the antisense strand comprises or consists of a sequence set forth in SEQ ID NO: 13;
the sense strand comprises or consists of a sequence set forth in SEQ ID NO: 34, and the antisense strand comprises or consists of a sequence set forth in SEQ ID NO: 13;
the sense strand comprises or consists of a sequence set forth in SEQ ID NO: 38, and the antisense strand comprises or consists of a sequence set forth in SEQ ID NO: 13;
the sense strand comprises or consists of a sequence set forth in SEQ ID NO: 39, and the antisense strand comprises or consists of a sequence set forth in SEQ ID NO: 13;
the sense strand comprises or consists of a sequence set forth in SEQ ID NO: 35, and the antisense strand comprises or consists of a sequence set forth in SEQ ID NO: 27;
the sense strand comprises or consists of a sequence set forth in SEQ ID NO: 36, and the antisense strand comprises or consists of a sequence set forth in SEQ ID NO: 27;
the sense strand comprises or consists of a sequence set forth in SEQ ID NO: 40, and the antisense strand comprises or consists of a sequence set forth in SEQ ID NO: 27;
the sense strand comprises or consists of a sequence set forth in SEQ ID NO: 41, and the antisense strand comprises or consists of a sequence set forth in SEQ ID NO: 27;
the sense strand comprises or consists of a sequence set forth in SEQ ID NO: 35, and the antisense strand comprises or consists of a sequence set forth in SEQ ID NO: 28;
the sense strand comprises or consists of a sequence set forth in SEQ ID NO: 36, and the antisense strand comprises or consists of a sequence set forth in SEQ ID NO: 28;
the sense strand comprises or consists of a sequence set forth in SEQ ID NO: 37, and the antisense strand comprises or consists of a sequence set forth in SEQ ID NO: 28;
the sense strand comprises or consists of a sequence set forth in SEQ ID NO: 40, and the antisense strand comprises or consists of a sequence set forth in SEQ ID NO: 28;
the sense strand comprises or consists of a sequence set forth in SEQ ID NO: 41, and the antisense strand comprises or consists of a sequence set forth in SEQ ID NO: 28; or
the sense strand comprises or consists of a sequence set forth in SEQ ID NO: 42, and the antisense strand comprises or consists of a sequence set forth in SEQ ID NO: 28.

In a particularly preferred embodiment, the present invention provides a double-stranded RNAi agent for inhibiting the expression of ANGPTL3 in a cell, wherein the double-stranded RNAi agent comprises a sense strand and an antisense strand that form a duplex region, wherein the sense strand comprises SEQ ID NO: 19: 5'-mG*mA*mGmAfAmGfAfAfCmUmAmCmAmUmAmUmAmAmA-3' and the sense strand is no more than 21 nucleotides in length, the antisense strand comprises SEQ ID NO: 27: 5'-mU*fU*mUmAfUmAfUmGmUmAmGmUmUfCmUfUmCmUmC*-

mA*mG-3' and is no more than 23 nucleotides in length,

wherein mA, mU, mG, and mC are 2'-OMe A, 2'-OMe U, 2'-OMe G, and 2'-OMe C, respectively; fA, fU, fG, and fC are 2'-F A, 2'-F U, 2'-F G, and 2'-F C, respectively; * is a phosphorothioate linkage;
wherein 3'-end of the sense strand is conjugated to a ligand that targets a hepatocyte; preferably, the ligand comprises a galactose moiety, a galactosamine moiety, or an N-acetylgalactosamine moiety; more preferably, the ligand is a trivalent or tetravalent N-acetylgalactosamine moiety.

In the aforementioned particularly preferred embodiment, the ligand can have the structure of formula (IV):

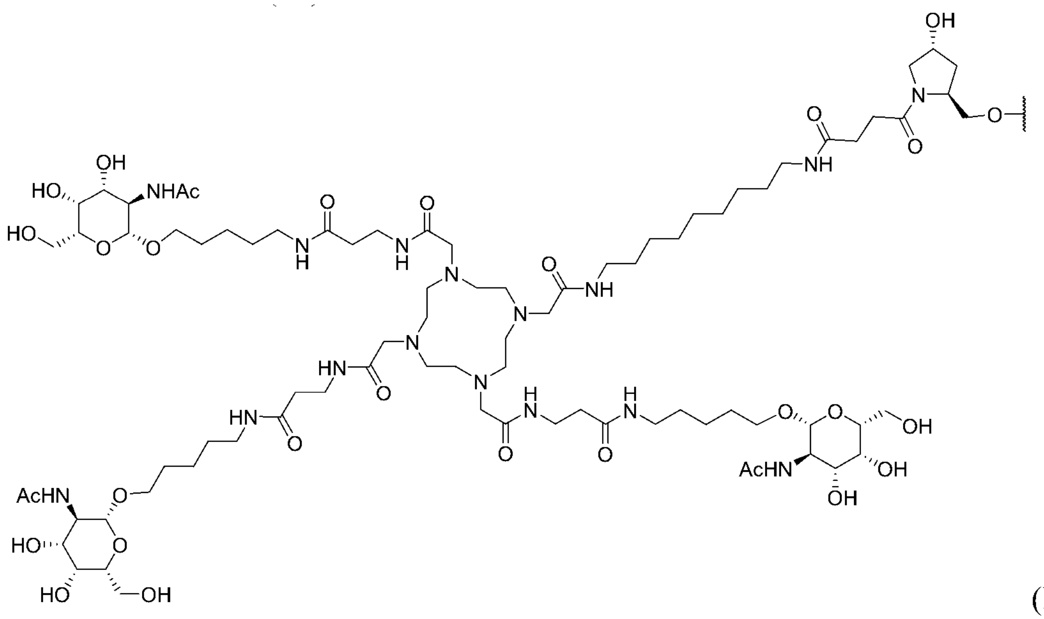

(IV),

and is connected to the sense strand via a phosphate ester group or a thiophosphate ester group.

In a particularly preferred embodiment, the present invention provides a double-stranded RNAi agent for inhibiting the expression of ANGPTL3 in a cell, wherein the double-stranded RNAi agent comprises a sense strand and an antisense strand that form a duplex region, wherein the sense strand comprises SEQ ID NO: 40: 5'-mG*mA*mGmAfAmGfAfAfCmUmAmCmAmUmAmUmAmAmA-YHZY12001-3' and the sense strand is no more than 21 nucleotides in length, the antisense strand comprises SEQ ID NO: 27: 5'-mU*fU*mUmAfUmAfUmGmUmAmGmUmUfCmUfUmCmUmC*mA*mG-3' and is no more than 23 nucleotides in length,

wherein mA, mU, mG, and mC are 2'-OMe A, 2'-OMe U, 2'-OMe G, and 2'-OMe C, respectively; fA, fU, fG, and fC are 2'-F A, 2'-F U, 2'-F G, and 2'-F C, respectively; * is a phosphorothioate linkage,
wherein YHZY12001 has the structure of formula (IV) and is conjugated to 3'-end of the sense strand as follows:

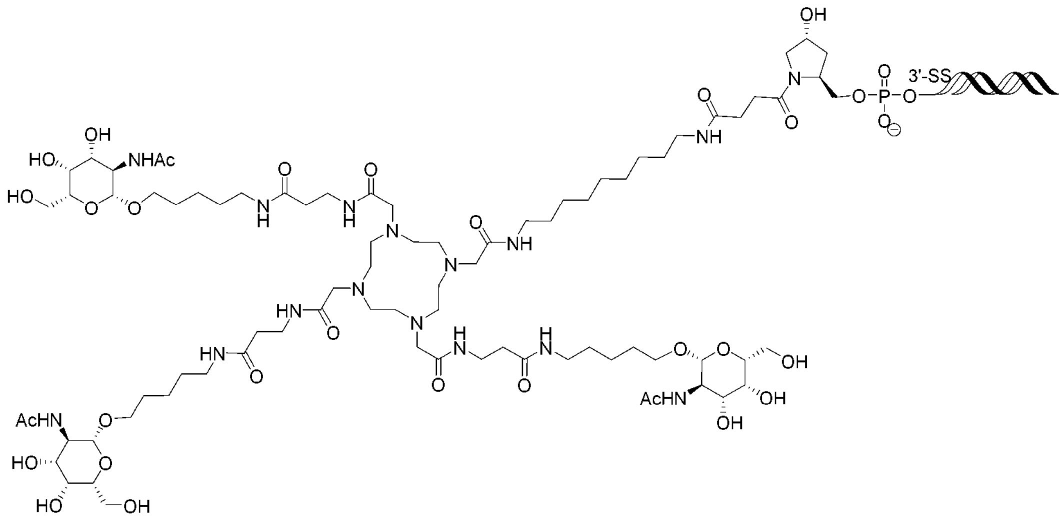

.

## Pharmaceutical Compositions

The present invention also encompasses pharmaceutical compositions and formulations comprising the RNAi agents described herein and a pharmaceutically acceptable carrier, excipient, or diluent. Such compositions and formulations can be used to reduce the expression of the ANGPTL3 gene in a patient in need thereof. Considering clinical application, the pharmaceutical compositions and formulations will be prepared in a form suitable for the intended application. Typically, this will involve preparing compositions substantially free of pyrogens and other impurities that may be harmful to humans or animals.

The ingredients and methods for formulating pharmaceutical compositions depend on many criteria, including, but not limited to, the route of administration, the type and extent of the disease or condition to be treated, or the dosage to be administered. In some embodiments, the pharmaceutical composition is formulated based on the intended delivery route. For example, in certain embodiments, the pharmaceutical composition is formulated for parenteral delivery. Parenteral administration forms include intravenous, intra-arterial, subcutaneous, intrathecal, intraperitoneal, or intramuscular injection or infusion. In one embodiment, the pharmaceutical composition is formulated for intravenous delivery. In such embodiments, the pharmaceutical composition may comprise a lipid-based delivery vehicle. In another embodiment, the pharmaceutical composition is formulated for subcutaneous delivery. In such embodiments, the pharmaceutical composition may comprise a targeting ligand (e.g., a GalNAc-containing or antibody-containing ligand as described herein).

In some embodiments, the pharmaceutical composition comprises an effective amount of the RNAi agent described herein. An "effective amount" refers to an amount sufficient to produce a beneficial or desired clinical outcome. In some embodiments, the effective amount is an amount sufficient to reduce the expression of the ANGPTL3 gene in a specific tissue or cell type of the patient (e.g., the liver or hepatocytes). The effective amount of the RNAi agent of the present invention can be from about 0.01 mg/kg body weight to about 100 mg/kg body weight, and can be administered daily, weekly, monthly, or at longer intervals. The precise determination of the specific effective dosage amount and administration frequency may be based on several factors, including the patient's body size, age, and general condition, the type of disease to be treated (e.g., myocardial infarction, coronary artery disease, peripheral artery disease, stroke), the specific RNAi agent used, and the route of administration.

Administration of the pharmaceutical compositions of the invention can be by any common route provided the target tissue is accessible via that route. These routes include, but are not limited to, parenteral (e.g., subcutaneous, intramuscular, intraperitoneal, or intravenous), oral, nasal, buccal, intradermal, transdermal, and sublingual routes, or by direct injection into liver tissue or delivery via the hepatic portal vein. In some embodiments, the pharmaceutical composition is administered parenterally. For example, in certain embodiments, the pharmaceutical composition is administered intravenously. In other embodiments, the pharmaceutical composition is administered subcutaneously.

Colloidal dispersion systems can be used as delivery vehicles for the RNAi agents of the present invention, such as macromolecular complexes, nanocapsules, microspheres, beads, and lipid-based systems, including oil-in-water emulsions, micelles, mixed micelles, and liposomes. Commercially available fat emulsions suitable for delivering the nucleic acids of the present invention include Intralipid® (Baxter International Inc.), Liposyn® (Abbott Pharmaceuticals), Lipsyn®II (Hospira), Liposyn®III (Hospira), Nutrilipid (B. Braun Medical Inc.), and other similar fat emulsions. A preferred colloidal system for use as an in vivo delivery vehicle is the liposome (i.e., an artificial membrane vesicle). The RNAi agents of the present invention can be encapsulated within liposomes or can form complexes therewith, particularly with cationic

liposomes. Alternatively, the RNAi agents of the present invention can be complexed with lipids, particularly with cationic lipids. Suitable cationic lipids are, for example, dioleoyltrimethylammonium propane (DOTAP) and dioleoylphosphatidylethanolamine (DOTMA).

Pharmaceutical compositions suitable for injectable use comprise, for example, sterile aqueous solutions or dispersions and sterile powders for the extemporaneous preparation of sterile injectable solutions or dispersions. Generally, such formulations are sterile and possess sufficient fluidity for ease of syringeability. The formulations shall remain stable under manufacturing and storage conditions and shall be preserved against the contaminating action of microorganisms such as bacteria and fungi. Suitable solvents or dispersion media may include, for instance, water, ethanol, polyhydric alcohols (e.g., glycerol, propylene glycol, liquid polyethylene glycols, and the like), suitable mixtures thereof, and vegetable oils. Appropriate fluidity may be maintained, for example, by the use of a coating material such as lecithin, by maintaining required particle size in dispersions, or by employing surfactants. Prevention of microbial action may be achieved through various antibacterial and antifungal agents, including but not limited to parabens, chlorobutanol, phenol, sorbic acid, thimerosal, and the like. In many cases, it is preferable to include isotonic agents, such as sugars or sodium chloride. Prolonged absorption of injectable compositions may be accomplished by incorporating agents delaying absorption in the composition, for example, aluminum monostearate and gelatin.

Sterile injectable solutions may be prepared by incorporating the requisite quantity of the active compound with any additional ingredients (e.g., those enumerated supra) into an appropriate solvent, followed by filtration sterilization. Generally, dispersions are prepared by incorporating various sterilized active ingredients into a sterile vehicle medium containing essential dispersion agents and other requisite components, for instance, as described above. In the case of sterile powders for preparing sterile injectable solutions, preferred preparation methods include vacuum desiccation and lyophilization techniques, which yield powders of the active ingredient(s) together with any additional desired constituents from their prior sterile-filtered solutions.

The compositions of the present invention may generally be formulated in neutral form or as pharmaceutically acceptable salts. Such salts include, for example, acid addition salts (formed with free amino groups) derived from inorganic acids (e.g., hydrochloric or phosphoric acids) or organic acids (e.g., acetic, oxalic, tartaric, mandelic acids, etc.). Salts formed with free carboxyl groups may likewise be derived from inorganic bases (e.g., sodium, potassium, ammonium, calcium, or ferric hydroxides) or organic bases (e.g., isopropylamine, trimethylamine, histidine, procaine, etc.). In some embodiments, the RNAi agent of the invention is formulated as a sodium salt.

For parenteral administration in aqueous solution form, for example, the solution is typically appropriately buffered, and the liquid diluent is first rendered isotonic with sufficient saline or glucose, for instance. Such aqueous solutions may be employed for intravenous, intramuscular, subcutaneous, and intraperitoneal administration, among others. Preferably, sterile aqueous media are utilized. By way of example, a unit dose may be dissolved in 1 mL of isotonic NaCl solution and either added to 1000 mL of subcutaneous infusion fluid or injected at the proposed infusion site. For administration to humans, the formulations shall comply with sterility, pyrogenicity, general safety, and purity standards as required by local Food and Drug Administration regulations. In certain embodiments, the pharmaceutical composition of the invention comprises the sterile saline solution described herein and the RNAi agent, or consists essentially of these components. In other embodiments, the pharmaceutical composition of the invention comprises the RNAi agent described herein and sterile water (e.g., Water for Injection, WFI), or consists essentially thereof. In yet other embodiments, the pharmaceutical composition of the invention comprises the RNAi agent described herein and Phosphate-Buffered Saline (PBS), or consists essentially thereof.

In some embodiments, the pharmaceutical compositions of the present invention are packaged with or stored within an administration device. Devices for injectable formulations comprise, but are not limited to, injection ports, pre-filled syringes, auto-injectors, infusion pumps, implantable injectors, and injection pens. Devices for nebulized or powdered formulations comprise, but are not limited to, inhalers, insufflators, nebulizers, and the like. Accordingly, the present invention encompasses an administration device containing the pharmaceutical composition of the invention, for treating or preventing one or more diseases or disorders as described herein.

## Therapeutic Methods and Uses

The present invention provides a method for reducing or inhibiting the expression of the ANGPTL3 gene in a cell (e.g., a hepatocyte) by contacting the cell with any of the RNAi agents described herein. The cell can be in vitro or in vivo. ANGPTL3 gene expression can be assessed by measuring the amount or level of ANGPTL3 mRNA or another biomarker associated with ANGPTL3 expression, such as serum ANGPTL3 protein, triacylglycerols, LDL-C, or HDL-C. The reduction in ANGPTL3 expression in cells or animals treated with the RNAi agents of the invention can be determined relative to ANGPTL3 expression in cells and animals not treated with the RNAi agent or treated with a control RNAi agent. For example, in some embodiments, the reduction in ANGPTL3 expression is assessed by (a) measuring the amount or level of ANGPTL3 mRNA in hepatocytes treated with an RNAi agent of the invention, (b) measuring the amount or level of ANGPTL3 mRNA in hepatocytes treated with a control RNAi agent (e.g., an RNAi agent targeting an RNA molecule not

expressed in hepatocytes or an RNAi agent having a nonsense or scrambled sequence) or no RNAi agent, and (c) comparing the ANGPTL3 mRNA level measured in the treated cells of (a) with the ANGPTL3 mRNA level in the control cells of (b). The ANGPTL3 mRNA levels in the treated and control cells may be normalized to the RNA level of a control gene (e.g., 18S ribosomal RNA or a housekeeping gene) prior to comparison. ANGPTL3 mRNA levels can be measured by various methods, including Northern blot analysis, nuclease protection assay, fluorescent in situ hybridization (FISH), reverse transcriptase (RT)-PCR, real-time RT-PCR, quantitative PCR, droplet digital PCR, and the like.

In some embodiments, the method for assessing ANGPTL3 expression levels is performed in vitro in cells that naturally express the ANGPTL3 gene (e.g., hepatocytes) or cells that have been engineered to express ANGPTL3. In certain embodiments, the method is performed in vitro in hepatocytes. Suitable hepatocytes include, but are not limited to, primary hepatocytes (e.g., human or non-human primate hepatocytes), HepAD38 cells, HuH-6 cells, HuH-7 cells, HuH-5-2 cells, BNLCL2 cells, Hep3B cells, or HepG2 cells. In one embodiment, the hepatocyte is a HuH-7 cell. In another embodiment, the hepatocyte is a human primary hepatocyte.

In other embodiments, the method for assessing ANGPTL3 expression levels is performed in vivo. The RNAi agent and any control RNAi agent can be administered to an animal (e.g., a transgenic animal expressing the ANGPTL3 gene or a non-human primate), and ANGPTL3 mRNA or serum ANGPTL3 protein levels are assessed in liver tissue harvested from the animal post-treatment. Alternatively or additionally, biomarkers or functional phenotypes associated with ANGPTL3 expression can be assessed in the treated animal. For example, ANGPTL3 protein is the expression product of ANGPTL3 mRNA in serum or plasma. Therefore, the serum or plasma levels of ANGPTL3 protein can be measured in an animal treated with an RNAi agent of the invention to assess the functional efficacy of reducing ANGPTL3 expression.

In certain embodiments, the expression of ANGPTL3 in hepatocytes is reduced by the RNAi agent of the invention by at least 40%, at least 45%, or at least 50%. In some embodiments, the expression of ANGPTL3 in hepatocytes is reduced by the RNAi agent of the invention by at least 60%, at least 65%, at least 70%, at least 75%, at least 80%, or at least 85%. In other embodiments, the expression of ANGPTL3 in hepatocytes is reduced by the RNAi agent of the invention by about 90% or more, e.g., 91%, 92%, 93%, 94%, 95%, 96%, 97%, 98%, 99%, or more. The percentage reduction in ANGPTL3 expression can be measured by any of the methods described herein, as well as other methods known in the art.

The present invention provides methods for reducing or inhibiting the expression of the ANGPTL3 gene, thereby reducing or inhibiting the production of ANGPTL3 protein, in a patient in need thereof, as well as methods for treating or preventing a disease or disorder associated with ANGPTL3 expression or activity. A "disease or disorder associated with ANGPTL3 expression" refers to a disease or disorder characterized by altered levels of ANGPTL3 expression, or a condition where elevated levels of ANGPTL3 expression are associated with an increased risk of developing the disease or disorder. Diseases or disorders associated with ANGPTL3 expression may also include those caused by abnormal changes in lipoprotein metabolism, such as changes leading to abnormal or elevated levels of ANGPTL3 protein, cholesterol, lipids, triglycerides, etc., or impaired clearance of these molecules. In certain embodiments, the RNAi agents of the invention are particularly useful for treating or preventing metabolic diseases and reducing circulating levels of ANGPTL3.

Diseases and disorders associated with ANGPTL3 expression that can be treated or prevented according to the methods of the invention include, but are not limited to, metabolic diseases such as hypertriglyceridemia, obesity, hyperlipidemia, abnormal lipid and/or cholesterol metabolism, atherosclerosis, Type 2 diabetes mellitus, cardiovascular disease, coronary artery disease, non-alcoholic steatohepatitis, non-alcoholic fatty liver disease, homozygous or heterozygous familial hypercholesterolemia, and statin-resistant hypercholesterolemia.

In certain embodiments, the present invention provides a method for reducing ANGPTL3 expression in a patient in need thereof, comprising administering to the patient any RNAi agent described herein. Preferably, the level of ANGPTL3 expression in the patient's hepatocytes is reduced following administration of the RNAi agent, compared to the level of ANGPTL3 expression in a patient not receiving the RNAi agent, or compared to the level of ANGPTL3 expression in the patient prior to administration of the RNAi agent. In some embodiments, after administration of the RNAi agent of the invention, ANGPTL3 expression in the patient is reduced by at least 30%, at least 35%, at least 40%, at least 45%, at least 50%, at least 55%, at least 60%, at least 65%, at least 70%, at least 75%, at least 80%, at least 85%, or at least 90%, e.g., 91%, 92%, 93%, 94%, 95%, 96%, 97%, 98%, or 99%. The percentage reduction in ANGPTL3 expression can be measured by any of the methods described herein, as well as other methods known in the art. In certain embodiments, the percentage reduction in ANGPTL3 expression is determined by assessing the level of ANGPTL3 protein in the patient's serum or plasma according to the methods described herein.

In some embodiments, the patient's serum or plasma triglyceride (TG) level is reduced after administration of the RNAi agent by at least about 10%, 20%, 30%, 35%, 40%, 45%, 50%, 55%, 60%, 65%, 70%, 75%, 80%, 85%, 90%, 95%, 96%, 97%, 98%, 99%, or greater than 99%, compared to the level in a patient not receiving the RNAi agent or compared to the level in the patient prior to administration of the RNAi agent.

In some embodiments, the patient's total cholesterol level is reduced after administration of the RNAi agent by at

least about 10%, 20%, 30%, 35%, 40%, 45%, 50%, 55%, 60%, 65%, 70%, 75%, 80%, 85%, 90%, 95%, 96%, 97%, 98%, 99%, or greater than 99%, compared to the level in a patient not receiving the RNAi agent or compared to the level in the patient prior to administration of the RNAi agent.

In some embodiments, the patient's low-density lipoprotein cholesterol (LDL-C) level is reduced after administration of the RNAi agent by at least about 10%, 20%, 30%, 35%, 40%, 45%, 50%, 55%, 60%, 65%, 70%, 75%, 80%, 85%, 90%, 95%, 96%, 97%, 98%, 99%, or greater than 99%, compared to the level in a patient not receiving the RNAi agent or compared to the level in the patient prior to administration of the RNAi agent.

In certain embodiments, the patient in need of reducing ANGPTL3 expression is a patient diagnosed with or at risk of a metabolic disease. Accordingly, the present invention includes a method for treating or preventing a metabolic disease in a patient in need thereof by administering any RNAi agent of the invention. In some embodiments, the present invention includes the use of any RNAi agent described herein in the manufacture of a medicament for treating or preventing a metabolic disease in a patient in need thereof. In other embodiments, the present invention provides an ANGPTL3-targeting RNAi agent for use in a method of treating or preventing a metabolic disease in a patient in need thereof. Metabolic diseases include, but are not limited to, hypertriglyceridemia, obesity, hyperlipidemia, abnormal lipid and/or cholesterol metabolism, atherosclerosis, Type 2 diabetes mellitus, cardiovascular disease, coronary artery disease, non-alcoholic steatohepatitis, non-alcoholic fatty liver disease, homozygous or heterozygous familial hypercholesterolemia, and statin-resistant hypercholesterolemia. In some embodiments, the disease to be treated or prevented according to the method of the invention is hypertriglyceridemia. In other embodiments, the disease to be treated or prevented is obesity. In other embodiments, the disease to be treated or prevented is hyperlipidemia. In other embodiments, the disease to be treated or prevented is abnormal lipid metabolism. In other embodiments, the disease to be treated or prevented is abnormal cholesterol metabolism. In other embodiments, the disease to be treated or prevented is abnormal lipid and cholesterol metabolism. In other embodiments, the disease to be treated or prevented is atherosclerosis. In other embodiments, the disease to be treated or prevented is type II diabetes. In other embodiments, the disease to be treated or prevented is cardiovascular disease. In other embodiments, the disease to be treated or prevented is coronary artery disease. In other embodiments, the disease to be treated or prevented is non-alcoholic steatohepatitis. In other embodiments, the disease to be treated or prevented is non-alcoholic fatty liver disease. In other embodiments, the disease to be treated or prevented is homozygous or heterozygous familial hypercholesterolemia. In other embodiments, the disease to be treated or prevented is statin-resistant hypercholesterolemia.

In certain embodiments, administration of the RNAi agents described herein reduces the risk of non-fatal myocardial infarction, fatal and non-fatal stroke, certain types of heart surgery (e.g., angioplasty, bypass surgery), hospitalization for heart failure, chest pain in patients with heart disease, and/or cardiovascular events in patients with established heart disease (e.g., prior myocardial infarction, prior heart surgery, and/or chest pain with evidence of arterial blockage). In some embodiments, administration of the RNAi agents described herein according to the methods of the invention can be used to reduce the risk of recurrent cardiovascular events.

**In** certain other embodiments, the patient in need of reducing ANGPTL3 expression is a patient with elevated circulating ANGPTL3 levels. Accordingly, in some embodiments, the present invention provides a method for reducing the serum or plasma level of ANGPTL3 protein in a patient in need thereof by administering to the patient any RNAi agent described herein. In some embodiments, the present invention includes the use of any RNAi agent described herein in the manufacture of a medicament for reducing the serum or plasma level of ANGPTL3 protein in a patient in need thereof. In other embodiments, the present invention provides an ANGPTL3-targeting RNAi agent for use in a method of reducing the serum or plasma level of ANGPTL3 protein in a patient in need thereof.

## Sequence Listings

**Table 1. Nucleotide-unmodified ANGPTL3 siRNAs**

| Duplex | SEQ ID NO. | Sequence (5'-3') (Sense strand listed first for each duplex, antisense strand second) |
|---|---|---|
| 2002 | 1 | CAUAGUGAAGCAAUCUAAU |
| | 3 | AUUAGAUUGCUUCACUAUGGA |
| 2008 | 2 | GAGAAGAACUACAUAUAAA |
| | 4 | UUUAUAUGUAGUUCUUCUCAG |

**Table 2. Nucleotide-modified ANGPTL3 siRNAs**

| Duplex Name | SEQ ID NO | Sequence (5'-3') (Sense strand listed first for each duplex, antisense strand second) |
|---|---|---|
| 2002-004 | 5 | mC*mA*mUmAfGmUfGfAfAmGmCmAmAmUmCmUmAmAmU |
| | 10 | mA*fU*mUmAmGmAmUmUmGmCmUmUmCfAmCfUmAmUmG*mG*m A |
| 2002-005 | 5 | mC*mA*mUmAfGmUfGfAfAmGmCmAmAmUmCmUmAmAmU |
| | 11 | mA*fU*mUmAmGfAmUmUmGmCmUmUmCfAmCmUmAmUmG*mG*m A |
| 2002-009 | 5 | mC*mA*mUmAfGmUfGfAfAmGmCmAmAmUmCmUmAmAmU |
| | 12 | mA*fU*mUmAmGfAmUmUmGmCmUfUmCfAmCfUmAmUmG*mG*mA |
| 2002-010 | 5 | mC*mA*mUmAfGmUfGfAfAmGmCmAmAmUmCmUmAmAmU |
| | 13 | mA*fU*mUmAfGmAfUmUmGmCmUmUmCfAmCfUmAmUmG*mG*mA |
| 2002-011 | 5 | mC*mA*mUmAfGmUfGfAfAmGmCmAmAmUmCmUmAmAmU |
| | 14 | mA*fU*mUmAfGmAfUmUmGmCmUfUmCfAmCmUmAmUmG*mG*mA |
| 2002-006 | 5 | mC*mA*mUmAfGmUfGfAfAmGmCmAmAmUmCmUmAmAmU |
| | 15 | mA*mU*mUmAfGmAmUmUmGmCmUfUmCfAmCmUmAmUmG*mG*m A |
| 2002-007 | 5 | mC*mA*mUmAfGmUfGfAfAmGmCmAmAmUmCmUmAmAmU |
| | 16 | mA*mU*mUmAmGfAmUmUmGmCmUfUmCfAmCmUmAmUmG*mG*m A |
| 2002-008 | 5 | mC*mA*mUmAfGmUfGfAfAmGmCmAmAmUmCmUmAmAmU |
| | 17 | mA*mU*mUmAmGmAfUmUmGmCmUfUmCfAmCmUmAmUmG*mG*m A |
| 2002-046 | 6 | mC*mA*mUmAfGmUfGmAfAmGmCmAmAmUmCmUmAmAmU |
| | 18 | mA*fU*mUmAmGfAmUfUfGmCmUmUmCfAmCfUmAmUmG*mG*mA |
| 2002-047 | 7 | mC*mA*mUmAmGmUfGmAfAmGfCmAmAmUmCmUmAmAmU |
| | 18 | mA*fU*mUmAmGfAmUfUfGmCmUmUmCfAmCfUmAmUmG*mG*mA |
| 2002-048 | 8 | mC*mA*mUmAmGmUfGfAfAmGmCmAmAmUmCmUmAmAmU |
| | 18 | mA*fU*mUmAmGfAmUfUfGmCmUmUmCfAmCfUmAmUmG*mG*mA |
| 2002-049 | 9 | mC*mA*mUmAfGmUfGmAdAmGfCmAmAmUmCmUmAmAmU |
| | 18 | mA*fU*mUmAmGfAmUfUfGmCmUmUmCfAmCfUmAmUmG*mG*mA |
| 2008-004 | 19 | mG*mA*mGmAfAmGfAfAfCmUmAmCmAmUmAmUmAmAmA |
| | 24 | mU*fU*mUmAmUmAmUmGmUmAmGmUmUfCmUfUmCmUmC*mA*m |
| | | G |
| 2008-005 | 19 | mG*mA*mGmAfAmGfAfAfCmUmAmCmAmUmAmUmAmAmA |
| | 25 | mU*fU*mUmAmUfAmUmGmUmAmGmUmUfCmUmUmCmUmC*mA*m G |
| 2008-009 | 19 | mG*mA*mGmAfAmGfAfAfCmUmAmCmAmUmAmUmAmAmA |
| | 26 | mU*fU*mUmAmUfAmUmGmUmAmGfUmUfCmUfUmCmUmC*mA*mG |
| 2008-010 | 19 | mG*mA*mGmAfAmGfAfAfCmUmAmCmAmUmAmUmAmAmA |
| | 27 | mU*fU*mUmAfUmAfUmGmUmAmGmUmUfCmUfUmCmUmC*mA*mG |

(continued)

| Duplex Name | SEQ ID NO | Sequence (5'-3') (Sense strand listed first for each duplex, antisense strand second) |
|---|---|---|
| 2008-011 | 19 | mG*mA*mGmAfAmGfAfAfCmUmAmCmAmUmAmUmAmAmA |
| | 28 | mU*fU*mUmAfUmAfUmGmUmAmGfUmUfCmUmUmCmUmC*mA*mG |
| 2008-006 | 19 | mG*mA*mGmAfAmGfAfAfCmUmAmCmAmUmAmUmAmAmA |
| | 29 | mU*mU*mUmAfUmAmUmGmUmAmGfUmUfCmUmUmCmUmC*mA*mG |
| 2008-007 | 19 | mG*mA*mGmAfAmGfAfAfCmUmAmCmAmUmAmUmAmAmA |
| | 30 | mU*mU*mUmAmUfAmUmGmUmAmGfUmUfCmUmUmCmUmC*mA*mG |
| 2008-008 | 19 | mG*mA*mGmAfAmGfAfAfCmUmAmCmAmUmAmUmAmAmA |
| | 31 | mU*mU*mUmAmUmAfUmGmUmAmGfUmUfCmUmUmCmUmC*mA*mG |
| 2008-046 | 20 | mG*mA*mGmAmAmGfAfAfCmUmAmCmAmUmAmUmAmAmA |
| | 32 | mU*fU*mUmAmUfAmUfGfUmAmGmUmUfCmUfUmCmUmC*mA*mG |
| 2008-047 | 21 | mG*mA*mGmAfAmGfAmAfCmUmAmCmAmUmAmUmAmAmA |
| | 32 | mU*fU*mUmAmUfAmUfGfUmAmGmUmUfCmUfUmCmUmC*mA*mG |
| 2008-048 | 22 | mG*mA*mGmAmAmGfAmAfCmUfAmCmAmUmAmUmAmAmA |
| | 32 | mU*fU*mUmAmUfAmUfGfUmAmGmUmUfCmUfUmCmUmC*mA*mG |
| 2008-049 | 23 | mG*mA*mGmAfAmGfAmAdCmUfAmCmAmUmAmUmAmAmA |
| | 32 | mU*fU*mUmAmUfAmUfGfUmAmGmUmUfCmUfUmCmUmC*mA*mG |
| 2002-050 | 5 | mC*mA*mUmAfGmUfGfAfAmGmCmAmAmUmCmUmAmAmU |
| | 18 | mA*fU*mUmAmGfAmUfUfGmCmUmUmCfAmCfUmAmUmG*mG*mA |
| 2008-050 | 19 | mG*mA*mGmAfAmGfAfAfCmUmAmCmAmUmAmUmAmAmA |
| | 32 | mU*fU*mUmAmUfAmUfGfUmAmGmUmUfCmUfUmCmUmC*mA*mG |

**Table 3. GalNAc-conjugated modified ANGPTL3 siRNAs**

| Duplex Name | SEQ ID NO | Sequence (5'-3') (Sense strand listed first for each duplex, antisense strand second) |
|---|---|---|
| 2002-033-002 | 33 | mC*mA*mUmAmGmUfGmAfAmGfCmAmAmUmCmUmAmAmU-YHZY12002 |
| | 12 | mA *fU*mUmAmGfAmUmUmGmCmUfUmCfAmCfUmAmUmG*mG*mA |
| 2002-034-002 | 33 | mC*mA*mUmAmGmUfGmAfAmGfCmAmAmUmCmUmAmAmU-YHZY12002 |
| | 13 | mA*fU*mUmAfGmAfUmUmGmCmUmUmCfAmCfUmAmUmG*mG*mA |
| 2002-018-002 | 34 | mC*mA*mUmAmGmUfGfAfAmGmCmAmAmUmCmUmAmAmU-YHZY12002 |
| | 13 | mA*fU*mUmAfGmAfUmUmGmCmUmUmCfAmCfUmAmUmG*mG*mA |
| 2008-010-002 | 35 | mG*mA*mGmAfAmGfAfAfCmUmAmCmAmUmAmUmAmAmA-YHZY12002 |
| | 27 | mU*fU*mUmAfUmAfUmGmUmAmGmUmUfCmUfUmCmUmC*mA*mG |

(continued)

| Duplex Name | SEQ ID NO | Sequence (5'-3') (Sense strand listed first for each duplex, antisense strand second) |
|---|---|---|
| 2008-011-002 | 35 | mG*mA*mGmAfAmGfAfAfCmUmAmCmAmUmAmUmAmAmA-YHZY12002 |
| | 28 | mU*fU*mUmAfUmAfUmGmUmAmGfUmUfCmUmUmCmUmC*mA*mG |
| 2008-018-002 | 36 | mG*mA*mGmAmAmGfAfAfCmUmAmCmAmUmAmUmAmAmA-YHZY12002 |
| | 27 | mU*fU*mUmAfUmAfUmGmUmAmGmUmUfCmUfUmCmUmC*mA*mG |
| 2008-019-002 | 36 | mG*mA*mGmAmAmGfAfAfCmUmAmCmAmUmAmUmAmAmA-YHZY12002 |
| | 28 | mU*fU*mUmAfUmAfUmGmUmAmGfUmUfCmUmUmCmUmC*mA*mG |
| 2008-035-002 | 37 | mG*mA*mGmAmAmGfAmAfCmUfAmCmAmUmAmUmAmAmA-YHZY12002 |
| | 28 | mU*fU*mUmAfUmAfUmGmUmAmGfUmUfCmUmUmCmUmC*mA*mG |
| 2002-033-001 | 38 | mC*mA*mUmAmGmUfGmAfAmGfCmAmAmUmCmUmAmAmU-YHZY12001 |
| | 12 | mA *fU*mUmAmGfAmUmUmGmCmUfUmCfAmCfUmAmUmG*mG*mA |
| 2002-034-001 | 38 | mC*mA*mUmAmGmUfGmAfAmGfCmAmAmUmCmUmAmAmU-YHZY12001 |
| | 13 | mA*fU*mUmAfGmAfUmUmGmCmUmUmCfAmCfUmAmUmG*mG*mA |
| 2002-018-001 | 39 | mC*mA*mUmAmGmUfGfAfAmGmCmAmAmUmCmUmAmAmU-YHZY12001 |
| | 13 | mA*fU*mUmAfGmAfUmUmGmCmUmUmCfAmCfUmAmUmG*mG*mA |
| 2008-010-001 | 40 | mG*mA*mGmAfAmGfAfAfCmUmAmCmAmUmAmUmAmAmA-YHZY12001 |
| | 27 | mU*fU*mUmAfUmAfUmGmUmAmGmUmUfCmUfUmCmUmC*mA*mG |
| 2008-011-001 | 40 | mG*mA*mGmAfAmGfAfAfCmUmAmCmAmUmAmUmAmAmA-YHZY12001 |
| | 28 | mU*fU*mUmAfUmAfUmGmUmAmGfUmUfCmUmUmCmUmC*mA*mG |
| 2008-018-001 | 41 | mG*mA*mGmAmAmGfAfAfCmUmAmCmAmUmAmUmAmAmA-YHZY12001 |
| | 27 | mU*fU*mUmAfUmAfUmGmUmAmGmUmUfCmUfUmCmUmC*mA*mG |
| 2008-019-001 | 41 | mG*mA*mGmAmAmGfAfAfCmUmAmCmAmUmAmUmAmAmA-YHZY12001 |
| | 28 | mU*fU*mUmAfUmAfUmGmUmAmGfUmUfCmUmUmCmUmC*mA*mG |
| 2008-035-001 | 42 | mG*mA*mGmAmAmGfAmAfCmUfAmCmAmUmAmUmAmAmA-YHZY12001 |
| | 28 | mU*fU*mUmAfUmAfUmGmUmAmGfUmUfCmUmUmCmUmC*mA*mG |
| 2002-050-L96 | 43 | mC*mA*mUmAfGmUfGfAfAmGmCmAmAmUmCmUmAmA mU-L96 |
| | 18 | mA*fU*mUmAmGfAmUfUfGmCmUmUmCfAmCfUmAmUmG*mG*mA |

(continued)

| Duplex Name | SEQ ID NO | Sequence (5'-3') (Sense strand listed first for each duplex, antisense strand second) |
|---|---|---|
| ARO-05488-003-NAG37 | 44 | (NAG37)* (invAb)* mGmCmUmCmAmAmCmAfUfAfUmU mUmGmAmUmCmAmGmUmA*(invAb) |
| | 45 | mU*fA*mC*fUmGfAmUfCmAfAmAfUmAfUmGfUmUfGmAfG*mC |

**Table 4. Exemplary ANGPTL3 siRNA duplexes (without ligand)**

| Duplex Name | SEQ ID NO | Sequence (5'-3') (Sense strand listed first for each duplex, antisense strand second) |
|---|---|---|
| 2002-010 | 5 | mC*mA*mUmAfGmUfGfAfAmGmCmAmAmUmCmUmAmAmU |
| | 13 | mA*fU*mUmAfGmAfUmUmGmCmUmUmCfAmCfUmAmUmG*mG*mA |
| 2002-011 | 5 | mC*mA*mUmAfGmUfGfAfAmGmCmAmAmUmCmUmAmAmU |
| | 14 | mA*fU*mUmAfGmAfUmUmGmCmUfUmCfAmCmUmAmUmG*mG*mA |
| 2008-010 | 19 | mG*mA*mGmAfAmGfAfAfCmUmAmCmAmUmAmUmAmAmA |
| | 27 | mU*fl_J*mUniAfLJniAfl_JmGmUniAmGmUmUfCmUfl_JmCmUmC*mA*mG |
| 2008-011 | 19 | mG*mA*mGmAfAmGfAfAfCmUmAmCmAmUmAmUmAmAmA |
| | 28 | mU*fU*mUmAfUmAfUnmGmUmAmGfUmUfCmUmUmCmUmC*mA*mG |
| 2002-034 | 7 | mC*mA*mUmAmGmUfGmAfAmGfCmAmAmUmCmUmAmAmU |
| | 13 | mA*fU*mUmAfGmAfUmUmGmCmUmUmCfAmCfUmAmUmG*mG*mA |
| 2002-018 | 8 | mC*mA*mUmAmGmUfGfAfAmGmCmAmAmUmCmUmAmAmU |
| | 13 | mA*fU*mUmAfGmAfUmUmGmCmUmUmCfAmCfUmAmUmG*mG*mA |
| 2008-018 | 20 | mG*mA*mGmAmAmGfAfAfCmUmAmCmAmUmAmUmAmAmA |
| | 27 | mU*fU*mUmAfUmAfUmGmUmAmGmUmUfCmUfUmCmUmC*mA*mG |
| 2008-019 | 20 | mG*mA*mGmAmAmGfAfAfCmUmAmCmAmUmAmUmAmAmA |
| | 28 | mU*fU*mUmAfUmAfUnmGmUmAmGIUmUfCmUmUmCmUmC*mA *mG |
| 2008-035 | 22 | mG*mA*mGmAmAmGfAmAfCmUfAmCmAmUmAmUmAmAmA |
| | 28 | mU*fU*mUmAfUmAfUnmGmUmAmGfUmUfCmUmUmCmUmC*mA*mG |

wherein m represents a 2'-O-methyl modification; f represents a 2'-fluoro modification; * represents a phosphorothioate linkage;

YHZY12001 =

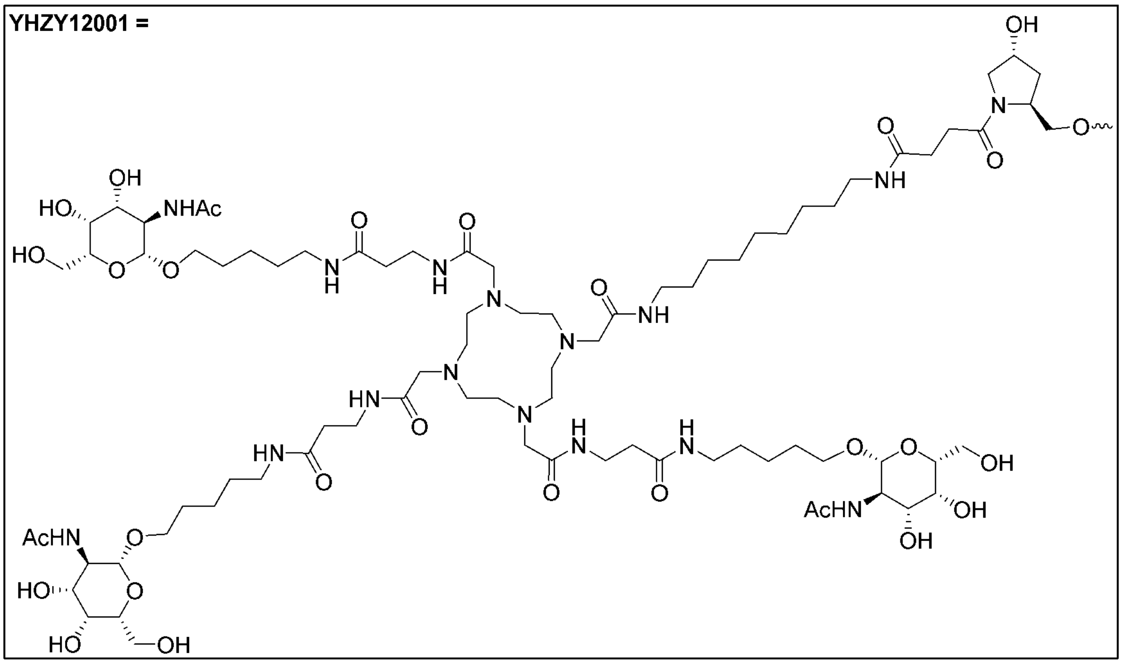

YHZY12002 =

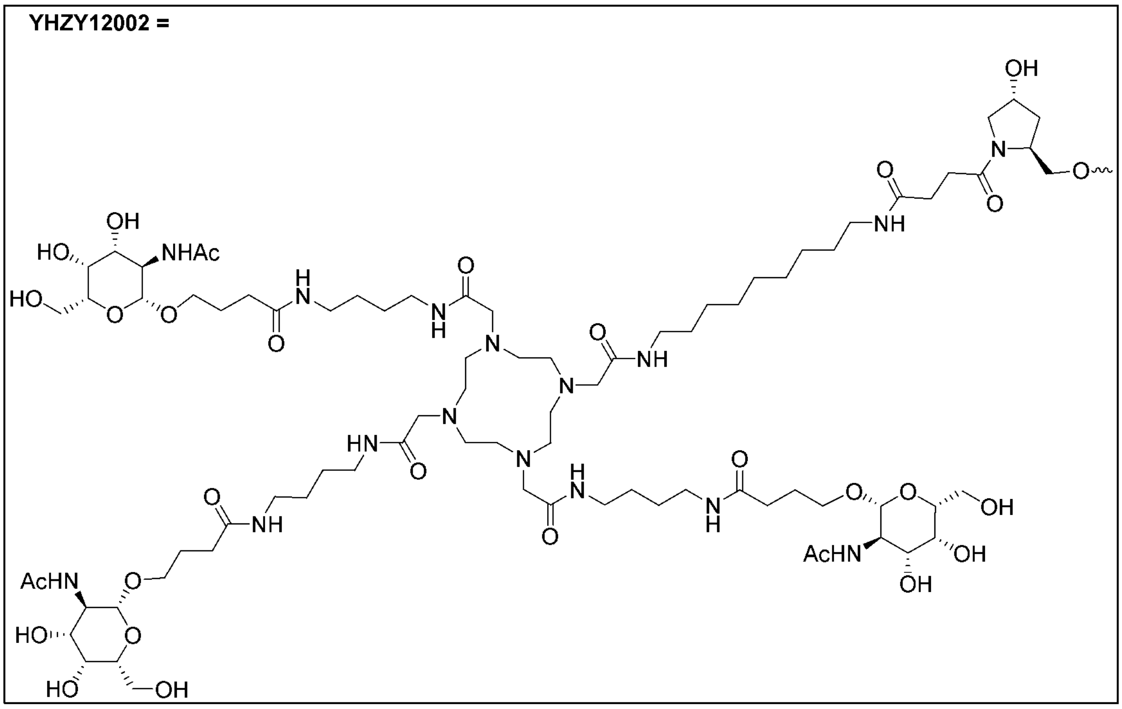

L96 =

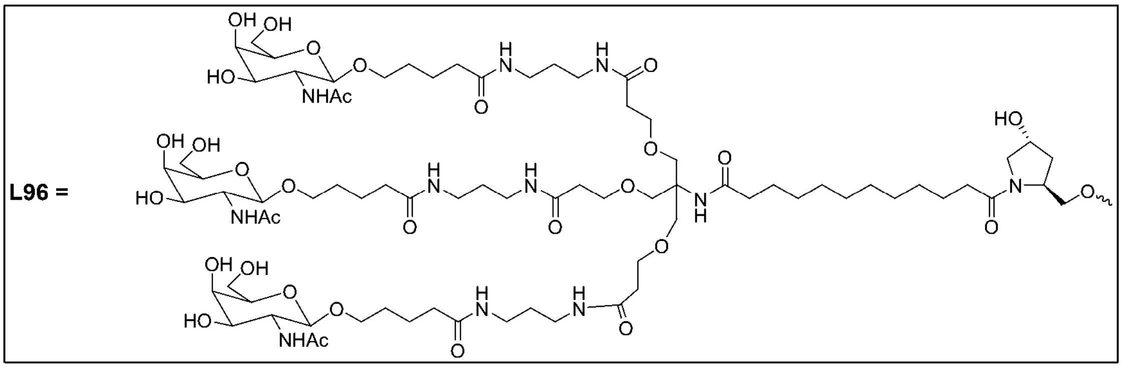

NAG37 = 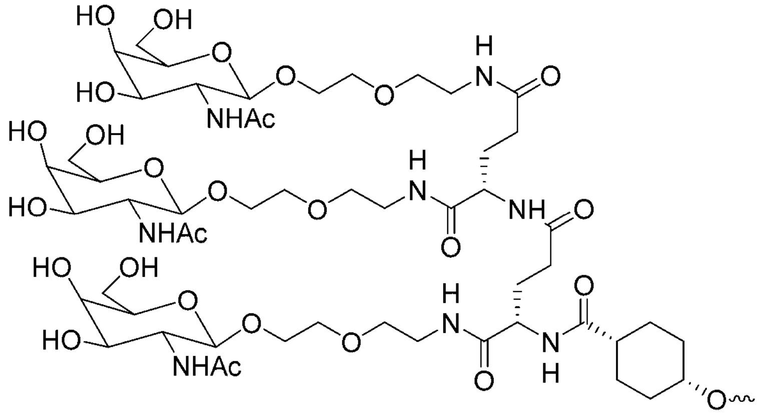

invAb = 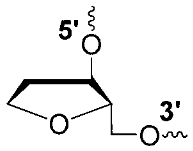

## Examples

## Experimental Methods and Materials

### Target Sequence Screening

siRNAs were designed based on the full-length ANGPTL3 mRNA sequence (NM_014495.4). All sequences were sourced from the NCBI Gene database. All siRNA designs ensured homology with sequences from humans (Gene ID: 27329, NM_014495.4) and cynomolgus monkeys (Gene ID: 102136264, XM_005543185.3).

### GalNAc Resin Synthesis

GalNAc is a terminal reactive group-containing compound, including carboxylic acid-reactive compounds such as Gal-C7, Gal-C6, Gal-C5, Gal-C4, Gal-C3, etc., and amine-reactive compounds such as Gal-C7-$NH_2$, Gal-C6-$NH_2$, Gal-C5-$NH_2$, Gal-C4-$NH_2$, Gal-C3-$NH_2$. These were synthesized according to reported methods, with specific details provided in Table 5.

Table 5. List of chemical structures, nomenclature and synthesis methods for terminal reactive group-containing GalNAc compounds

| Chemical Structures (terminal reactive group-containing GalNAc compounds) | ID | References (reported synthesis methods) |
|---|---|---|
| 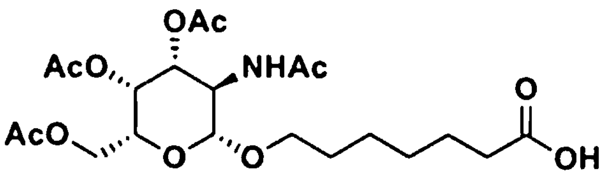 | Gal-C7 | WO2021037205, 2021 |
| 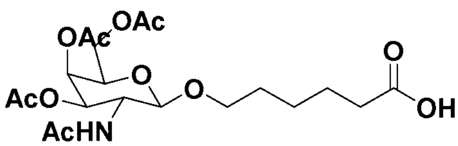 | Gal-C6 | WO2015042447A1, 2015 |

(continued)

| Chemical Structures (terminal reactive group-containing GalNAc compounds) | ID | References (reported synthesis methods) |
|---|---|---|
| 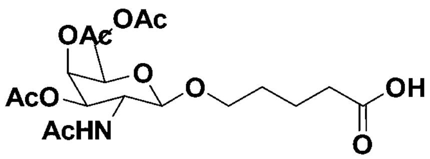 | Gal-C5 | US20120035115, 2012 |
| 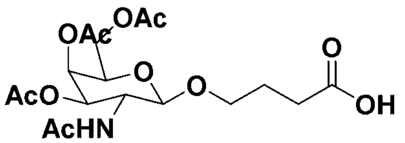 | Gal-C4 | J. Org. Chem., 2020, 85, 10, 6593-6604 |
| 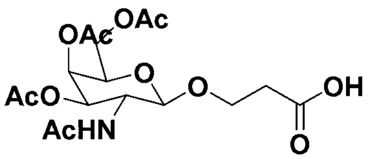 | Gal-C3 | Same method used in US20120035115 |
| 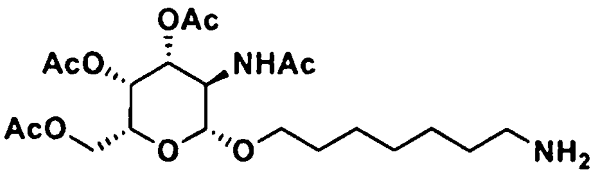 | Gal-C7-$NH_2$ | Same method used in WO2015042447A1 |
| 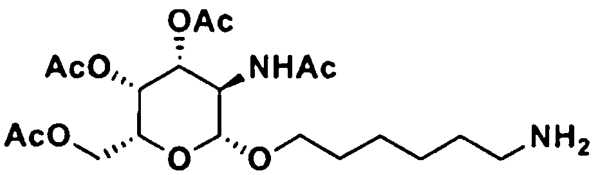 | Gal-C6-$NH_2$ | WO2015042447A1, 2015 |
| 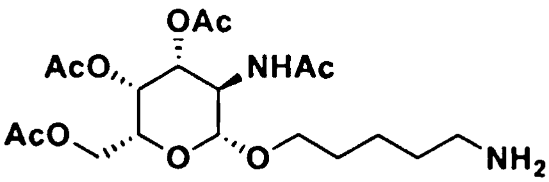 | Gal-C5-$NH_2$ | WO02021049504A1, 2021 |
| 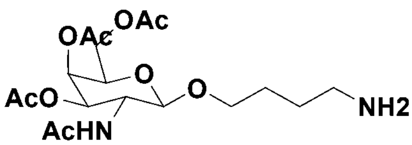 | Gal-C4-$NH_2$ | J. Med. Chem. 2016, 59, 6, 2718-2733 |
| 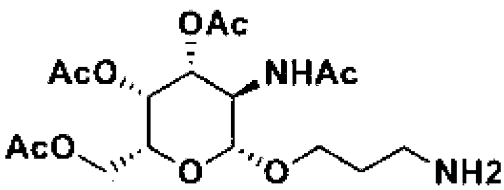 | Gal-C3-$NH_2$ | WO2021049504A1, 2021 |

(continued)

| Chemical Structures (terminal reactive group-containing GalNAc compounds) | ID | References (reported synthesis methods) |
|---|---|---|
| 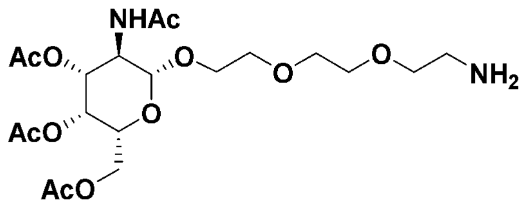 | GalNAc-AEEA | WO2020093053, 2020 |

Synthesis of key intermediate compound **A**

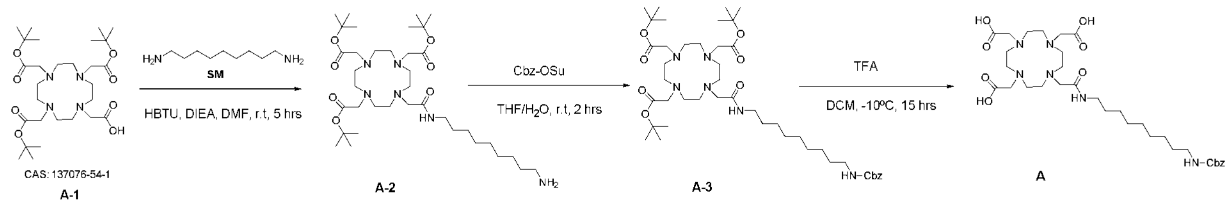


Synthesis of Compound **A-2**

Compound **A-1** (3.0 g, 5.24 mmol, 1.0 equiv.) was dissolved in DMF (20 mL), followed by sequential addition of HBTU (3.9 g, 10.5 mmol, 2.0 equiv.) and DIPEA (1.73 mL, 2.0 equiv.). The reaction mixture was stirred at room temperature for 5 minutes, whereupon 1,9-diaminononane (993 mg, 1.2 equiv.) dissolved in DMF (10 mL) was added. The reaction was stirred at room temperature for 5 hours. Upon reaction completion, the mixture was quenched with water (30 mL) and extracted with ethyl acetate (20 mL). The organic phase was sequentially washed with 5% aqueous sodium bicarbonate solution (20 mL) and saturated brine (10 mL), dried over anhydrous sodium sulfate, and concentrated to afford crude product **A-2** (3.65 g). This crude material was used directly in the subsequent reaction without further purification. LCMS: (ESI) *m*/*z* = 714 $[M+H]^+$.

Synthesis of Compound **A-3**

The crude product **A-2** obtained in the previous step (3.65 g, 5.13 mmol) was dissolved in THF (10 mL), followed by sequential addition of Cbz-OSu (1.28 g, 5.13 mmol) and saturated aqueous sodium bicarbonate solution (10 mL). The mixture was stirred at room temperature for 2 hours. Upon reaction completion, the reaction mixture was diluted with water (20 mL) and extracted with ethyl acetate (3 × 20 mL). The combined organic phases were washed with saturated brine (20 mL), dried over anhydrous sodium sulfate, and concentrated. The resultant residue was purified by preparative column chromatography to afford **A-3** (1.1 g, yield: 25.6%) as a white solid. LCMS: (ESI) *m*/*z* = 847.5 $[M+H]^+$.

HPLC purification conditions: column: Xbridge C18, 19 × 250 mm, 10 μm, 130 Å; mobile phase: A: water (containing 0.05% TFA), B: acetonitrile (containing 0.05% TFA); gradient program: ramp from 5% B to 95% B over 20 min.

Synthesis of Compound **A**

Intermediate **A-3** (1.1 g, 1.30 mmol) was dissolved in DCM (5 mL) and cooled to - 10°C using a low-temperature reactor. A mixture of TFA and DCM (15 mL, *v*:*v* = 1:1) was slowly added. After complete addition, the reaction was stirred at -10°C for 15 hours. Upon reaction completion confirmed by LCMS, cold methyl tert-butyl ether (MTBE) (40 mL) was added, resulting in a white suspension. The mixture was centrifuged at 3200 rpm for 3 minutes, and the supernatant was decanted. The solid residue was resuspended in MTBE (40 mL), sonicated for 3 minutes, and recentrifuged (3200 rpm, 3 minutes). The supernatant was discarded, and the solid residue was dried to afford compound **A** (660 mg, yield: 74.9%) as a white solid. LCMS: (ESI) *m*/*z* = 679 $[M+H]^+$.

Synthesis of **I-A** class molecules

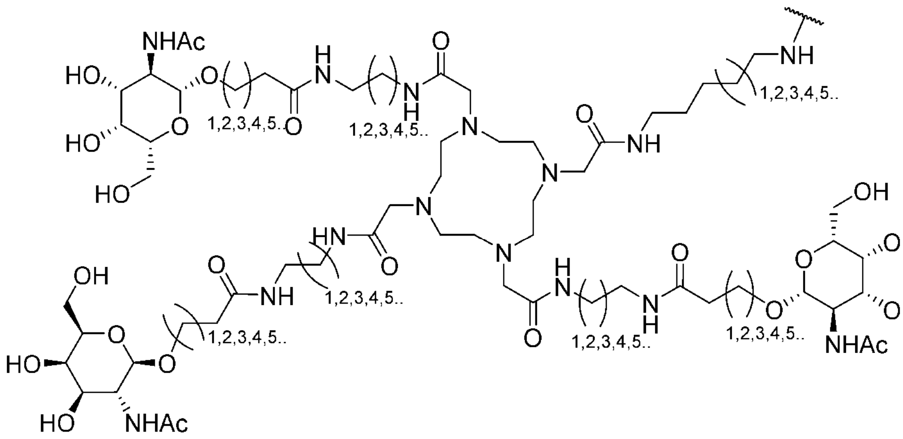

**3.1** Synthesis of **I-A10-SFAM**

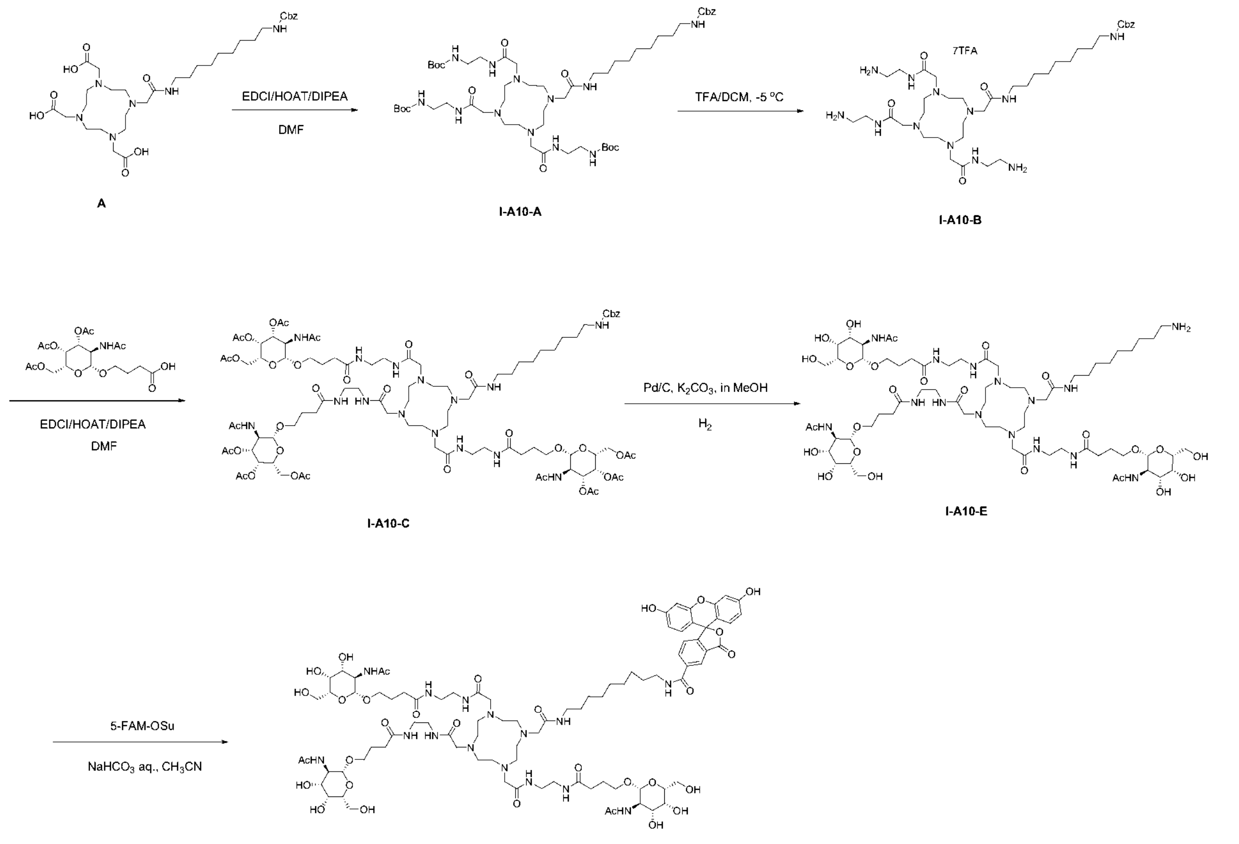


A

I-A10-A

I-A10-B

I-A10-C

I-A10-E

I-A10-5FAM

Synthesis of intermediate **I-A10-A**

1-tert-butoxycarbonylethylenediamine (71 mg, 0.442 mmol, 5.0 equiv.) and intermediate A (60 mg, 0.0884 mmol) were dissolved in DMF (3 mL), followed by sequential addition of EDCI·HCl (68 mg, 0.354 mmol), HOAt (48 mg, 0.354 mmol, 4.0 equiv.), and DIPEA (151 $\mu$L, 0.884 mmol). The reaction mixture was stirred at room temperature overnight. The crude mixture was directly subjected to purification by preparative column chromatography to afford **I-A10-A** as a white

solid (68 mg, yield: 68.0%). LCMS: (ESI) *m*/*z* = 1127.7 $[M+Na]^+$; 1105.7 $[M+H]^+$; 553.5 $[M+2H]/2^+$.

HPLC purification conditions: column: Welch Topsil C18, 21.2 × 250 mm, 5 μm, 150 Å; mobile phase: A: water (containing 0.05% TFA), B: acetonitrile (containing 0.05% TFA); gradient program: ramp from 20% B to 70% B over 20 min.

Synthesis of intermediate **I-A10-B**

**I-A10-A** (68 mg, 0.0435 mmol, 1.0 equiv.) was dissolved in DCM (3 mL) and cooled to -5 °C in an ice-salt bath. Trifluoroacetic acid (TFA, 3 mL) was added dropwise. The reaction mixture was stirred at -5°C for 2 hours. The solution was concentrated and dried to afford the trifluoroacetate salt of intermediate **I-A10-B** as a colorless transparent liquid (80 mg, yield: >100%). LCMS: (ESI) *m*/*z* = 805.7 $[M+H]^+$; 403.5 $[M+2H]/2^+$.

Synthesis of intermediate **I-A10-C**

Intermediate **I-A10-B** (80 mg, 0.0435 mmol) and **Gal-C4** (94 mg, 0.218 mmol) were dissolved in DMF (2 mL), followed by sequential addition of HOAt (30 mg, 0.218 mmol), EDCI·HCl (42 mg, 0.218 mmol), and DIPEA (74 μL, 0.435 mmol). The reaction mixture was stirred at room temperature overnight. Upon substantial reaction completion, the crude mixture was directly purified by preparative column chromatography to afford **I-A10-C** as a white solid (56 mg, yield: 62.7%). LCMS: (ESI) *m*/*z* = 1026.6 $[M+2H]/2^+$; 684.5 $[M+3H]/3^+$.

HPLC purification conditions: column: Xbridge C18 21.2 mm × 250 mm, 10 μm, 110 Å; mobile phase: A: water (containing 10 mmol/L $NH_4HCO_3$), B: acetonitrile; gradient program: ramp from 38% B to 48% B over 20 min.

Synthesis of intermediate **I-A10-E**

**I-A10-C** (25 mg, 0.0122 mmol) was dissolved in methanol (3 mL), followed by sequential addition of potassium carbonate (10 mg) and Pd/C (10% w/w on activated carbon, 5 mg). The mixture was stirred under a hydrogen atmosphere for 2 hours, with reaction progress monitored by LC-MS. The reaction mixture was filtered, and approximately 2 g of dry ice was added to the filtrate with stirring for 5 minutes. The mixture was then concentrated to afford crude **I-A10-E,** which was used directly in the subsequent reaction without further purification. LCMS: (ESI) *m*/*z* = 770.2 $[M+2H]/2^+$; 668.5 $[M+2H-204]/2^+$.

Synthesis of compound **I-A10-5FAM**

The crude product **I-A10-E** (0.0122 mmol) obtained in the previous step was dissolved in water (2 mL) and acetonitrile (0.5 mL), followed by sequential addition of **5-FAM-OSu** (8 mg, 0.0165 mmol) and saturated aqueous sodium bicarbonate solution (0.5 mL). The reaction mixture was stirred at room temperature for 1 hour. The crude mixture was directly purified by preparative HPLC to afford **I-A10-5FAM** as an orange-yellow solid (10.0 mg, yield over two steps: 43.3%). LCMS: (ESI) *m*/*z* = 967.8 $[M+H+K]/2^+$; 949.3 $[M+2H]/2^+$; 645.8 $[M+2H+K]/3^+$;633.3 $[M+3H]/3^+$.

HPLC purification conditions: column: Gemini C18 21.2 mm × 250 mm, 10 μm, 110 Å; mobile phase: A: water (containing 10 mmol/L $NH_4HCO_3$), B: acetonitrile; gradient program: ramp from 14% B to 24% B over 20 min.

HPLC: 91.1578% (214 nm), RT = 13.049 min; 7.2549% (214 nm), RT = 13.406 min
Mobile phase: A: water (0.05%TFA), B: acetonitrile (0.05%TFA)
Gradient program: 5% B equilibration for 3 min, ramped to 65% B over 20 min, ramped to 95% B over 2 min, and 95% B for 5 min.
Flow rate: 1.0 mL/min
Column: XBridge peptide BEH column C18, 4.6 × 150 mm, 3.5 μm, 130 Å
Temperature: 60 °C

**3.2** Synthesis of **I-A11-5FAM**

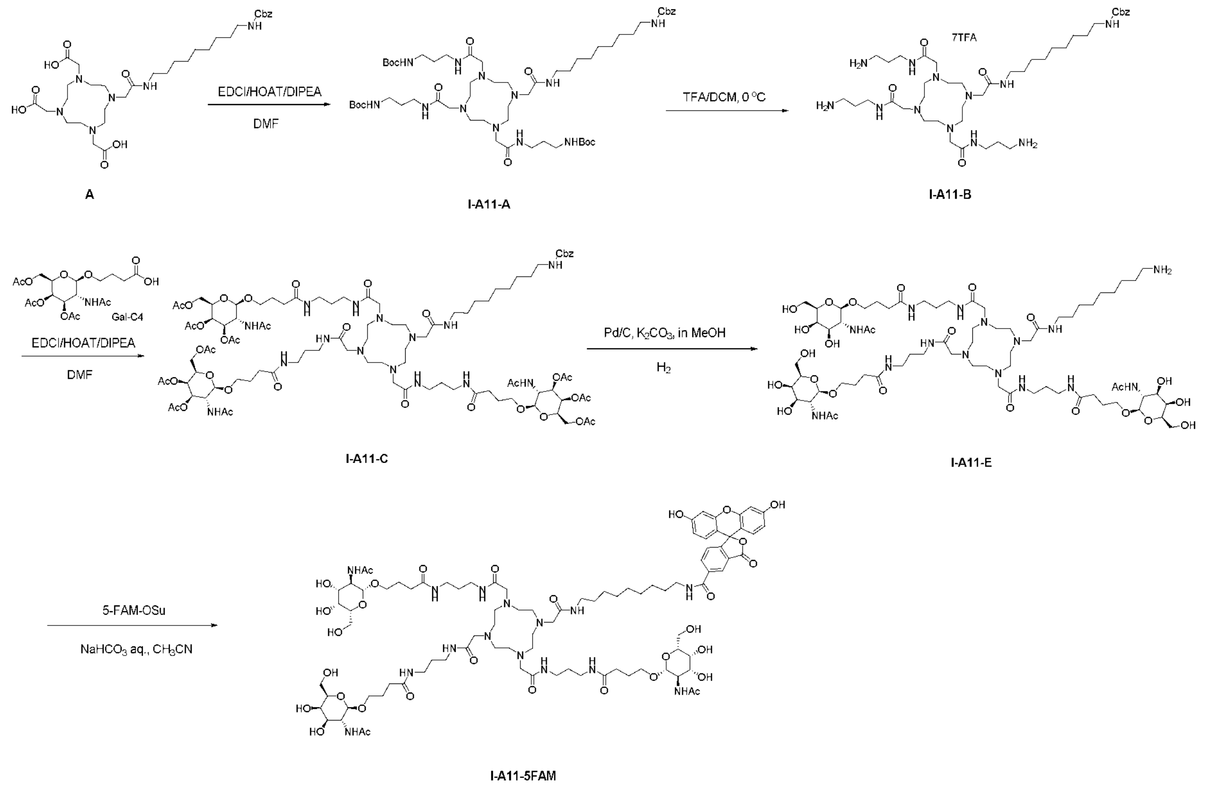


A

I-A11-A

I-A11-B

I-A11-C

I-A11-E

I-A11-5FAM

Synthesis of intermediate **I-A11-A**

Following the procedure for synthesizing intermediate **I-A10-A,** compound A (60 mg, 0.0884 mmol) was reacted with N-t-butyloxy carbonyl-propanediamine (73 mg, 0.442 mmol) in DMF (3 mL). After purification, **I-A11-A** was obtained as a white solid (70 mg, yield: 68.0%). LCMS: (ESI) *m/z* = 1147.8 $[M+H]^+$.

Synthesis of intermediate **I-A11-B**

Following the procedure for synthesizing intermediate **I-A10-B, I-A11-B** (80 mg, yield: quantitative) was obtained as a colorless oily liquid from **I-A11-A** (70 mg, 0.0436 mmol). LCMS: (ESI) *m/z* = 847.7 $[M+H]^+$.

Synthesis of intermediate **I-A11-C**

Following the procedure for synthesizing intermediate **I-A10-C, I-A11-C** (32 mg, yield: 35.1%) was obtained as a white solid from crude **I-A11-B.** LCMS: (ESI) *m/z* = 1047.7 $[M+2H]/2^+$.

HPLC purification conditions: column: Gemini C18 21.2 mm × 250 mm, 10 μm, 110 Å; mobile phase: A: water (containing 10 mmol/L $NH_4HCO_3$), B: acetonitrile; gradient program: ramp from 41% B to 51% B over 20 min.

Synthesis of intermediate **I-A11-E**

Following the procedure for synthesizing intermediate **I-A10-E, I-A11-E** (40 mg, yield: quantitative) was obtained as a white solid from **I-A11-C** (32 mg, 0.0156 mmol). LCMS: (ESI) *m/z* =791.0 $[M+2H]/2^+$.

Synthesis of compound **I-A11-5FAM**

Following the procedure for synthesizing **I-A10-5FAM, I-A11-5FAM** (6.3 mg, yield: 20.8%) was obtained as an orange solid from **I-A11-E** (40 mg). LCMS: (ESI) *m/z* = 989.2 $[M+H+K]/2^+$.

HPLC purification conditions: column: Gemini C18 21.2 mm × 250 mm, 10 μm, 110 Å; mobile phase: A: water (containing 10 mmol/L $NH_4HCO_3$), B: acetonitrile; gradient program: ramp from 13% B to 23% B over 20 min.

HPLC purity: 60.0611% (214 nm), RT = 12.894 min; 37.2783% (214 nm), RT = 13.256 min

Analysis conditions were the same as those for **I-A10-5FAM.**

Synthesis of **I-A12-5FAM**

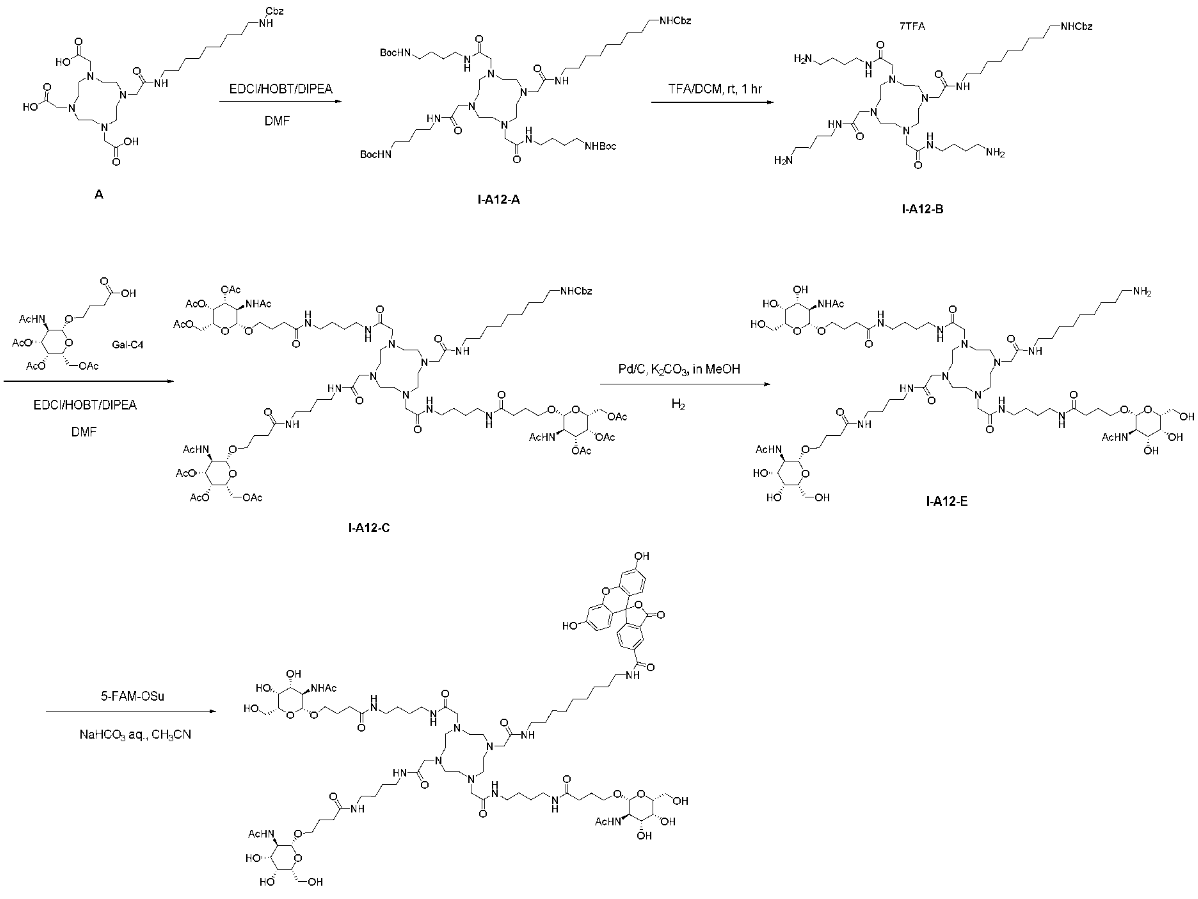


I-A12-5FAM

Synthesis of intermediate **I-A12-A**

Following the procedure for synthesizing intermediate **I-A10-A,** compound A (60 mg, 0.0884 mmol) was reacted with N-Boc-butanediamine (75 mg, 0.396 mmol) in DMF (2 mL). The product was purified to afford **I-A12-A** (73 mg, yield: 69.5%) as a white solid. LCMS: (ESI) *m/z* = 595.5 $[M+2H]/2^+$.

HPLC purification conditions: column: Xbridge C18, 19 × 250 mm, 10 μm, 130 Å; mobile phase: A: water (containing 0.05% TFA), B: acetonitrile (containing 0.05% TFA); gradient program: ramp from 20% B to 80% B over 20 min.

Synthesis of intermediate **I-A12-B**

**I-A12-A** (73 mg, 0.061 mmol) was dissolved in DCM (2 mL), followed by dropwise addition of TFA (2 mL). The mixture was reacted in room temperature for 1 hour and the reaction mixture was concentrated and dried to afford **I-A12-B** (53 mg yield: quantitative) as colorless oily liquid. LCMS: (ESI) *m/z* = 911.7 $[M+Na]^+$; 445.4 $[M+2H]/2^+$.

Synthesis of intermediate **I-A12-C**

**Gal-C4** (1.2 g, 2.77 mmol, 5.0 eq.) was dissolved in DMF (20 mL), followed by sequential addition of **I-A12-B** (900 mg, 0.553 mmol), EDCI (530 mg, 2.77 mmol), HOAt (376 mg, 2.77 mmol) and DIPEA (944 μL, 5.53 mmol). The reaction mixture was stirred at room temperature for 3 hours. Upon reaction completion as confirmed by LCMS, the reaction mixture was purified by preparative chromatographic column directly to afford **I-A12-C** (900 mg, yield: 76%).LCMS: (ESI) *m/z* = 1068.8 $[M+2H]/2^+$; 903.8 [M+2H-330]/2; 712.8 $[M+3H]/3^+$.$^1$H NMR (400 MHz, DMSO) δ 8.07-7.71 (m, 10H, 10NH), 7.39-7.19 (m, 6H, Bn-5H+1NH), 5.20 (d, J = 3.2 Hz, 3H, 3Gal-OCHO), 5.00 (s, 2H, Ph-CH2), 4.96 (dd, J = 3.2, 11.2 Hz, 3H, 3Gal-OCHC), 4.48 (d, *J* = 8.4 Hz, 3H, 3Gal-OCHC), 4.02 (brs, 9H), 3.92-3.80 (m, 3H, 3Gal-OCHC), 3.70-3.63 (m, 3H,

3Gal-OCHC), 3.47-3.39 (m, 3H, 3Gal-OCHC), 3.131-2.90 (m, 26H), 2.72-2.52 (m, 14H), 2.16-2.03 (m, 6H), 2.10 (s, 9H, 3CH3), 1.99 (s, 9H, 3CH3), 1.89 (s, 9H, 3CH3), 1.76 (s, 9H, 3CH3), 1.74-1.61 (m, 6H), 1.47-1.30 (m, 16H), 1.23 (brs, 10H).
HPLC purification conditions: column: Xbridge C18, 19 × 250 mm, 10 μm, 130 Å; mobile phase: A: water (containing 10 mmol/L $NH_4HCO_3$), B: acetonitrile; gradient program: ramp from 39% B to 49% B over 10 min.

Synthesis of intermediate **I-A12-E**

Following the procedure for synthesizing intermediate **I-A10-E, I-A12-E** (33 mg, quantitative) was obtained as a white solid from **I-A12-C** (30 mg, 0.0140 mmol). LCMS: (ESI) *m*/*z* = 812 $[M+2H]/2^+$.

Synthesis of compound **I-A12-5FAM**

Following the procedure for synthesizing **I-A10-5FAM, I-A12-5FAM** (8.5 mg, yield: 21.5%) was obtained as an orange solid from **I-A12-E** (33 mg, 0.020 mmol). LCMS: (ESI) *m*/*z* = 991.0 $[M+H+K]/2^+$; 674.0 $[M+2H+K]/3^+$.
HPLC purification conditions: column: Gemini C18 21.2 mm × 250 mm, 10 μm, 110 Å; mobile phase: A: water (containing 10 mmol/L $NH_4HCO_3$), B: acetonitrile; gradient program: ramp from 5% B to 35% B over 10 min.
HPLC purity: 9.5195 % (214 nm), RT = 13.588 min; 87.8220 % (214 nm), RT = 13.008 min
Analysis conditions were the same as those for **I-A10-5FAM.**

**3.4** Synthesis of**I-A12-Cy5**

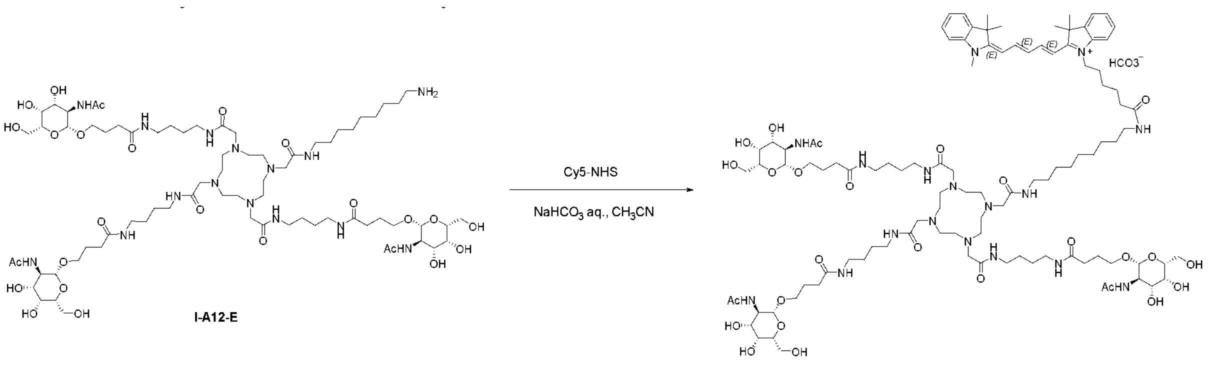


I-A12-E

I-A12-Cy5

Following the procedure for synthesizing **I-A12-5FAM, I-A12-Cy5** (8.0 mg, yield: 26.5%) was obtained as an orange solid from **I-A12-E** (30 mg, 0.020 mmol). LCMS: (ESI) *m*/*z* = 1044.5 $[M+2H]/2^+$, 709.5 $[M+3H+K]/3^+$, 696.8 $[M+3H]/3^+$.
HPLC purification conditions: column: Gemini C18 21.2 mm × 250 mm, 10 μm, 110 Å; mobile phase: A: water (containing 10 mmol/L $NH_4HCO_3$), B: acetonitrile; gradient program: ramp from 5% B to 35% B over 10 min.
HPLC purity: 84.9 % (214 nm), RT = 17.535 min; 12.1 % (214 nm), RT = 17.945 min

**3.5** Synthesis of compound **I-A12-Ac-5FAM**

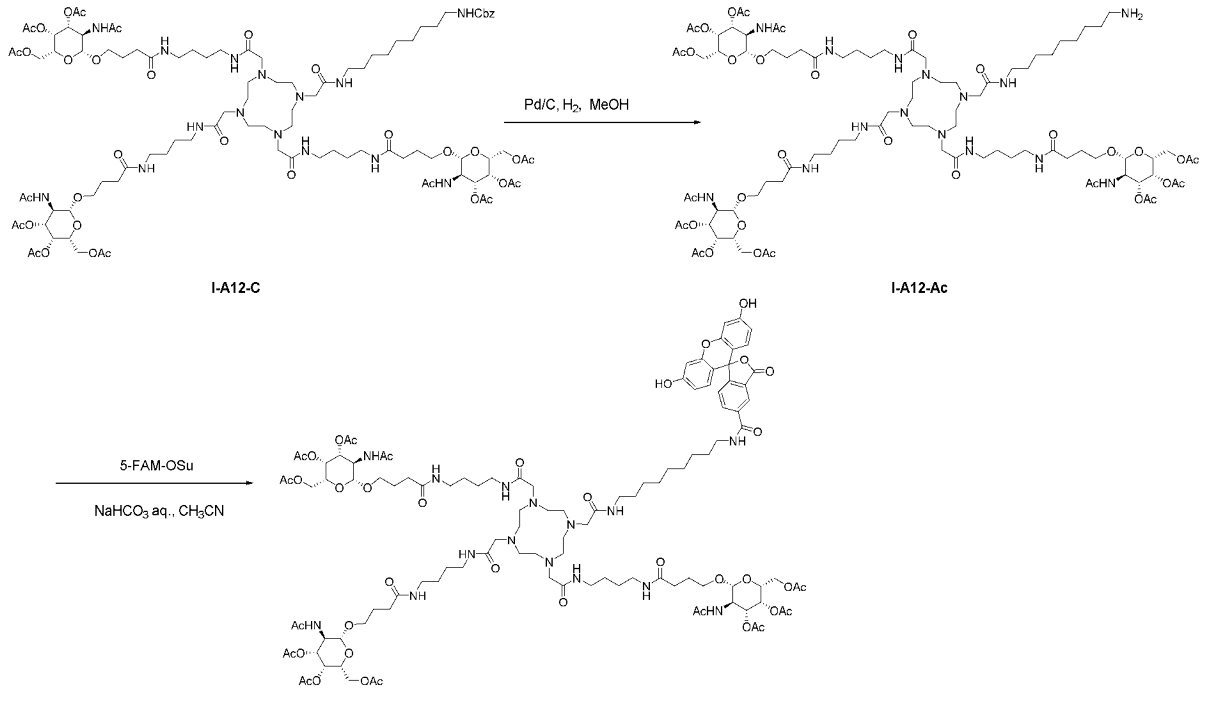


I-A12-C I-A12-Ac I-A12-Ac-5FAM

**I-A12-C** (40 mg, 0.0187 mmol) was dissolved in methanol (5 mL) followed by addition of Pd/C (10% w/w adsorbed on activated carbon, 8 mg). The mixture was stirred in hydrogen atmosphere for 13 hours and the reaction is monitored by LC-MS. The reaction mixture was filtered, concentrated and purified to afford **I-A12-Ac** (5 mg, yield: 13.3%) which was used directly for next step. LCMS: (ESI) *m/z* = 1001.7 $[M+2H]/2^{+}$; 680.7 $[M+2H+K]/3^{+}$.

The **I-A12-Ac** (5 mg, 0.00250 mmol) obtained in the previous step was dissolved in water (2 mL) and acetonitrile (0.5 mL), followed by subsequential addition of **5-FAM-OSu** (1.2 mg, 0.0165 mmmol) and saturated aqueous sodium bicarbonate solution (0.5 mL). The reaction mixture was stirred at room temperature for 1 hour and then purified by preparative column chromatography to afford **I-A12-Ac-5FAM** (3.5 mg, yield: 59.3%) as an orange solid. LCMS: (ESI) *m/z* = 800.4 $[M+2H+K]/3^{+}$; 787.5 $[M+3H]/3^{+}$.

HPLC purification conditions: preparative column: Gemini C18 21.2 mm × 250 mm, 10 μm, 110 Å; mobile phase: A: water (containing 10 mmol/L $NH_4HCO_3$), B: acetonitrile; gradient program: ramp from 35% B to 55% B over 20 min.

HPLC purity: 75.08% (214 nm), RT = 17.237 min; 18.82% (214 nm), RT = 17.738 min

Analysis conditions were the same as those for **I-A10-5FAM.**

**3.6** Synthesis of **I-A13-5FAM**

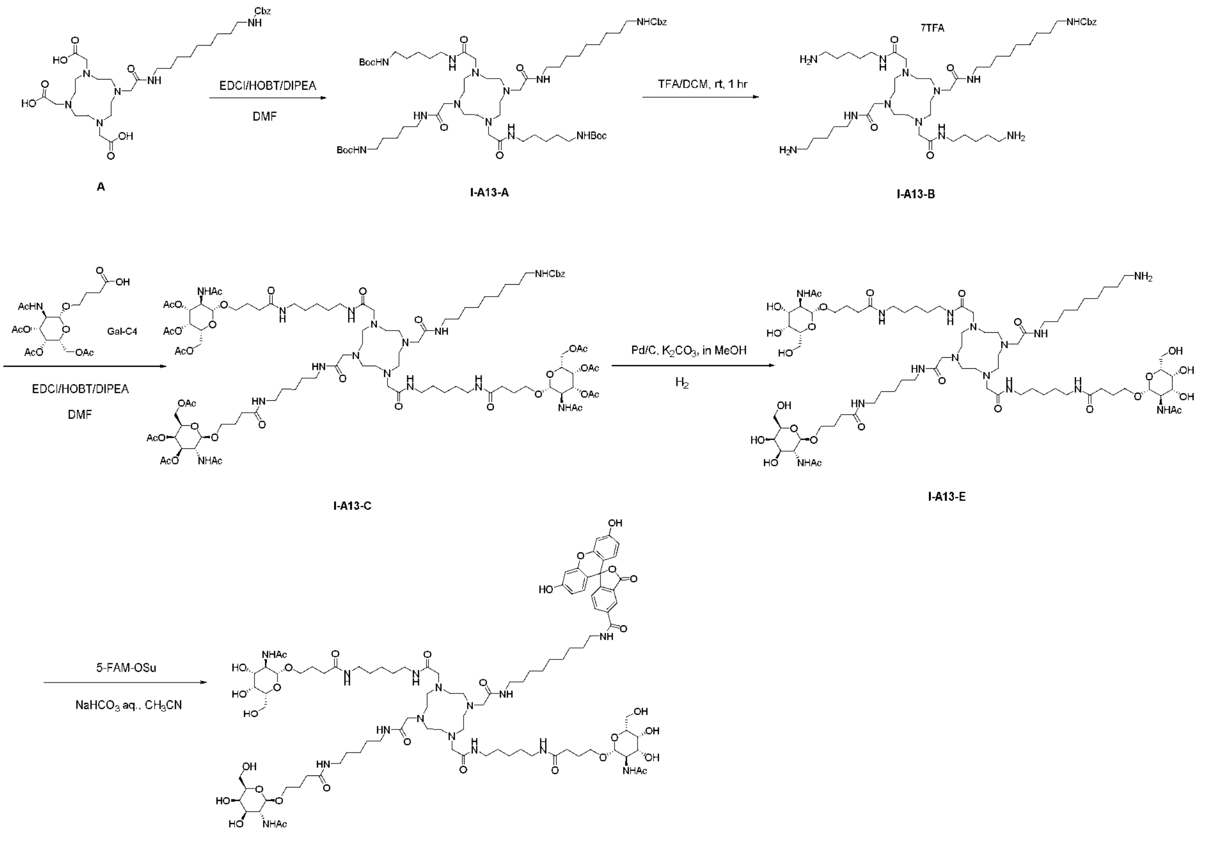


I-A13-5FAM

Synthesis of intermediate **I-A13-A**

Following the procedure for synthesizing intermediate **I-A10-A,** compound **A** (60 mg, 0.0884 mmol) was reacted with N-Boc-butanediamine (89 mg, 0.442 mmol) in DMF (2 mL) at room temperature for 2 hours. The reaction mixture was directly purified to afford **I-A13-A** (72 mg, yield: 66.7%) as a white solid. LCMS: (ESI) *m/z* = 1231.7 $[M+H]^+$; 616.3 $[M+2H]/2^+$.

HPLC purification conditions: preparative column: Xbridge C18, 19 × 250 mm, 10 μm, 130 Å; mobile phase: A: water (containing 10 mmol/L $NH_4HCO_3$), B: acetonitrile; gradient program: ramp from 33% B to 43% B over 20 min.

Synthesis of intermediate **I-A13-B**

Following the procedure for synthesizing intermediate **I-A12-B, I-A13-B** (65 mg, yield: quantitative) was obtained as colorless oily liquid from **I-A13-A** (72 mg, 0.058 mmol). LCMS: (ESI) *m/z* = 953.8 $[M+Na]^+$; 931.7 $[M+H]^+$; 466.5 $[M+2H]/2^+$.

Synthesis of intermediate **I-A13-C**

Following the procedure for synthesizing intermediate **I-A10-C, I-A13-C** (20 mg, yield: 13.4%) was obtained as a white solid from the crude **I-A13-B** as obtained in the previous step. LCMS: (ESI) *m/z* = 1089.4 $[M+2H]/2^+$; 924 $[M+2H-330]/2^+$.

HPLC purification conditions: preparative column: Xbridge C18, 19 × 250 mm, 10 μm, 130 Å; mobile phase: A: water (containing 10 mmol/L $NH_4HCO_3$), B: acetonitrile; gradient program: ramp from 43% B to 53% B over 10 min.

Synthesis of intermediate **I-A13-E**

Following the procedure for synthesizing intermediate **I-A10-E, I-A13-E** (20 mg, yield: quantitative) was obtained as a white solid from **I-A13-C** (20 mg, 0.0090 mmol). LCMS: (ESI) *m/z* = 833.0 $[M+2H]/2^+$; 731.4 $[M+2H-204]/2^+$.

Synthesis of intermediate **I-A13-5FAM**

Following the procedure for synthesizing **I-A10-5FAM, I-A13-5FAM** (10.0 mg, yield: 29.4%) was obtained as an orange solid from **I-A13-E** (20 mg, 0.0090 mmol). LCMS: (ESI) *m/z* = 1031 $[M+H+K]/2^+$; 1012.7 $[M+2H]/2^+$; 687.0 $[M+2H+K]/3^+$; 675.3 $[M+3H]/3^+$.

HPLC purification conditions: preparative column: Gemini C18 21.2 mm × 250 mm, 10 μm, 110 Å; mobile phase: A: water (containing 10 mmol/L $NH_4HCO_3$), B: acetonitrile; gradient program: ramp from 5% B to 35% B over 10 min.

HPLC purity: 24.2020 % (214 nm), RT = 14.195 min; 71.6996 % (214 nm), RT = 13.440 min

Analysis conditions were the same as those for **I-A10-5FAM.**

Synthesis of **I-B** class molecules

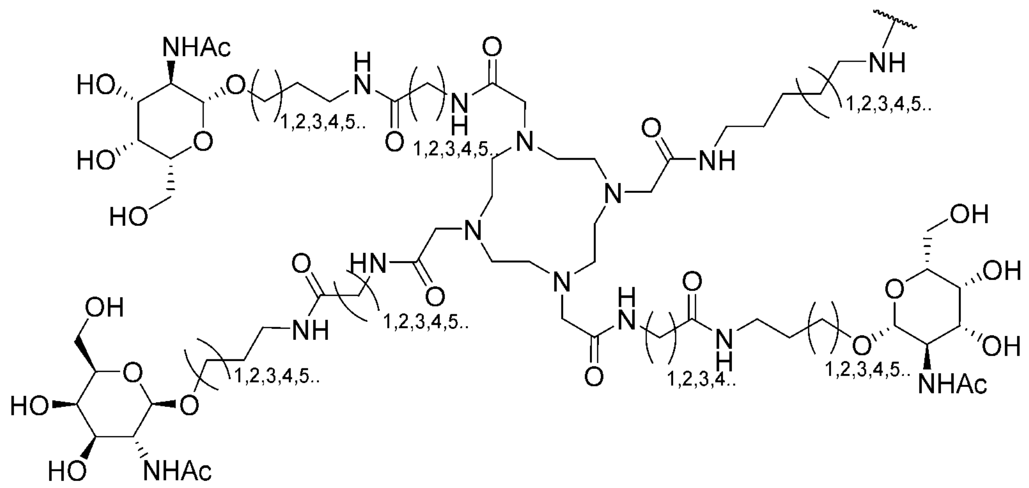

**4.1** Synthesis of **I-B11-5FAM**

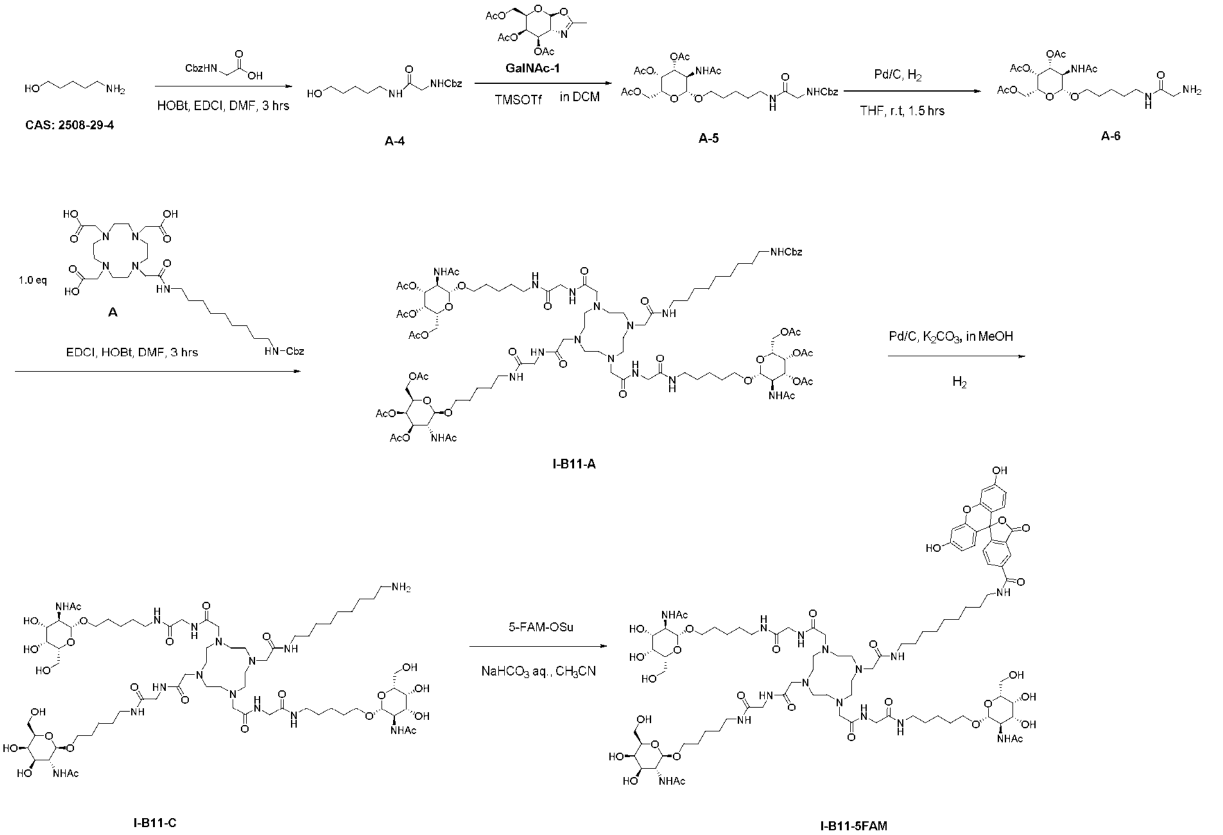


Synthesis of intermediate **A-4**

5-aminopentan-1-ol (5.0 g, 23.9 mmol) was dissolved in DMF (50 mL), followed by sequential addition of N-Cbz-glycine (2.96 g, 28.7 mmol), HOBt (4.84 g, 35.9 mmol), EDCI (6.9 g, 35.9 mmol), and DIPEA (14.7 mL, 81.1 mmol). The mixture was stirred at room temperature for 3 h. The reaction was quenched with $H_2O$ (50 mL) and extracted with ethyl acetate (60 mL). The organic layer was washed with 5% aqueous $NaHCO_3$ (40 mL), dried over anhydrous $Na_2SO_4$, and concentrated. The crude product was purified by reversed-phase column chromatography to afford compound **A-4** (6.3 g, 90% yield) as a white solid. LCMS: (ESI) *m*/*z* = 295.1 $[M+H]^+$.

Purification conditions: column: 120 g C18; mobile phase: A: water (containing 10 mmol/L $NH_4HCO_3$), B: acetonitrile; gradient program: ramp from 5% B to 95% B over 20 min.

Synthesis of intermediate **A-5**

In a 100 mL three-necked flask, activated 4 Å molecular sieves (3.0 g), freshly prepared **GalNAc-1** (2.0 g, 6.079 mmol), and intermediate **A-4** (1.96 g, 6.687 mmol) were added. The system was purged with $N_2$ (three cycles), followed by addition of anhydrous 1,2-dichloroethane (20 mL). After stirring at room temperature for 10 min, TMSOTf (110 $\mu$L, 0.608 mmol) was added, and the mixture was stirred at room temperature for 3 h. The reaction mixture was filtered to remove molecular sieves. The filtrate was quenched with saturated aqueous $NaHCO_3$ (15 mL), and the organic layer was separated. The aqueous phase was extracted with DCM (20 mL $\times$ 2). The combined organic phases were dried over anhydrous $Na_2SO_4$ and concentrated. The crude product was purified by reversed-phase column chromatography to afford intermediate **A-5** (400 mg, yield 10.5%) as a white solid. LCMS: (ESI) *m*/*z* = 624.1 $[M+H]^+$.

Purification conditions: column: 120 g C18; mobile phase: A: water (containing 10 mmol/L $NH_4HCO_3$), B: acetonitrile; gradient program: ramp from 20% B to 60% B over 20 min.

Synthesis of intermediate **A-6**

In a 50 mL round-bottom flask, intermediate **A-5** (400 mg, 0.642 mmol) and Pd/C (10%, 40 mg) were added. The system was purged with $H_2$ (twice), followed by addition of THF (5 mL). The mixture was stirred under $H_2$ atmosphere at room temperature for 90 min. The reaction mixture was filtered and concentrated to afford the crude product **A-6** (320 mg) as a pale black oily liquid, which was directly used in the next step. LCMS: (ESI) *m*/*z* = 490.1 $[M+H]^+$.

Synthesis of intermediate **I-B11-A**

Compound A (50 mg, 0.074 mmol) was dissolved in DMF (2 mL), followed by sequential addition of HOBt (35 mg, 0.258 mmol), EDCI (45 mg, 0.258 mmol), DIPEA (88 μL, 0.516 mmol), and intermediate **A-6** (126 mg, 0.258 mmol). The mixture was stirred at room temperature for 3 h. The reaction mixture was directly purified by preparative reversed-phase column chromatography to afford **I-B11-A** (50 mg, yield 32.3%) as a white solid. LCMS: (ESI) *m*/*z* = 1047.0 $[M+2H]/2^+$.
Preparation conditions: HPLC preparative column: Xbridge C18, 19 × 250 mm, 10 μm, 130 Å; mobile phase: A: water (containing 10 mmol/L $NH_4HCO_3$), B: acetonitrile; gradient program: ramp from 40% B to 50% B over 10 min.

Synthesis of intermediate **I-B11-C**

In a 50 mL round-bottom flask, **I-B11-A** (50 mg, 0.024 mmol), $K_2CO_3$ (10 mg, 0.150 mmol), and Pd/C (10%, 12 mg) were sequentially added. Methanol (5 mL) was then introduced, and the mixture was stirred under $H_2$ atmosphere for 3 h. The reaction mixture was filtered and concentrated to afford **I-B11-C** (40 mg, crude product) as a white solid. LCMS: (ESI) *m*/*z* = 791.0 $[M+2H]/2^+$.

Synthesis of compound **I-B11-5FAM**

The crude product **I-B11-C** obtained from the previous step (40 mg, 0.024 mmol) was dissolved in a mixture of water (2 mL) and acetonitrile (0.5 mL), followed by sequential addition of SFAM-OSu (14 mg, 0.030 mmol) and saturated aqueous $NaHCO_3$ (0.5 mL). The mixture was stirred at room temperature for 60 min. The reaction mixture was directly purified by preparative chromatography to afford **I-B11-5FAM** (9.5 mg, yield 19.8%) as an orange-yellow solid. LCMS: (ESI) *m*/*z* = 989 $[M+H+K]/2^+$.
Preparation conditions: HPLC preparative column: Gemini C18 21.2 mm × 250 mm, 10 μm, 110 Å; mobile phase: A: water (containing 10 mmol/L $NH_4HCO_3$), B: acetonitrile; gradient program: ramp from 17% B to 27% B over 20 min.
HPLC purity: 9.5962% (214 nm), RT = 13.531 min; 88.8773% (214 nm), RT = 13.301 min
Analysis conditions were the same as those for **I-A10-5FAM.**

**4.2** Synthesis of **I-B12-5FAM**

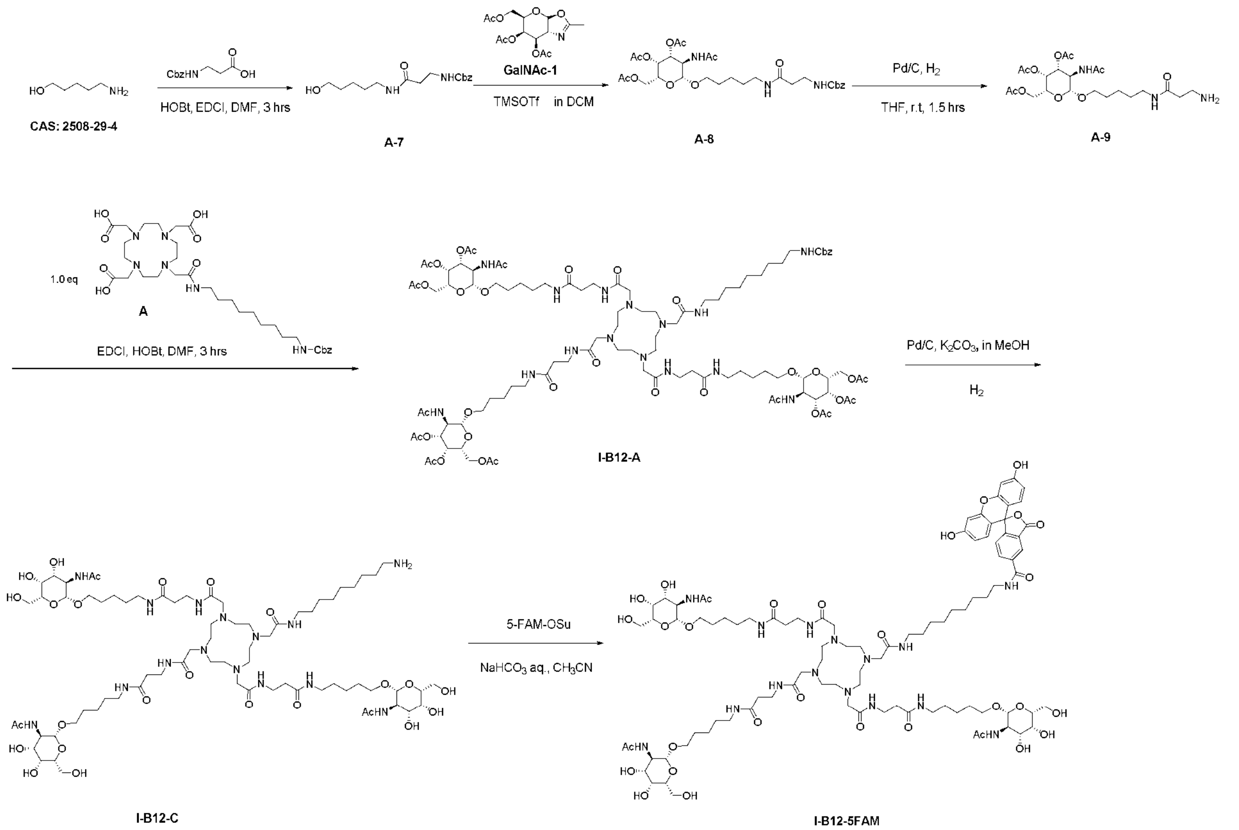


Synthesis of intermediate **A-7**

5-aminopentan-1-ol (5.0 g, 22.4 mmol) was dissolved in DMF (50 mL), followed by sequential addition of N-Cbz-β alanine (2.77 g, 26.9 mmol), HOBt (4.54 g, 33.6 mmol), EDCI (6.46 g, 33.6 mmol), and DIPEA (18.3 mL, 101. mmol). The mixture was stirred at room temperature for 3 h. The reaction was quenched with $H_2O$ (50 mL) and extracted with ethyl acetate (60 mL). The organic layer was washed with 5% aqueous $NaHCO_3$ (40 mL), dried over anhydrous $Na_2SO_4$, and concentrated. The crude product was purified by reversed-phase column chromatography to afford compound **A-7** (7.0 g, 92% yield) as a white solid. LCMS: (ESI) *m*/*z* = 309.1 $[M+H]^+$.

Purification conditions: column: 120 g C18; mobile phase: A: water (containing 10 mmol/L $NH_4HCO_3$), B: ACN; gradient program: ramp from 5% B to 95% B over 20 min.

Synthesis of intermediate **A-8**

In a 100 mL three-necked flask, activated 4 Å molecular sieves (3.0 g), DCM (20 mL), freshly prepared **GalNAc-1** (2.0 g, 6.08 mmol), and intermediate **A-7** (2.0 g, 6.69 mmol) were added. After stirring at room temperature for 10 min, TMSOTf (110 μL, 0.608 mmol) was added, and the mixture was stirred at room temperature for 3 h. The reaction mixture was filtered to remove molecular sieves. The filtrate was quenched with saturated aqueous $NaHCO_3$ (15 mL), and the organic layer was separated. The aqueous phase was extracted with DCM (20 mL × 2). The combined organic phases were dried over anhydrous $Na_2SO_4$ and concentrated. The crude product was purified by reversed-phase column chromatography to afford intermediate **A-8** (2.0 g, yield 51.7%) as a white solid. LCMS: (ESI) *m*/*z* = 638.2 $[M+H]^+$.

Purification conditions: column: 120 g C18; mobile phase: A: water (containing 10 mmol/L $NH_4HCO_3$), B: acetonitrile; gradient program: ramp from 5% B to 65% B over 20 min.

Synthesis of intermediate **A-9**

In a 50 mL round-bottom flask, intermediate **A-8** (400 mg, 0.628 mmol) and Pd/C (10%, 40 mg) were added. The system was purged with $H_2$ (twice), followed by addition of THF (5 mL). The mixture was stirred under $H_2$ atmosphere at room temperature for 90 min. The reaction mixture was filtered and concentrated to afford the crude product **A-9** (320 mg) as a pale black oily liquid, which was directly used in the next step. LCMS: (ESI) m/z = 504.1 $[M+H]^+$.

Synthesis of intermediate **I-B12-A**

Compound **A** (45 mg, 0.066 mmol) was dissolved in DMF (2 mL), followed by sequential addition of HOBt (31 mg, 0.232 mmol), EDCI (45 mg, 0.232 mmol), DIPEA (79 μL, 0.464 mmol), and intermediate **A-9** (117 mg, 0.232 mmol). The mixture was stirred at room temperature for 3 h. The reaction mixture was directly purified by preparative reversed-phase column chromatography to afford **I-B12-A** (72 mg, yield 51.1%) as a white solid. LCMS: (ESI) *m/z* = 1068.4 [M+2H]/2+.
Preparation conditions: HPLC preparative column: Xbridge C18, 19 × 250 mm, 10 μm, 130 Å; mobile phase: A: water (containing 10 mmol/L $NH_4HCO_3$), B: acetonitrile; gradient program: ramp from 39% B to 49% B over 10 min.

Synthesis of intermediate **I-B12-C**

In a 50 mL round-bottom flask, **I-B12-A** (72 mg, 0.034 mmol), $K_2CO_3$ (28 mg, 0.202 mmol), and Pd/C (10%, 5 mg) were sequentially added. Methanol (2 mL) was then introduced, and the mixture was stirred under $H_2$ atmosphere for 3 h. The reaction mixture was filtered and concentrated to afford **I-B11-C** (60 mg, crude product) as a white solid. LCMS: (ESI) *m/z* = 812.1 [M+2H]/2+; 711.0 [M+2H]/2+; 541.8 [M+3H]/3+.

Synthesis of compound **I-B12-5FAM**

The crude product **I-B12-C** obtained from the previous step (40 mg, 0.025 mmol) was dissolved in a mixture of water (1.2 mL) and acetonitrile (0.3 mL), followed by sequential addition of 5FAM-OSu (14 mg, 0.029 mmol) and saturated aqueous $NaHCO_3$ (0.3 mL). The mixture was stirred at room temperature for 60 min. The reaction mixture was directly purified by preparative chromatography to afford **I-B11-5FAM** (12.5 mg, yield 25.3%) as an orange-yellow solid. LCMS: (ESI) *m/z* = 1010.3 [M+H+K]/2+; 991.0 [M+2H]/2+; 673.7 [M+2H+K]/3+; 661.3 [M+3H]/3+.
Preparation conditions: HPLC preparative column: Gemini C18 21.2 mm × 250 mm, 10 μm, 110 Å; mobile phase: A: water (containing 10 mmol/L $NH_4HCO_3$), B: acetonitrile; gradient program: ramp from 17% B to 27% B over 20 min.
HPLC purity: 93.1525 % (214 nm), RT = 13.435 min; 4.4401 % (214 nm), RT = 13.806 min
Analysis conditions were the same as those for **I-A10-5FAM.**

**4.3** Synthesis of **I-B12-Cy5**

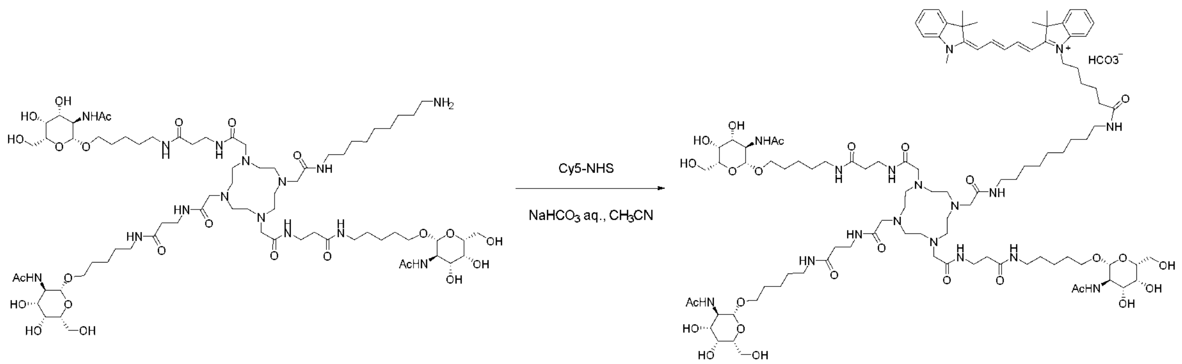


I-B12-C

I-B12-Cy5

Following the procedure for synthesizing **I-A12-Cy5**, starting from **I-B12-E** (0.0124 mmol), **I-B12-Cy5** (5.0 mg, yield 19.2%) was obtained as a deep blue solid. LCMS: (ESI) *m/z* = 1045.3 [M++H]/2+; 697.0 [M++2H]/3+.
HPLC purification conditions: column: Xbridge C18, 19 × 250 mm, 10 μm, 130 Â; mobile phase: A: water (containing 0.05% TFA), B: acetonitrile (containing 0.05% TFA); gradient program: ramp from 31% B to 41% B over 20 min.
HPLC purity: 90.9250 % (214 nm), RT = 17.723 min (M); 8.5560 % (214 nm), RT = 17.878 min (M+K).

Synthesis of **I-C** class molecules

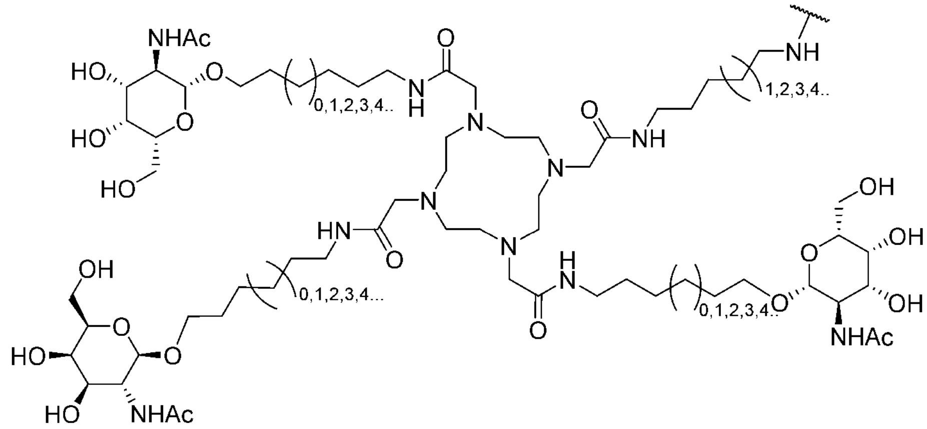

**5.1** Synthesis of compound **I-C10-5FAM**

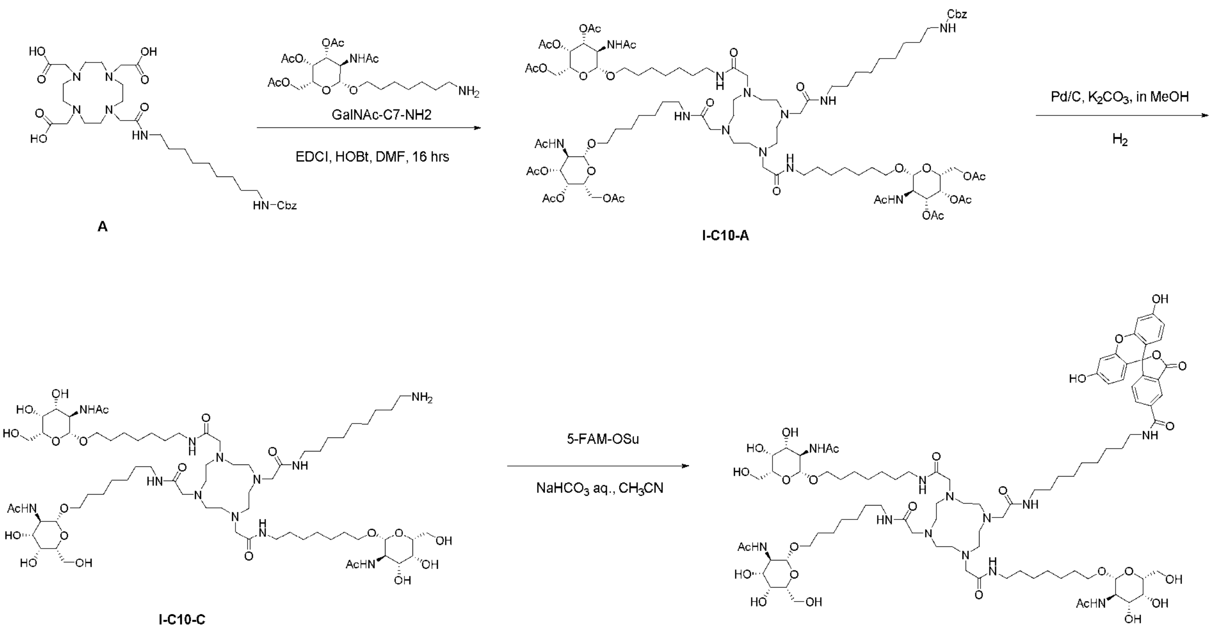


A

I-C10-A

I-C10-C

I-C10-5FAM

Synthesis of intermediate **I-C10-A**

Compound **A** (100 mg, 0.147 mmol) was dissolved in DMF (3 mL), followed by sequential addition of HOBt (138 mg, 1.029 mmol), EDCI (198 mg, 1.029 mmol), and **GalNAc-C7-$NH_2$** (408 mg, 0.884 mmol). The mixture was stirred at room temperature for 16 h. The reaction was quenched with $H_2O$ (5 mL) and extracted with ethyl acetate (8 mL). The organic layer was washed with 5% aqueous $NaHCO_3$ (10 mL), dried over anhydrous $Na_2SO_4$, and concentrated. The residue was purified by preparative chromatography to afford **I-C10-A** (170 mg, yield 57.6%) as a white solid. LCMS: (ESI) *m/z* = 1003.0 $[M+2H]/2^+$.

Preparation conditions: HPLC preparative column: Xbridge C18, 19 × 250 mm, 10 μm, 130 Å; mobile phase: A: water (containing 10 mmol/L $NH_4HCO_3$), B: acetonitrile; gradient program: ramp from 20% B to 80% B over 20 min.

Synthesis of intermediate **I-C10-C**

In a 50 mL round-bottom flask, **I-C10-A** (50 mg, 0.025 mmol), $K_2CO_3$ (21 mg, 0.150 mmol), and Pd/C (10%, 12 mg) were added. Methanol (5 mL) was then introduced, and the mixture was stirred under $H_2$ atmosphere for 3 h. The reaction mixture was filtered and concentrated to afford **I-C10-C** (50 mg, crude product) as a white solid. LCMS: (ESI) *m/z* = 766.5 $[M+H+K]/2^+$. 511.6 $[M+2H+K]/3^+$.

Synthesis of compound **I-C10-5FAM**

The crude product **I-C10-C** obtained from the previous step (50 mg, 0.025 mmol) was dissolved in a solution of saturated aq. $NaHCO_3$ in $CH_3CN/H_2O$ (1.8 mL, v/v = 1:4:4), followed by addition of **5-FAM-OSu** (19 mg, 0.040 mmol). The mixture was stirred at room temperature for 1 h. The reaction mixture was directly purified by preparative chromatography to afford **I-C10-5FAM** (23 mg, yield 37.7%) as an orange-yellow solid. LCMS: (ESI) *m/z* = 946 $[M+H+K]/2^+$.

Preparation conditions: HPLC preparative column: Gemini C18 21.2 mm × 250 mm, 10 μm, 110 Å; mobile phase: A: water (containing 10 mmol/L $NH_4HCO_3$), B: acetonitrile; gradient program: ramp from 22% B to 32% B over 20 min.

HPLC purity: 7.4271% (214 nm), RT = 14.574 min; 91.5431% (214 nm), RT = 15.705 min.

Synthesis of **I-D** class molecules

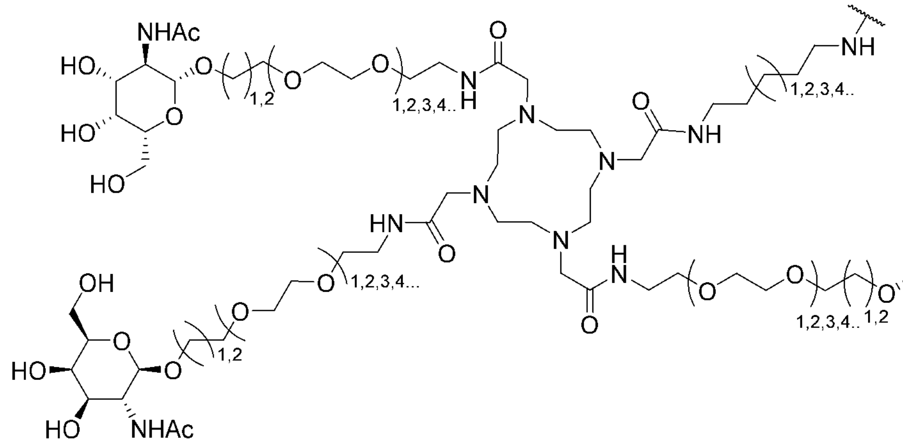

**6.1** Synthesis of compound **I-D11-5FAM**

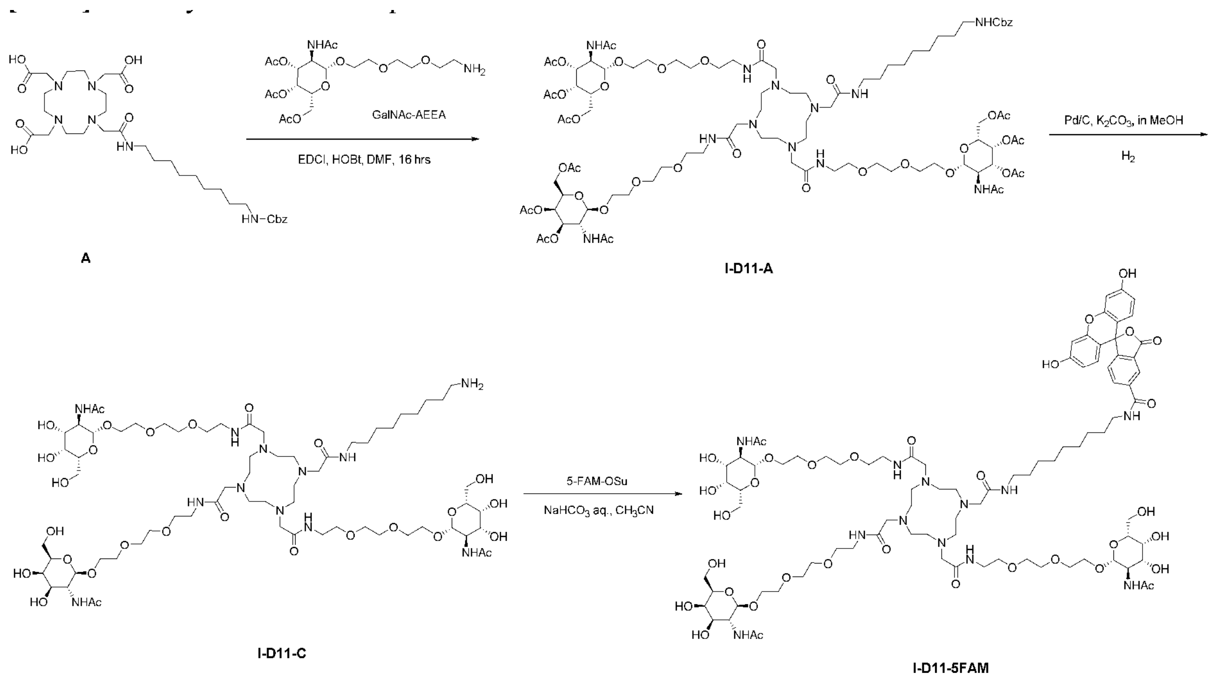


A

I-D11-A

I-D11-C

I-D11-5FAM

Synthesis of GalNAc-Ligand-Loaded Resin for Oligonucleotide Solid-Phase Synthesis

7.1 Synthesis of intermediate **A-13**

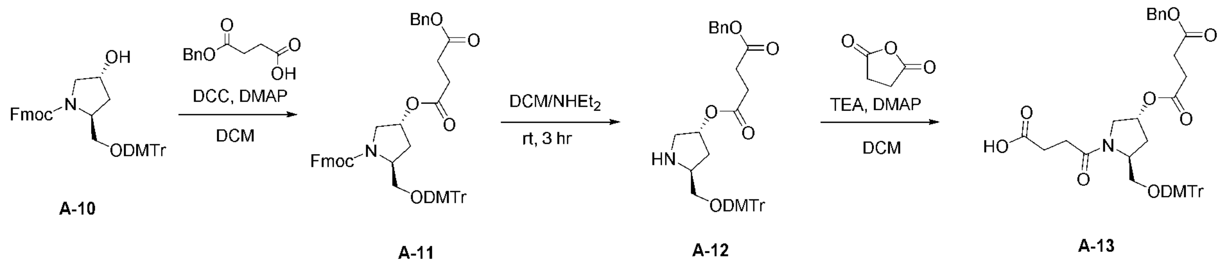


A-10 A-11 A-12 A-13

Compound **A-10** (3.2 g, 5.0 mmol, 1.0 equiv.) was dissolved in DCM (50 mL), followed by sequential addition of benzyl succinate (1.25 g, 6.0 mmol, 1.2 equiv.), DCC (1.24 g, 6.0 mmol, 1.2 equiv.), and DMAP (122 mg, 1 mmol, 0.2 equiv.). The reaction mixture was stirred at room temperature overnight, then concentrated. The residue was dissolved in DCM (20 mL) with stirring, and the insoluble white solid was filtered off. The filtrate was concentrated and dried to afford crude product **A-11** as a pale-yellow oily liquid, which was directly used in the next step.

The crude intermediate **A-11** from the previous step was dissolved in DCM (25 mL), followed by addition of diethylamine (25 mL). The mixture was stirred at room temperature for 3 h. After reaction completion confirmed by monitoring, the mixture was concentrated and dried to afford a crude product **A-12** as a pale-yellow oily liquid, which was directly used in the next step.

The crude intermediate **A-12** from the previous step was dissolved in DCM (100 mL), followed by sequential addition of succinic anhydride (1.5 g, 15 mmol), triethylamine (2.1 mL, 15 mmol), and DMAP (122 mg). The reaction mixture was stirred at room temperature overnight and concentrated. The residue was purified by reversed-phase column chromatography to afford intermediate **A-13** (2.95 g, overall yield 83% over three steps) as a white solid. LCMS: (ESI) *m/z* = 708.2 $[M-H]^-$.

Reversed-phase purification conditions: column: 120 g C18 column; Mobile phase: A: water (containing 10 mmol/L $NH_4HCO_3$); B: ACN; Gradient: ramped from 20% B to 60% B over 20 min.

7.2 Synthesis of resin **I-A12-H-PS**

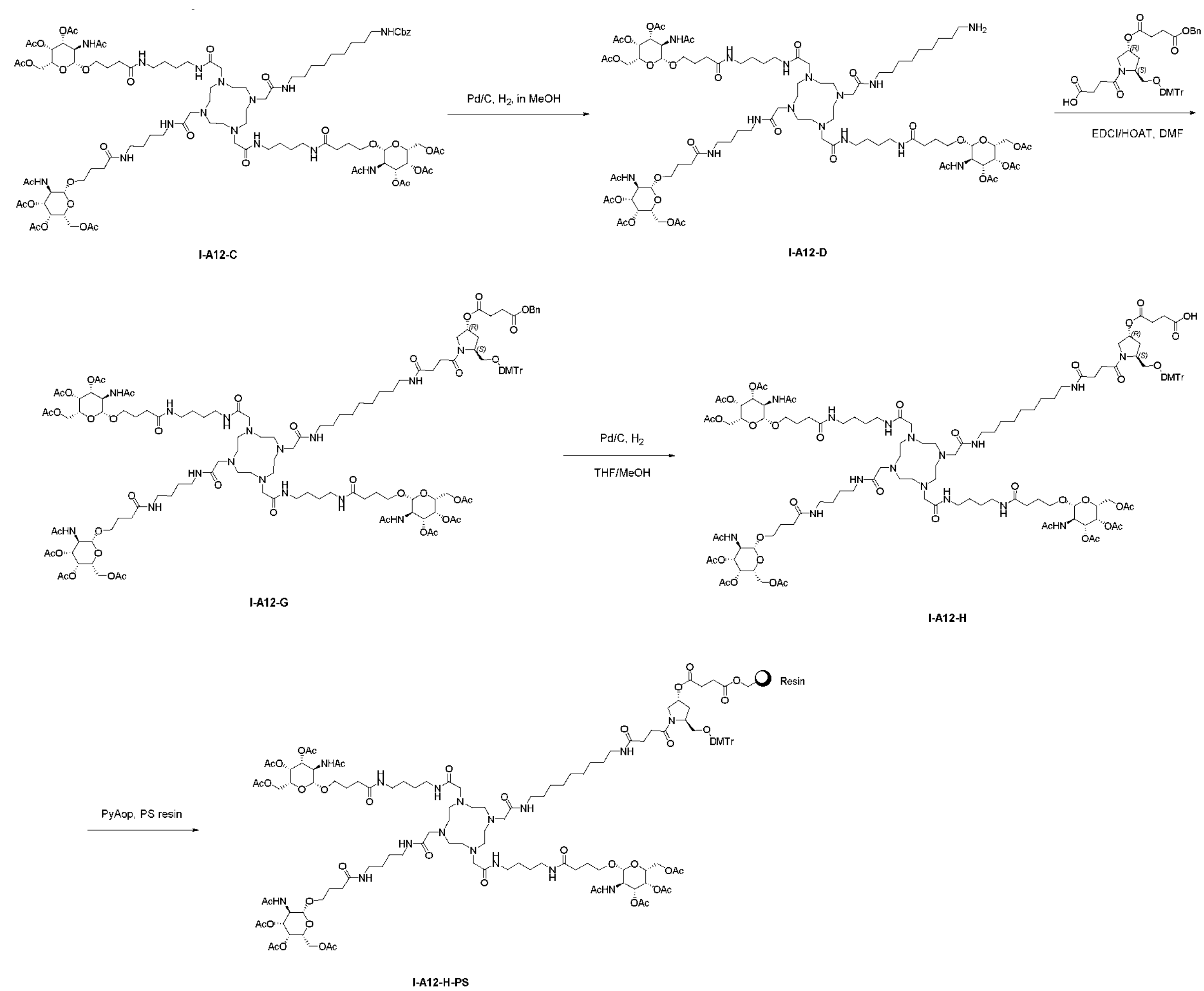


I-A12-C

I-A12-D

I-A12-G

I-A12-H

I-A12-H-PS

Synthesis of intermediate **I-A12-D**

The intermediate **I-A12-C** (900 mg, 0.422 mmol, 1.0 equiv.) was dissolved in a mixture of THF (40 mL) and methanol (5 mL), followed by addition of Pd/C (10%, 200 mg). The reaction was stirred under $H_2$ atmosphere for 3 h. The mixture was filtered and concentrated to afford a crude product **I-A12-D**, which was directly used in the next step. LCMS: (ESI) *m/z* = 1001.7 $[M+2H]/2^+$; 681.1 $[M+3H]/3^+$.

Synthesis of intermediate **I-A12-G**

The intermediate **A-13** (359 mg, 0.506 mmol, 1.2 equiv.) was dissolved in DMF (10 mL), followed by sequential addition of **I-A12-D** (863 mg, 0.422 mmol), EDCI (97 mg, 0.506 mmol), HOAt (69 mg, 0.506 mmol), and DIPEA (430 μL, 2.52 mmol). The mixture was stirred at room temperature for 3 h. Reaction completion was confirmed by LCMS, and the mixture was directly purified by preparative chromatography to afford **I-A12-G** (420 mg, yield 37%) as a white solid. LCMS: (ESI) *m/z* = 1196.1 $[M+2H]/2^+$; 797.7 $[M+3H]/3^+$. $^1H$ NMR (400 MHz, DMSO) δ 8.08-7.73 (m, 11 H, 11NH), 7.37-7.15 (m, 14 H, DMTr+Bn), 6.88 (d, *J* = 7.2 Hz, 4 H, DMTr), 5.38-5.31 (m, 1H, proline-CH-OH), 5.21 (d, *J* = 3.2 Hz, 3H, GalNAc-OCHO-), 5.09 (s, 2H, Ph-CH2-), 4.96 (dd, *J* = 11.6, 3.2 Hz, 3H, GalNAc), 4.48 (d, *J* = 7.6 Hz, 3H, GalNAc), 4.25-4.07 (m, 2H), 4.02 (brs, 9H), 3.93-3.70 (m, 5H), 3.73 (brs, 6H), 3.70-3.53 (m, 4H), 3.49-3.37 (m, 5H), 3.23-2.87 (m, 28H), 2.70-2.53 (m, 17H), 2.46-2.02 (m, 5H), 2.10 (s, 9H, GalNAc-OCOCH3), 1.99 (s, 9H, GalNAc-OCOCH3), 1.89 (s, 9H, GalNAc-OCOCH3), 1.78 (s, 9H, GalNAc-NHCOCH3), 1.74-1.61 (m, 7H), 1.36 (brs, 17H), 1.21 (brs, 11H).

Preparation conditions: HPLC column: Xbridge C18, 19 × 250 mm, 10 μm, 130 Å; mobile phase: A: water (containing 10 mmol/L $NH_4HCO_3$), B: acetonitrile; gradient program: ramp from 51% B to 61% B over 20 min.

Synthesis of intermediate **I-A12-H**

The intermediate **I-A12-G** (400 mg, 0.149 mmol, 1.0 equiv.) was dissolved in a mixture of THF (12 mL) and

methanol (4.0 mL), followed by addition of Pd/C (10%, 100 mg). The reaction was stirred under $H_2$ atmosphere for 3 h. The mixture was filtered, concentrated, and the crude product was purified by preparative chromatography to afford **I-A12-H** (350 mg, yield 90%) as a white solid. LCMS: (ESI) *m/z* = 1321.2 $[M+H+K]/2^+$, 1150.7 $[M+2H-DMTr]/2^+$. $^1H$ NMR (400 MHz, DMSO) δ 8.08-7.73 (m, 11 H, 11NH), 7.34-7.15 (m, 9H, DMTr), 6.88 (d, *J* = 8.8 Hz, 4 H, DMTr), 5.38-5.31 (m, 1H, proline-CH-OH), 5.21 (d, *J* = 3.2 Hz, 3H, GalNAc-OCHO-), 4.95 (dd, *J* = 11.2, 3.2 Hz, 3H, GalNAc), 4.48 (d, *J= 8.0* Hz, 3H, GalNAc), 4.25-4.07 (m, 2H), 4.02 (brs, 9H), 3.93-3.70 (m, 5H), 3.73 (brs, 6H), 3.70-3.53 (m, 5H), 3.49-3.37 (m, 5H), 3.23-2.87 (m, 28H), 2.70-2.39 (m, 19H), 2.46-2.02 (m, 5H), 2.10 (s, 9H, GalNAc-OCOCH3), 1.99 (s, 9H, GalNAc-OCOCH3), 1.89 (s, 9H, GalNAc-OCOCH3), 1.78 (s, 9H, GalNAc-NHCOCH3), 1.74-1.61 (m, 7H), 1.37 (brs, 17H), 1.22 (brs, 11H).

HPLC preparation conditions: column: Xbridge C18, 19 × 250 mm, 10 µm, 130 Å; mobile phase: A: water (containing 10 mmol/L $NH_4HCO_3$), B: acetonitrile; gradient program: ramp from 39% B to 49% B over 20 min.

HPLC purity: >99% % (214 nm), RT = 14.992 min
Mobile phase: A: water (10 mM $NH_4HCO_3$), B: acetonitrile
Gradient: 5% B (equilibration for 1 min), ramped to 95% B over 20 min, ramp to 95% B over 2min and held for 5 min.
Flow rate: 1.0 mL/min
HPLC analysis column: XBridge peptide BEH column C18, 4.6 × 150 mm, 3.5 µm, 300 Å
Column temperature: 45°

Synthesis of resin **I-A12-H-PS**

Macroporous aminomethyl resin (250 mg, 0.4 mmol/g, 2.17 equiv.) was placed in a 5 mL fritted plastic syringe, soaked in DCM (3 mL) for 30 min, and washed via aspiration-shaking-expulsion cycles with DCM (3 mL × 3) followed by DMF (3 mL × 3). A solution of**I-A12-H** (125 mg, 0.0461 mmol, 1.0 equiv.), DIPEA (39 µL, 0.231 mmol, 5.0 equiv.), and PyAOP (52 mg, 0.101 mmol, 2.2 equiv.) in DMF (3 mL) was aspirated into the pre-washed resin. The mixture was agitated at 25°C for 24 h. Kaiser test showed faint blue coloration. The resin was washed with DMF (3 mL × 6), then capped by agitation with pyridine/acetic anhydride mixture (3 mL, 2:1) for 3 h. After washing with DMF (3 mL × 6), Kaiser test indicated no coloration. The resin was further washed with DCM (3 mL × 3) and MTBE (3 mL × 3), then vacuum-dried overnight to afford off-white resin **I-A12-H-PS** (415 mg).

## Quantitation of Resin Loading

Exactly 18.2 mg of the resin was treated with 8.0 mL of 0.48 N HCl/MeCN solution under gentle agitation for 10 min; 2.0 mL of the supernatant was collected, and LCMS analysis showed an absorption peak area of 1340.29. The loading capacity was calculated as 164.8 µmol/g using the formula:

$$\text{Loading} = \frac{\frac{1340.29 - 51.991}{3435.6} \times 8}{18.2 \times 10^{-3}} \ \mu\text{mol/g} = 164.8\ \mu\text{mol/g}$$

## Resin Cleavage

The residual resin after DMTr removal was washed with acetonitrile (2 mL × 3), treated with concentrated aqueous ammonia (2 mL), and agitated at 60°C for 3 h. The mixture was filtered, and the filtrate was analyzed by HPLC, confirming the resin-bound product as high-purity GalNAc ligand (**I-A12-I**). LCMS: (ESI) *m/z* = 931.0 $[M+H+K]/2^+$; 911.7 $[M+2H]/2^+$; 608.3 $[M+3H]/3^+$. HPLC purity: 87.80% % (214 nm), RT = 9.588 min (M); 12.20% % (214 nm), RT = 10.398 min.

Mobile phase: A: water (10 mM $NH_4HCO_3$), B: acetonitrile
Gradient: 5%B (equilibration for 3 min), ramped to 65% B over 20 min and held for 5 min.
Flow rate: 1.0 mL/min
HPLC analysis column: XBridge peptide BEH column C18, 4.6 × 150 mm, 3.5 µm, 300 Å
Column temperature: 60°C

7.3 Synthesis of resin **I-B12-F-PS**

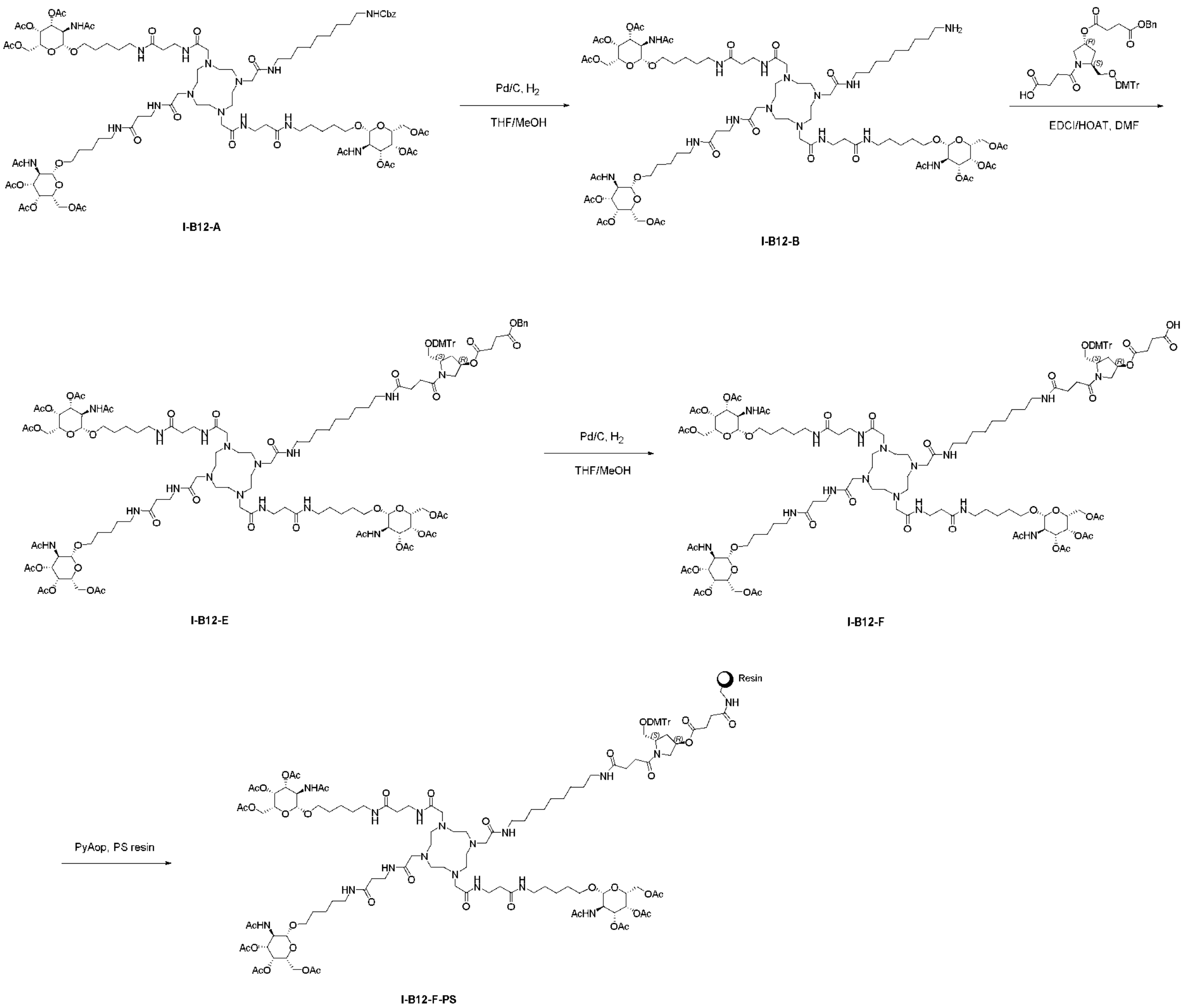


Synthesis of intermediate **I-B12-B**

The intermediate **I-B12-A** (400 mg, 0.187 mmol, 1.0 equiv.) was dissolved in THF (10 mL) and MeOH (1 mL). To this solution was added Pd/C (10%, 100 mg), and the reaction mixture was stirred under a hydrogen atmosphere for 16 hours. The reaction mixture was filtered and concentrated to afford **I-B12-B** as a crude product, which was used directly in the next step. LCMS: (ESI) *m/z* = 1001.7 $[M+2H]/2^+$; 668.2 $[M+3H]/3^+$.

Synthesis of intermediate **I-B12-E**

The intermediate **A-13** (153 mg, 0.22 mmol, 1.2 equiv.) was dissolved in DMF (10 mL), followed by sequential addition of **I-B12-B** (crude from the previous step), EDCI (42 mg, 0.22 mmol), HOAt (30 mg, 0.22 mmol), and DIPEA (92 $\mu$L, 0.54 mmol). The reaction mixture was stirred at room temperature for 3 hours. Reaction completion was confirmed by LCMS analysis, and the mixture was directly purified by preparative column chromatography to afford **I-B12-E** (350 mg, 72% yield) as a white solid. LCMS: (ESI) *m/z* = 1196.1 $[M+2H]/2^+$.

HPLC preparation conditions: column: Xbridge C18, 19 × 250 mm, 10 $\mu$m, 130 Å; mobile phase: A: water (containing 10 mmol/L $NH_4HCO_3$), B: acetonitrile; gradient program: ramp from 54% B to 64% B over 20 min.

Synthesis of intermediate **I-B12-F**

**I-B12-E** (200 mg, 0.074 mmol, 1.0 equiv.) was dissolved in THF (6 mL) and MeOH (2.0 mL), followed by addition of Pd/C (10%, 40 mg). The reaction mixture was stirred under a hydrogen atmosphere for 6 hours. After filtration and concentration, the crude product was purified by preparative HPLC to afford **I-B12-F** (150 mg, 78% yield) as a white solid. LCMS: (ESI) *m/z* = 1321.2 $[M+H+K]/2^+$; 1169.7 $[M+2H-DMTr]/2^+$.

HPLC preparation conditions: column: Xbridge C18, 19 × 250 mm, 10 $\mu$m, 130 Å; mobile phase: A: water

(containing 10 mmol/L $NH_4HCO_3$), B: acetonitrile; gradient program: ramp from 39% B to 49% B over 20 min.

HPLC purity: >99% % (214 nm), RT = 15.734 min

Analysis conditions were the same as those for **I-A12-H.**

Synthesis of resin **I-B12-F-PS**

**I-B12-F-PS** was synthesized following a similar procedure to resin **I-A12-H-PS.** The loading capacity was determined by the same method and recorded as 118.0 μmol/g.

$$\text{Loading} = \frac{\frac{938.75 \times 2 - 51.991}{3435.6} \times 4}{18.0 \times 10^{-3}}\ \mu mol/g = 118\ \mu mol/g$$

The residual resin after DMTr removal was washed with acetonitrile (2 mL × 3), treated with concentrated aqueous ammonia (2 mL), and agitated at 60°C for 3 h. The mixture was filtered, and the filtrate was analyzed by HPLC, confirming the resin-bound product as high-purity peptides. LCMS LCMS: (ESI) *m/z* = 930.8 $[M+H+K]/2^+$911.8 $[M+2H]/2^+$; 608.3 $[M+3H]/3^+$.

**Oligonucleotide Conjugates Synthesis using GalNAc Ligand-Loaded Resins**

Solid-phase synthesis of GalNAc-siRNA-SS-3'using a DNA/RNA synthesizer according to the following protocol

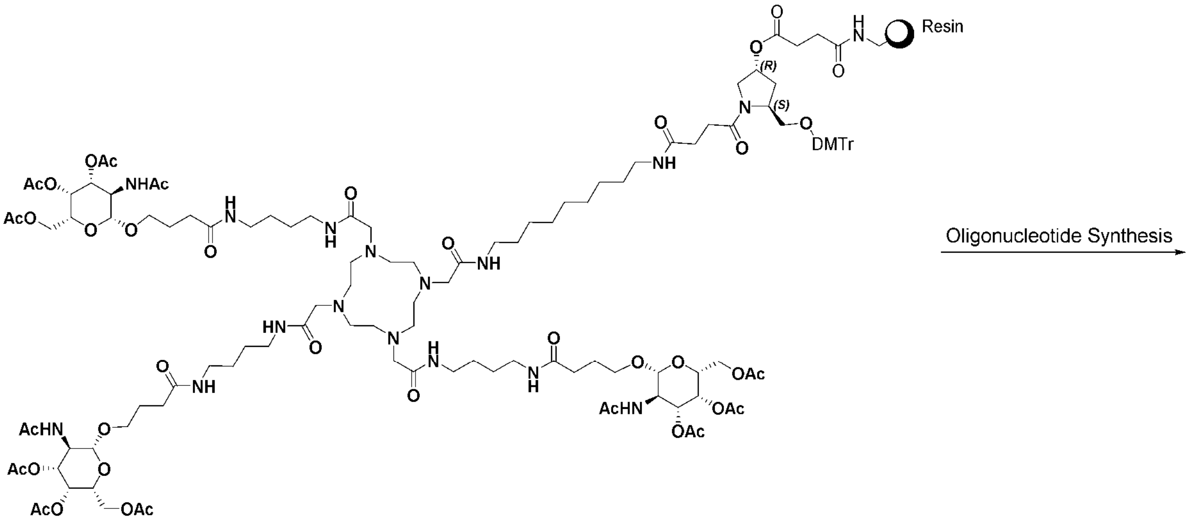


**I-A12-H-PS**

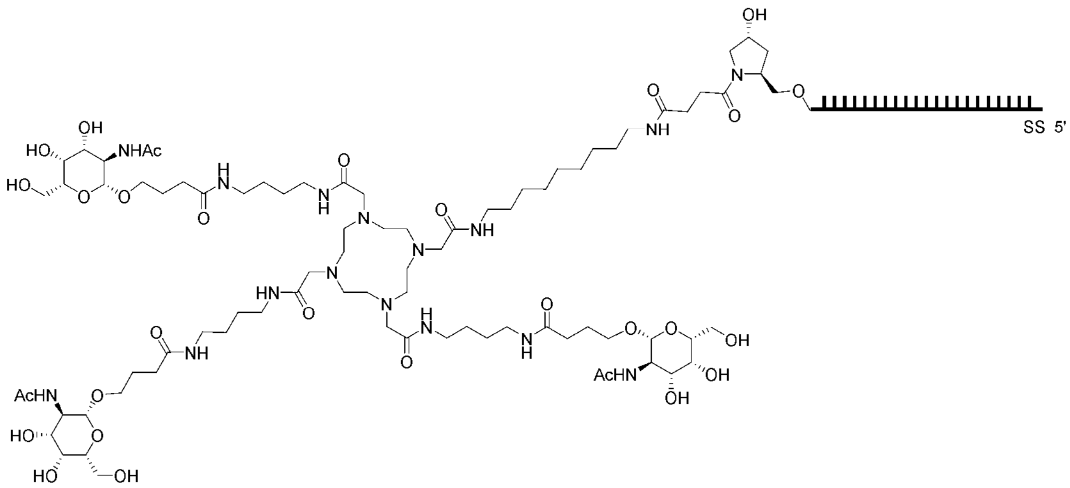

**I-A12-I-siRNA-SS'**

The synthesis for siRNA was identical to standard phosphoramidite solid-phase synthesis (all synthesized by Suzhou Biosyntech Co., Ltd.), comprising four iterative steps: deprotection, coupling, capping, and oxidation/sulfurization. For the sense strand incorporating the GalNAc delivery ligand, prefunctionalized amino resin supports (**I-A12-H-PS, I-B12-F-PS** resins) were employed to replace the usual Universal-CPG carrier. Synthesis initiated from the solid support, with nucleotides sequentially added in the 3'→5' direction using commercially available phosphoramidite monomers (2'-F RNA, 2'-OMe RNA; Shanghai Hongene Biotechnology Co., Ltd.). Upon completion of solid-phase synthesis, the solid support was transferred to a centrifuge tube and incubated with 28% $NH_4OH$ in ethanol/$H_2O$ (3:1 v/v) at 50°C for 16 hours to cleave the oligonucleotide from the solid support into solution. The supernatant was centrifuged and transferred to a new tube, concentrated to dryness, and redissolved in deionized water. Purification was performed using a C18 reversed-phase column with a mobile phase of 0.1 M TEAA and acetonitrile. The target oligonucleotide fraction was collected, lyophilized, identified as the desired product by LC-MS, and quantified by UV spectroscopy (260 nm). The obtained sense and antisense strands were annealed at an equimolar ratio to form the complementary double-stranded siRNA (dsRNA), which was subsequently adjusted to the desired concentration.

### Huh7 Transfection

On day 1, a mixture of RNAiMAX transfection reagent and Opti-MEM was prepared as required according to the ratio (RNAiMAX transfection reagent:Opti-MEM=1.5:48.5), followed by incubation at room temperature for 15 minutes. For each well of cells, a certain amount of the diluted compound was added to an equal volume of the RNAiMAX-Opti-MEM mixture, mixed well, and incubated at room temperature for 15 minutes. Huh7 cells were washed with DPBS, digested with

trypsin, and adjusted to a density of 2×$10^5$ cells/mL. During cell seeding, 20 μL of the mixture of Opti-MEM RNAiMAX and the compound was added to the cell culture plate. The cells were then seeded into a 96-well plate at a density of 20,000 cells per well (100 μL/well), with a final culture volume of 120 μL per well. The cells were cultured in a 5% $CO_2$ incubator at 37 °C for 24 hours.

**PHH Free Uptake**

PHH cells were thawed and adjusted to a density of 6×$10^5$ cells/mL. Then, 10 μL of the compound from the dilution plate was added to the cell culture plate, resulting in final compound concentrations of 100, 40, 12.5, 5, and 2 nM. The cells were seeded into a 96-well plate at a density of 54,000 cells per well (90 μL/well), with a final culture volume of 100 μL per well. The cells were cultured in a 5% $CO_2$ incubator at 37°C for 72 hours.

**PCH Free Uptake**

PCH cells were resuscitated using 10% FBS InvitroGRO™ HT Medium, and subsequently, the cell density was adjusted to 3×$10^5$ cells/mL using 10% FBS InvitroGRO™ CP Medium. Then, 10 μL of the compound from the dilution plate was added to the cell culture plate, resulting in final compound concentrations of 25, 5, and 1 nM. The cells were seeded into a 96-well plate at a density of 27,000 cells per well (90 μL/well), with a final culture volume of 100 μL per well. The cells were cultured in a 5% $CO_2$ incubator at 37 °C for 72 hours.

**RNA Extraction and Reverse Transcription**

According to the instructions of the RNA extraction kit (Qiagen, 74182), RNA was extracted from the Huh7 cells obtained in the previous steps. Subsequently, referring to the instructions of the FastKing cDNA First Strand Synthesis Kit (TianGen, KR116-02), the extracted RNA was reverse transcribed into cDNA.

**QPCR Detection and Data Analysis**

qPCR was used to detect the target gene cDNA. The qPCR reaction system preparation is shown in Table 6. GAPDH was used as the internal reference gene. qPCR was performed in 384-well plates. The qPCR reaction program was: 95 °C heating for 10 minutes, then entering cycle mode: 95 °C for 15 seconds, then 60 °C for 1 minute, for a total of 40 cycles.

**Table 6. RT-PCR Reaction System**

| **Component** | **Concentration** | **Volume (μL)/well** |
|---|---|---|
| FastStart Universal Probe Mast | 2× | 5 |
| ANGPTL3/GAPDH TaqMan® Assays and Arrays (60×) | 60× | 0.17 |
| RNase-free Water | / | 2.83 |
| Total | / | 8 |
| **Component** | **Concentration** | **Volume (μL)/well** |
| FastStart Universal Probe Mast | 2× | 5 |
| GAPDH forward primer | 10 μM | 0.4 |
| GAPDH reverse primer | 10 μM | 0.4 |
| GAPDH probe | 10 μM | 0.2 |
| RNase-free Water | / | 2 |
| Total | / | 8 |

Data Analysis: ΔΔCT method (Comparative CT method). This method requires the introduction of an internal reference gene, GAPDH, because the internal reference gene is expressed in all cells, its product is essential for

maintaining cell survival, and its expression level or genomic copy number is constant in cells, less affected by the environment. After qRT-PCR, the CT value of the internal reference is also recorded, referred to as Ct (GAPDH), and the CT value of the sample is referred to as Ct (sample).

$$\Delta Ct\ (sample) = Ct\ (sample) - Ct\ (GAPDH)$$

$$\Delta Ct\ (control) = Ct\ (control) - Ct\ (GAPDH)$$

$$\Delta\Delta Ct = \Delta Ct\ (sample) - \Delta Ct\ (control)$$

$$\text{Relative gene expression} = 2\text{^-}\Delta\Delta Ct$$

Inhibition % = (1 - Relative expression of sample / Average relative expression of control group) * 100

**ELISA Detection and Data Analysis**

The supernatant from the PHH and PCH cultures from the previous steps was collected, transferred to a new 96-well V-bottom plate, and centrifuged. Human Angiopoietin-like 3 protein (hANGPTL3 protein; DANL30) ELISA test was performed according to the instructions of HANGPTL 3 protein Kit.

**Data Analysis:** Four Parameter Logistic method. This method constructs a standard curve by plotting, with the y-axis as the average absorbance of the standards and the x-axis as the concentration, drawing a best-fit curve through the points on the graph. The OD value of the log concentration of the sample's HANGPTL3 protein is determined against the best-fit line of the log concentration by regression analysis.

Inhibition % = (Average content of control group - Sample content) / Average content of control group * 100

**Example 1. Study on Hepatic Cellular Uptake Activity of Poly-GalNAc Ligands with Macrocyclic Scaffold Structure**

Flow cytometry was applied to evaluate the uptake efficiency of FAM-labeled test articles in HepG2 cells.

The human hepatocellular carcinoma cell line (HepG2) was purchased from ATCC (USA). Cells were cultured in DMEM medium (Gibco, ThermoFisher Scientific, USA) containing 10% fetal bovine serum (ExCell Bio, South America), 100 units/mL penicillin, and 100 units/mL streptomycin, placed in a 37 °C, 5% $CO_2$ incubator.

On day 0, the HepG2 cell suspension was adjusted to an appropriate density (1.5E+05/wells) and cells were seeded into a 48-well plate. On day 1, the diluted fluorescently labeled test articles were added. The test articles were diluted to different concentrations: 1.6, 8, 40, 200, and 1000 nM. The GalNAc ligand used for Givosiran was the positive control, and 5FAM was the negative control. After incubating the test articles with cells for 4 hours or 24 hours, all liquid was removed, cells were washed twice with PBS, and then cells were collected. After digesting and fixing cells, the corresponding fluorescence in HepG2 cells was detected by flow cytometry (BD FACSCanto™ II, Becton Dickinson, America). The mean fluorescence intensity (MFI) of the test compounds' FAM was analyzed relative to the MFI of the positive compound. Table 7 shows data at the 4-hour time point, and Table 8 shows data at the 24-hour time point.

Table 7. Ratio of MFI for Hepatic Cellular Uptake of GalNAc Ligands to MFI of Positive Compound (FAM Fluorescence Channel, 4-hour time point)

| | 1000 nM | 200 nM | 40 nM | 8 nM |
|---|---|---|---|---|
| ID | MFI Ratio | | | |
| I-A10-5FAM | 2.16 | 2.86 | 2.86 | 3.28 |
| I-A11-5FAM | 1.69 | 2.68 | 2.78 | 2.19 |
| I-A12-5FAM | 2.00 | 3.22 | 3.25 | 3.09 |
| I-A13-5FAM | 0.667 | 0.552 | 0.525 | 0.438 |
| I-B11-5FAM | 1.84 | 2.62 | 3.14 | 3.41 |
| I-B12-5FAM | 1.97 | 2.98 | 3.68 | 3.53 |

(continued)

| | 1000 nM | 200 nM | 40 nM | 8 nM |
|---|---|---|---|---|
| ID | MFI Ratio | | | |
| 1-C10-5FAM | 0.603477 | 0.507752 | 0.496403 | - |

Table 8. Ratio of MFI for Hepatic Cellular Uptake of GalNAc Ligands to MFI of Positive Compound (FAM Fluorescence Channel, 24-hour time point)

| | 1000 nM | 200 nM | 40 nM | 8 nM |
|---|---|---|---|---|
| ID | MFI Ratio | | | |
| I-A10-5FAM | 2.24 | 3.65 | 2.92 | 2.01 |
| I-A11-5FAM | 1.72 | 5.08 | 3.45 | 3.14 |
| I-A12-5FAM | 3.65 | 6.44 | 4.24 | 4.18 |
| I-A13-5FAM | 1.41 | 1.76 | 0.834 | 0.810 |
| I-B11-5FAM | 2.46 | 4.20 | 3.77 | 3.44 |
| I-B12-5FAM | 2.86 | 6.49 | 4.85 | 4.88 |
| I-C10-5FAM | 1.16 | 0.770 | 0.645 | 0.416 |
| Negative control | 0.0036 | 0.0053 | - | - |

**Example 2. Screening of Modified siRNA Sequences**

To evaluate the in vitro inhibitory activity of modified siRNAs against ANGPTL3 mRNA in the Huh7 cell line, the designed siRNAs were functionally evaluated in vitro. Results are shown in Table 9.

**Table 9. In Vitro Inhibitory Activity of Modified siRNAs against** ANGPTL3 mRNA in **Huh7 Cell Line**

| **Compound ID** | **Average Inhibition in Huh7 %** | **SD** |
|---|---|---|
| | 0.5nM | 0.5nM |
| 2002-004 | 81.39 | 0.29 |
| 2002-005 | 76.16 | 1.90 |
| 2002-009 | 86.27 | *0.55* |
| 2002-010 | 83.95 | 1.79 |
| 2002-011 | 79.93 | 1.28 |
| 2002-006 | 62.46 | 2.06 |
| 2002-007 | 71.27 | 1.61 |
| 2002-008 | 66.19 | 2.09 |
| 2002-046 | 81.51 | 0.57 |
| 2002-047 | 81.66 | 0.69 |
| 2002-048 | 85.20 | 0.40 |
| 2002-049 | 78.49 | 2.17 |
| 2008-004 | 78.58 | 5.00 |
| 2008-005 | 77.36 | 1.24 |
| 2008-009 | 87.96 | 0.97 |
| 2008-010 | 89.12 | 0.60 |
| 2008-011 | 88.66 | 0.78 |

(continued)

| Compound ID | Average Inhibition in Huh7 % | SD |
|---|---|---|
| | 0.5nM | 0.5nM |
| 2008-006 | 74.14 | 3.26 |
| 2008-007 | 67.11 | 0.43 |
| 2008-008 | 73.04 | 0.83 |
| 2008-046 | 86.57 | 3.24 |
| 2008-047 | 77.47 | 2.03 |
| 2008-048 | 81.95 | 0.57 |
| 2008-049 | 78.75 | 0.92 |
| 2002-050 | 88.28 | 0.97 |
| 2008-050 | 86.48 | 1.22 |

The results indicate that the siRNAs of the present invention can effectively reduce the ANGPTL3 mRNA level in Huh7 cells, and some modification schemes are comparable to Alnylam Advanced ESC (**2002-050, 2008-050,** see Foster DJ, Brown CR, Shaikh S, et al. Advanced siRNA Designs Further Improve In Vivo Performance of GalNAc-siRNA Conjugates. Mol Ther. 2018;26(3):708-717. doi:10.1016/j.ymthe.2017.12.021).

**Example 3. MCPP-siRNA Screening**

Sequences with good activity in Table 8 were evaluated for in vitro inhibitory activity against ANGPTL3 Protein in primary human hepatocytes (PHH) and primary cynomolgus monkey hepatocytes (PCH). Results are shown in Table 10 and Table 11.

**Table 10. In Vitro Inhibitory Activity of MCPP-siRNAs against ANGPTL3 mRNA in PHH Cell Line**

| Compound ID | IC50 of ANGPTL3 (nM) in PHH |
|---|---|
| 2002-033-002 | 0.84 |
| 2002-034-002 | 1.30 |
| 2002-018-002 | 0.96 |
| 2008-010-002 | 0.98 |
| 2008-011-002 | 0.75 |
| 2008-018-002 | 0.96 |
| 2008-019-002 | 1.11 |
| 2008-035-002 | 2.23 |
| 2002-033-001 | 1.08 |
| 2002-034-001 | 1.38 |
| 2002-018-001 | 1.90 |
| 2008-010-001 | 0.81 |
| 2008-011-001 | 2.60 |
| 2008-018-001 | 2.34 |
| 2008-019-001 | 1.61 |
| 2008-035-001 | 2.34 |
| 2002-050-L96 | 2.60 |
| ARO-05488-003-NAG37 | 7.19 |

The results indicate that the MCPP-siRNAs of the present invention can effectively reduce the ANGPTL3 protein level in PHH cells. Some modification schemes are comparable to L96 (**2002-050-L96**).

**Table 11. In Vitro Inhibitory Activity of MCPP-siRNAs against ANGPTL3 mRNA in PCH Cell Line**

| Compound ID | Average Inhibition in PCH % | | | SD | | |
|---|---|---|---|---|---|---|
| | 25nM | 5nM | 1nM | 25nM | 5nM | 1nM |
| 2002-034-002 | 63.01 | 60.18 | 45.42 | 5.56 | 5.08 | 7.86 |
| 2008-011-002 | 76.57 | 71.25 | 54.65 | 2.18 | 3.70 | 6.87 |
| 2008-019-002 | 73.74 | 70.28 | 52.37 | 3.23 | 3.80 | 2.46 |
| 2002-033-001 | 68.80 | 67.45 | 53.11 | 1.29 | 0.31 | 1.22 |
| 2002-034-001 | 66.66 | 63.43 | 50.50 | 0.41 | 1.14 | 1.53 |
| 2008-010-001 | 78.39 | 74.73 | 62.75 | 0.08 | 0.05 | 0.90 |
| 2008-011-001 | 77.81 | 72.91 | 57.51 | 1.15 | 0.22 | 1.21 |
| 2008-018-001 | 76.55 | 73.29 | 59.56 | 0.55 | 0.12 | 0.84 |
| 2008-019-001 | 75.51 | 73.01 | 58.78 | 2.95 | 3.46 | 5.10 |
| 2008-035-001 | 68.96 | 63.23 | 46.03 | 3.57 | 4.91 | 10.06 |
| ARO-05488-003-NAG37 | 60.00 | 49.83 | 28.48 | 4.88 | 6.49 | 9.99 |

The results indicate that the MCPP-siRNAs of the present invention can effectively reduce the ANGPTL3 protein level in PCH cells.

**Example 4. In Vivo Efficacy of ANGPTL3 GalNAc-siRNAs in hANGPTL3 Humanized Mice**

To evaluate the in vivo efficacy of siRNA against ANGPTL3 in mice, female ANGPTL3 humanized mice (purchased from Shanghai Model Organisms Center, Inc.) were used for in vivo efficacy testing. Mice were randomly divided into 5 groups, with 5 mice per group: G1, Control (PBS) negative control group; G2, 2002-034-001 (3 mg/kg) treatment group; G3, 2008-010-001 (3 mg/kg) treatment group; G4, 2008-019-001 (3 mg/kg) treatment group; and G5, 2008-019-002 (3 mg/kg) treatment group.

On days -7 and -1, pre-dose plasma samples were obtained after a 4-hour fast. The above siRNA conjugates were diluted with saline and administered subcutaneously to the mice according to body weight on day 0. Plasma was collected after a 4-hour fast on days 7, 14, 21, and 28, respectively. The ANGPTL3 protein level in plasma was detected by ELISA (R&D Systems). Plasma triglycerides (TG), total cholesterol (CHO), high-density lipoprotein cholesterol (HDL-c), low-density lipoprotein cholesterol (LDL-c), alanine aminotransferase (ALT), and aspartate aminotransferase (AST) were tested by Shanghai Bikai Keyi Biotechnology Co., Ltd.

To evaluate the in vivo efficacy of the siRNAs against ANGPTL3 in mice, female ANGPTL3 humanized mice (purchased from Shanghai Model Organisms Center, Inc.) were used for the efficacy study. The mice were randomly allocated into five groups (n=5 per group) as follows:

G1: Control (PBS) negative control group;
G2: 2002-034-001 (3 mg/kg) treatment group;
G3: 2008-010-001 (3 mg/kg) treatment group;
G4: 2008-019-001 (3 mg/kg) treatment group;
G5: 2008-019-002 (3 mg/kg) treatment group.

The day of the first administration was designated as day 0. Accordingly, the day before administration was designated as day -1, and the day after administration as day 1.

On day -7, all mice were fasted for 4 hours, and mandibular blood was collected to obtain plasma for hANGPTL3 protein detection. Plasma preparation method: Whole blood was collected into heparin sodium-coated anticoagulant tubes, centrifuged at 4°C, 7000 g, for 10 minutes, and the supernatant was taken. 20 μL was transferred to an external laboratory for ELISA detection of plasma hANGPTL3 protein expression level (R&D Systems), and another 50 μL was sent to Shanghai Bikai Keyi Biotechnology for blood biochemical indicator detection of triglyceride (TG) level and total cholesterol (CHO). Based on the test results of the plasma samples and the body weight of the mice on day -7, 25 mice

were randomly divided into 5 groups.

On day -1, pre-dose plasma samples were obtained after a 4-hour fast. The above siRNA conjugates were diluted with saline and administered subcutaneously to the mice according to body weight on day 0. Plasma was collected after a 4-hour fast on days 7, 14, 21, and 28, respectively. The ANGPTL3 protein level in plasma was detected by ELISA (R&D Systems). Plasma triglycerides (TG) and total cholesterol (CHO) were tested by Shanghai Bikai Keyi Biotechnology Co., Ltd.

The plasma ANGPTL3 protein, triglyceride (TG) level, and total cholesterol (CHO) levels on day -1 in humanized mice were normalized. The data processing method for other time points was as follows: at a given time point, the plasma ANGPTL3 protein, triglyceride, and total cholesterol levels of each mouse were divided by the expression level of the corresponding mouse on day -1 before administration.

The results are shown in Fig. 1, indicating that the tested siRNA conjugate of 2008-010 significantly reduced the levels of ANGPTL3 protein, triglycerides (TG), and total cholesterol (CHO) in the plasma of humanized mice compared to the control.

**Example 5. In Vivo Efficacy of ANGPTL3 GalNAc-siRNA in Hyperlipidemic Cynomolgus Monkeys**

To evaluate the in vivo activity of siRNA against ANGPTL3, 6 HFD-induced hyperlipidemic cynomolgus monkeys were used for the experiment. Pre-dose serum samples were obtained after a 4-hour fast on days -7 and -14. The siRNA conjugate was diluted with saline and administered subcutaneously according to the experimental design on day 0. Blank saline was used as a negative control. Plasma was collected after a 4-hour fast on days 0, 7, 14, 28, 42, 49, and 56. Serum triglycerides were detected using an automatic biochemical analyzer.

The ANGPTL3 protein levels and triglyceride levels for each animal were normalized. For normalization, at a given time point, the ANGPTL3 protein and triglyceride levels of each animal were divided by the pre-treatment expression level of that animal (the average of the pre-experiment samples for that group) to determine the change in "normalization to pre-treatment".

The results are shown in Fig. 2. A single subcutaneous injection of YH2008-010-001 in cynomolgus monkeys significantly inhibited the serum ANGPTL3 protein level and significantly reduced triglyceride level in HFD-induced hyperlipidemic aged cynomolgus monkeys.

**Claims**

1. An RNAi agent comprising a sense strand and an antisense strand that form a duplex region, wherein the RNAi agent inhibits expression of ANGPTL3 in a cell, and wherein the antisense strand is no more than 23 nucleotides in length and comprises a nucleotide sequence selected from any one of SEQ ID NOs: 13, 14, 27, and 28.

2. The RNAi agent according to claim 1, wherein the sense strand is no more than 21 nucleotides in length and comprises a nucleotide sequence selected from any one of SEQ ID NOs: 5, 7, 8, 19, 20, and 22.

3. The RNAi agent according to claim 1 or 2, wherein:

   (a1) the sense strand comprises or consists of a sequence set forth in SEQ ID NO: 5, and the antisense strand comprises or consists of a sequence set forth in SEQ ID NO: 13;
   (a2) the sense strand comprises or consists of a sequence set forth in SEQ ID NO: 5, and the antisense strand comprises or consists of a sequence set forth in SEQ ID NO: 14;
   (a3) the sense strand comprises or consists of a sequence set forth in SEQ ID NO: 7, and the antisense strand comprises or consists of a sequence set forth in SEQ ID NO: 13;
   (a4) the sense strand comprises or consists of a sequence set forth in SEQ ID NO: 8, and the antisense strand comprises or consists of a sequence set forth in SEQ ID NO: 13;
   (a5) the sense strand comprises or consists of a sequence set forth in SEQ ID NO: 19, and the antisense strand comprises or consists of a sequence set forth in SEQ ID NO: 27;
   (a6) the sense strand comprises or consists of a sequence set forth in SEQ ID NO: 19, and the antisense strand comprises or consists of a sequence set forth in SEQ ID NO: 28;
   (a7) the sense strand comprises or consists of a sequence set forth in SEQ ID NO: 20, and the antisense strand comprises or consists of a sequence set forth in SEQ ID NO: 27;
   (a8) the sense strand comprises or consists of a sequence set forth in SEQ ID NO: 20, and the antisense strand comprises or consists of a sequence set forth in SEQ ID NO: 28;
   (a9) the sense strand comprises or consists of a sequence set forth in SEQ ID NO: 22, and the antisense strand

comprises or consists of a sequence set forth in SEQ ID NO: 28;

(a10) the sense strand comprises or consists of a sequence set forth in SEQ ID NO: 5, and the antisense strand comprises or consists of a sequence set forth in SEQ ID NO: 18; or

(a11) the sense strand comprises or consists of a sequence set forth in SEQ ID NO: 19, and the antisense strand comprises or consists of a sequence set forth in SEQ ID NO: 32.

**4.** The RNAi agent according to any one of claims 1 to 3, wherein the RNAi agent further comprises a ligand that targets a hepatocyte; preferably, the ligand comprises a galactose moiety, a galactosamine moiety, or an N-acetylgalactosamine moiety; more preferably, the ligand is a trivalent or tetravalent N-acetylgalactosamine moiety.

**5.** The RNAi agent according to claim 4, wherein the ligand comprises a structure of formula (I):

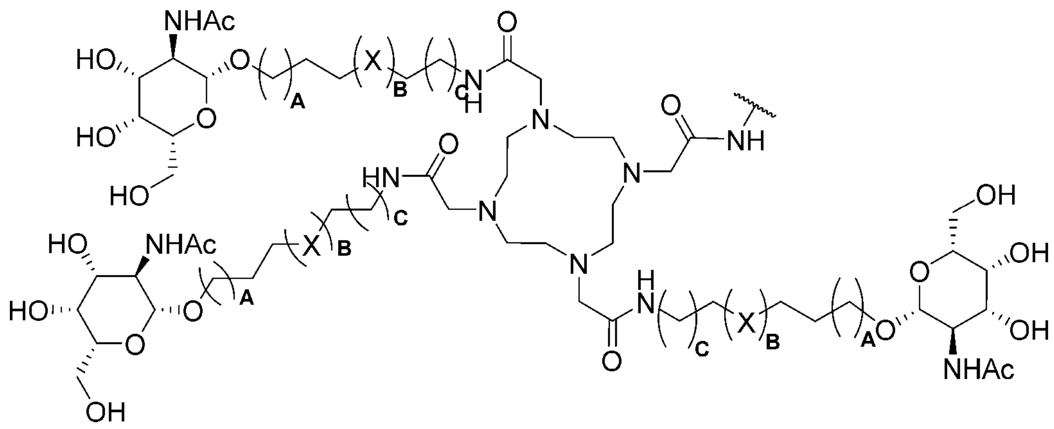

(I)

wherein,

X is -C(O)-NH-, -NH-C(O)-, $-OCH_2-CH_2O-$, -S-, or -S-S-;
A or C is independently 0 or an integer from 1 to 5;
B is 0 or an integer from 1 to 3; and
the wave line represents a site to which other part of the RNAi agent is attached.

**6.** The RNAi agent according to claim 4, wherein the ligand has a structure of formula (II) or formula (III):

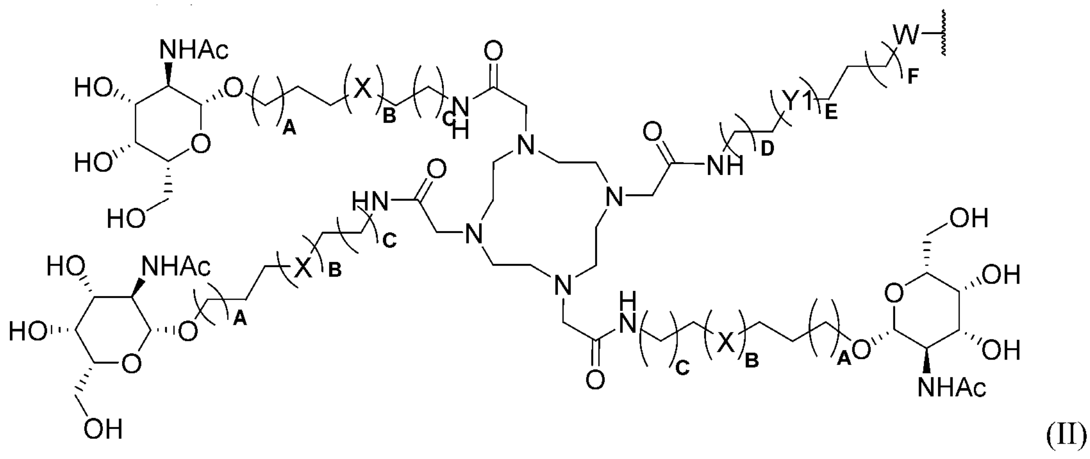

(II)

wherein,

X is -C(O)-NH-, -NH-C(O)-, $-OCH_2-CH_2O-$, -S-, or -S-S-;
Y1 is $-C(O)-NR_1-$, -NH-C(O)-, $-OCH_2-CH_2O-$, -S-S-,

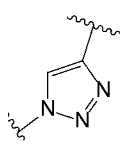 or 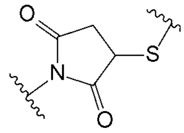 ,

wherein $R_1$ is an alkyl group having 1-6 carbons or hydrogen;
W is -NH-, -O- or

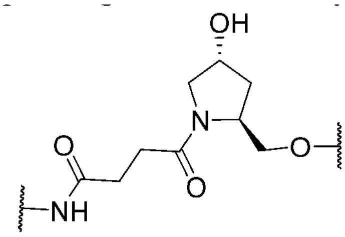 ,

wherein the wave line on the right represents a site to which the sense strand or antisense strand is attached; the wave line connected to W in formula (II) represents a site to which the sense strand or antisense strand is attached, wherein the ligand is connected to 5'- and/or 3'-end of the sense strand and/or antisense strand;
A, C, or F is 0 or an integer from 1 to 5;
B or E is 0 or an integer from 1 to 3;
D is an integer from 1 to 6;

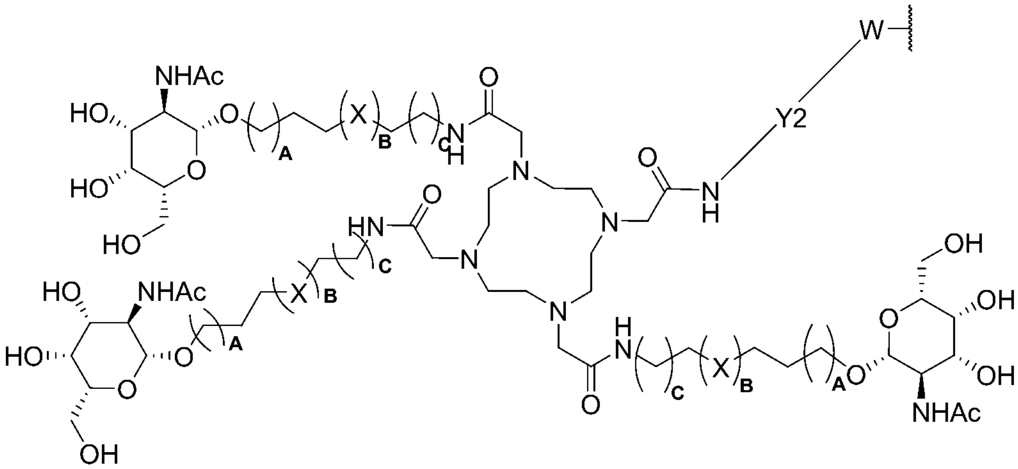

(III)

wherein,
X is -C(O)-NH-, -NH-C(O)-, $-OCH_2-CH_2O-$, -S-, or -S-S-;
Y2 is $-(CH_Z)p-(O-CH_2-CH_2)q-(CH_2)j-Z_3-$ , wherein $Z_3$ is O, NH or C(O), p is an integer from 1 to 3, q is an integer from 3 to 10; j is 0 or 1;
W is absent, or is

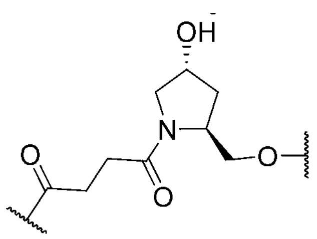 or 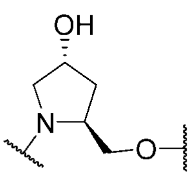 ,

wherein the wave line on the right represents a site to which the sense strand or antisense strand is attached; the wave line connected to W in formula (III) represents a site to which the sense strand or antisense strand is

attached, wherein the ligand is connected to 5'- and/or 3'-end of the sense strand and/or antisense strand;
A or C is 0 or an integer from 1 to 5;
B is 0 or an integer from 1 to 3.

**7.** The RNAi agent according to claim 5 or 6, wherein the ligand has a structure of formula (IV) or formula (V):

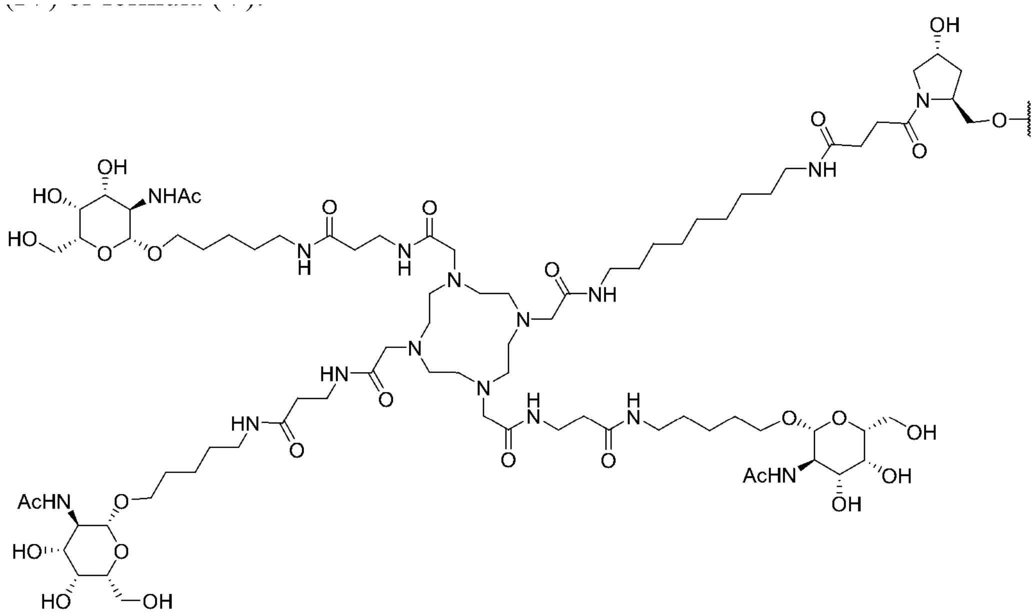

(IV)

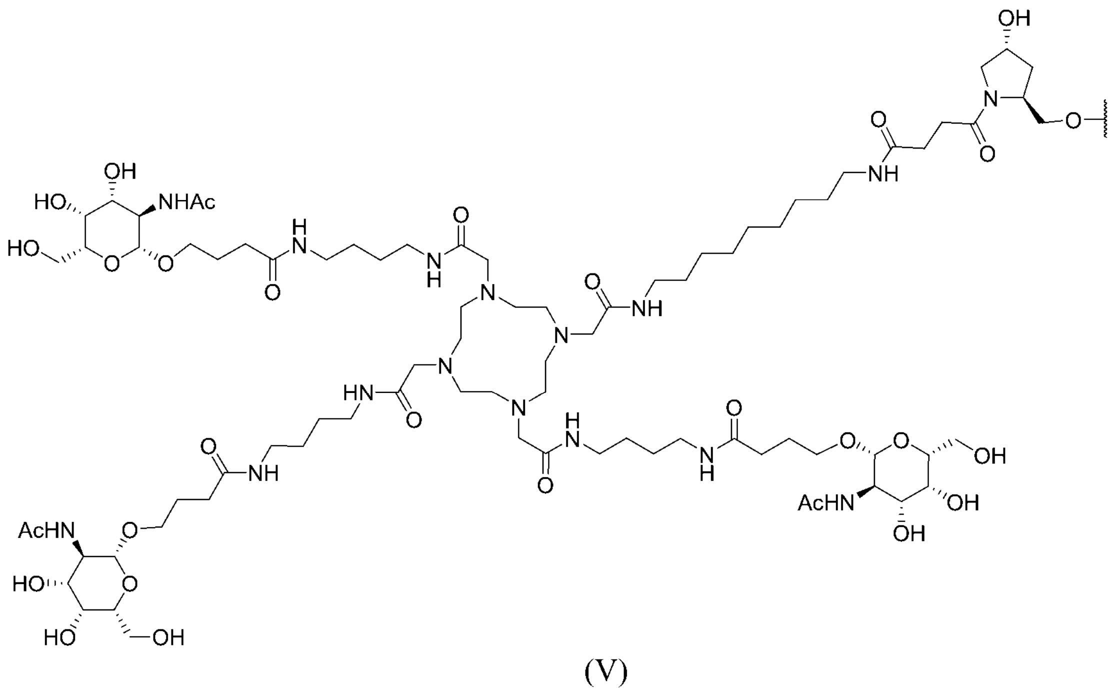

(V)

wherein the wave line represents a site to which the sense strand or antisense strand is attached, and the ligand is connected to 5'- and/or 3'-end of the sense strand; preferably, the ligand is connected to 3'-end of the sense strand; preferably, wherein the ligand is connected to the sense strand or antisense strand via a phosphate ester group, a thiophosphate ester group or a phosphonate ester group.

**8.** The RNAi agent according to any one of claims 5 to 7, wherein:

(b1) the sense strand comprises or consists of a sequence set forth in SEQ ID NO: 33, and the antisense strand comprises or consists of a sequence set forth in SEQ ID NO: 13;
(b2) the sense strand comprises or consists of a sequence set forth in SEQ ID NO: 34, and the antisense strand comprises or consists of a sequence set forth in SEQ ID NO: 13;
(b3) the sense strand comprises or consists of a sequence set forth in SEQ ID NO: 38, and the antisense strand comprises or consists of a sequence set forth in SEQ ID NO: 13;
(b4) the sense strand comprises or consists of a sequence set forth in SEQ ID NO: 39, and the antisense strand comprises or consists of a sequence set forth in SEQ ID NO: 13;
(b5) the sense strand comprises or consists of a sequence set forth in SEQ ID NO: 35, and the antisense strand comprises or consists of a sequence set forth in SEQ ID NO: 27;
(b6) the sense strand comprises or consists of a sequence set forth in SEQ ID NO: 36, and the antisense strand comprises or consists of a sequence set forth in SEQ ID NO: 27;
(b7) the sense strand comprises or consists of a sequence set forth in SEQ ID NO: 40, and the antisense strand comprises or consists of a sequence set forth in SEQ ID NO: 27;
(b8) the sense strand comprises or consists of a sequence set forth in SEQ ID NO: 41, and the antisense strand comprises or consists of a sequence set forth in SEQ ID NO: 27;
(b9) the sense strand comprises or consists of a sequence set forth in SEQ ID NO: 35, and the antisense strand comprises or consists of a sequence set forth in SEQ ID NO: 28;
(b10) the sense strand comprises or consists of a sequence set forth in SEQ ID NO: 36, and the antisense strand comprises or consists of a sequence set forth in SEQ ID NO: 28;
(b11) the sense strand comprises or consists of a sequence set forth in SEQ ID NO: 37, and the antisense strand comprises or consists of a sequence set forth in SEQ ID NO: 28;
(b12) the sense strand comprises or consists of a sequence set forth in SEQ ID NO: 40, and the antisense strand comprises or consists of a sequence set forth in SEQ ID NO: 28;
(b13) the sense strand comprises or consists of a sequence set forth in SEQ ID NO: 41, and the antisense strand comprises or consists of a sequence set forth in SEQ ID NO: 28; or
(b14) the sense strand comprises or consists of a sequence set forth in SEQ ID NO: 42, and the antisense strand comprises or consists of a sequence set forth in SEQ ID NO: 28.

**9.** A double-stranded RNAi agent for inhibiting expression of ANGPTL3 in a cell, wherein the double-stranded RNAi agent comprises a sense strand and an antisense strand that form a duplex region, wherein the sense strand comprises SEQ ID NO: 19: 5'-mG*mA*mGmAfAmGfAfAfCmUmAmCmAmUmAmUmAmAmA-3' and the sense strand is no more than 21 nucleotides in length, the antisense strand comprises SEQ ID NO: 27: 5'-mU*fU*mUmAfUmAfUmGmUmAmGmUmUfCmUfUmCmUmC*mA*mG-3' and is no more than 23 nucleotides in length,

wherein mA, mU, mG, and mC are 2'-OMe A, 2'-OMe U, 2'-OMe G, and 2'-OMe C, respectively; fA, fU, fG, and fC are 2'-F A, 2'-F U, 2'-F G, and 2'-F C, respectively; * is a phosphorothioate linkage;
wherein 3'-end of the sense strand is conjugated to a ligand that targets a hepatocyte; preferably, the ligand comprises a galactose moiety, a galactosamine moiety, or an N-acetylgalactosamine moiety; more preferably, the ligand is a trivalent or tetravalent N-acetylgalactosamine moiety.

**10.** The RNAi agent according to claim 9, wherein the ligand has a structure of formula (IV):

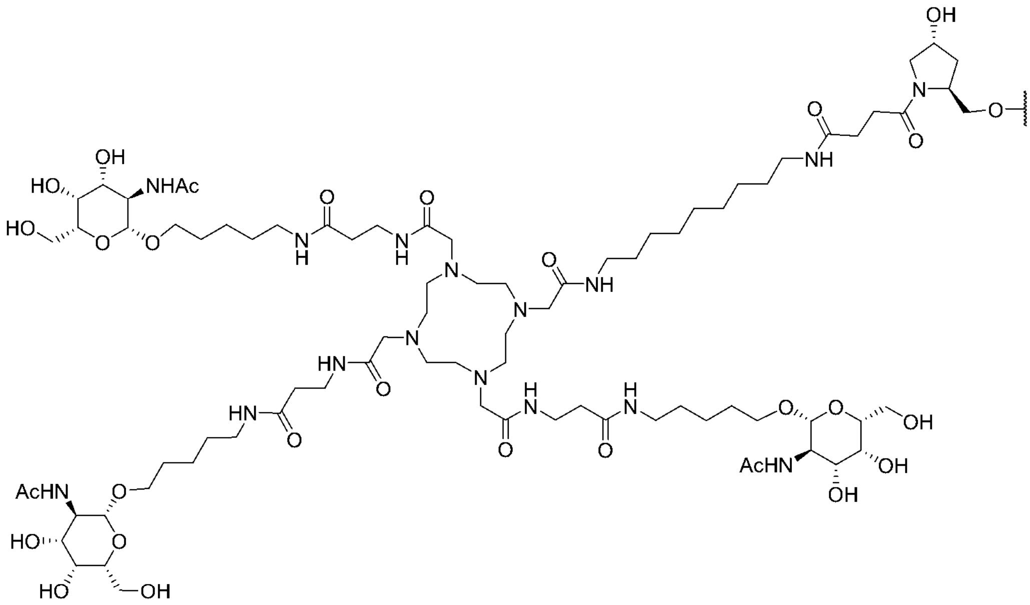

(IV),

and is connected to the sense strand via a phosphate ester group or a thiophosphate ester group.

**11.** The RNAi agent according to claim 9 or 10, wherein the sense strand comprises SEQ ID NO: 40: 5'-mG*mA*mGmAfAmGfAfAfCmUmAmCmAmUmAmUmAmAmA-YHZY12001-3' and the sense strand is no more than 21 nucleotides in length, the antisense strand comprises SEQ ID NO: 27: 5'-mU*fU*mUmAfUmAfUmGmUmAmGmUmUfCmUfUmCmUmC*mA*mG-3' and is no more than 23 nucleotides in length,

wherein mA, mU, mG, and mC are 2'-OMe A, 2'-OMe U, 2'-OMe G, and 2'-OMe C, respectively; fA, fU, fG, and fC are 2'-F A, 2'-F U, 2'-F G, and 2'-F C, respectively; * is a phosphorothioate linkage,
wherein YHZY12001 has a structure of formula (IV) and is conjugated to 3'-end of the sense strand as follows:

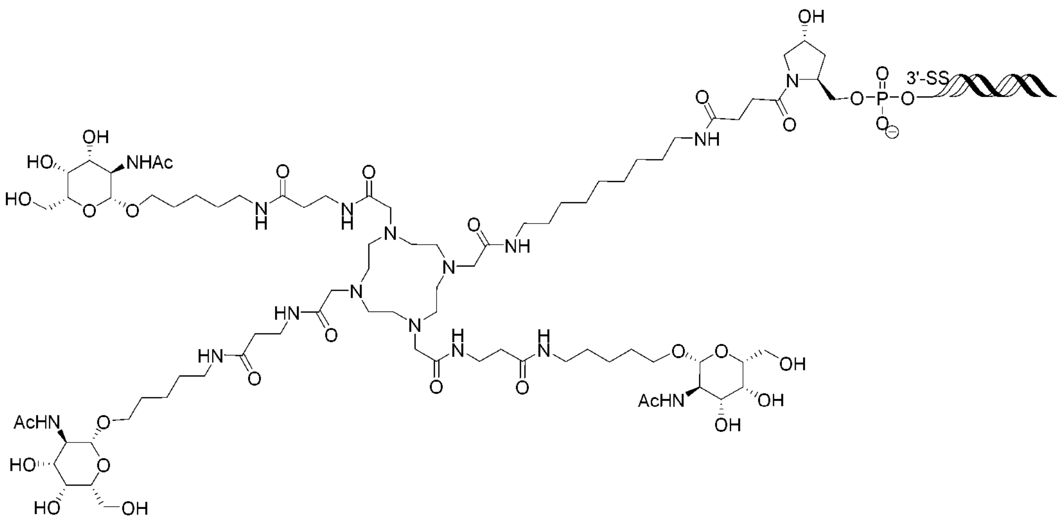

.

**12.** A pharmaceutical composition comprising the RNAi agent according to any one of claims 1 to 11 and a pharmaceutically acceptable carrier; preferably, the pharmaceutical composition is formulated for intravenous or subcutaneous injection.

**13.** Use of the RNAi agent according to any one of claims 1 to 11 in the manufacture of a medicament for:

(i) reducing expression level of ANGPTL3 in a cell;
(ii) preventing or treating a disease caused by an elevated ANGPTL3 expression level; or

(iii) preventing or treating a metabolic disease, wherein the metabolic disease is, for example, hypertriglyceridemia, obesity, hyperlipidemia, abnormal lipid and/or cholesterol metabolism, atherosclerosis, Type 2 diabetes mellitus, cardiovascular disease, coronary artery disease, non-alcoholic steatohepatitis, non-alcoholic fatty liver disease, homozygous or heterozygous familial hypercholesterolemia, statin-resistant hypercholesterolemia.

**14.** An RNAi agent comprising a sense strand and an antisense strand that form a duplex region, wherein:

in 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and
in 5' to 3' direction, the nucleotides at positions 7 and 9 of the sense strand are 2'-fluoro-modified nucleotides, and one or two of the nucleotides at positions 5, 8, and 11 of the sense strand are 2'-fluoro-modified nucleotides, while the nucleotides at remaining positions of the sense strand are all 2'-methoxy-modified nucleotides.

**15.** The RNAi agent according to claim 14, wherein the antisense strand comprises at least one phosphorothioate internucleotide linkage; preferably, the phosphorothioate internucleotide linkage is present at one or more of the following locations:

(i) between nucleotide positions 1 and 2 at 5'-end of the antisense strand;
(ii) between nucleotide positions 2 and 3 at 5'-end of the antisense strand;
(iii) between nucleotide positions 1 and 2 at 3'-end of the antisense strand; and
(iv) between nucleotide positions 2 and 3 at 3'-end of the antisense strand.

**16.** The RNAi agent according to claim 15, wherein the sense strand comprises at least one phosphorothioate internucleotide linkage; preferably, the phosphorothioate internucleotide linkage is present (i) between nucleotide positions 1 and 2 at 5'-end of the sense strand; and/or (ii) between nucleotide positions 2 and 3 at 5'-end of the sense strand.

**17.** The RNAi agent according to any one of claims 14 to 16, wherein the duplex region is 17 to 23 base pairs in length, preferably 18 to 21 base pairs, more preferably 19 base pairs.

**18.** The RNAi agent according to any one of claims 14 to 17, wherein each of the sense strand and the antisense strand is 17 to 23 nucleotides in length, preferably 19 to 21 nucleotides.

**19.** The RNAi agent according to any one of claims 14 to 18, wherein the RNAi agent comprises one or two blunt ends, preferably one blunt end.

**20.** The RNAi agent according to any one of claims 14 to 19, wherein the RNAi agent comprises one or two overhangs, preferably one overhang, each overhang comprising 1 to 4 unpaired nucleotides, preferably 2 unpaired nucleotides.

**21.** The RNAi agent according to claim 20, wherein the overhang is located at 3'-end of the sense strand, 3'-end of the antisense strand, or simultaneously at 3'-end of the sense strand and 3'-end of the antisense strand; preferably, the overhang is located at 3'-end of the antisense strand, and more preferably, the RNAi agent has one blunt end.

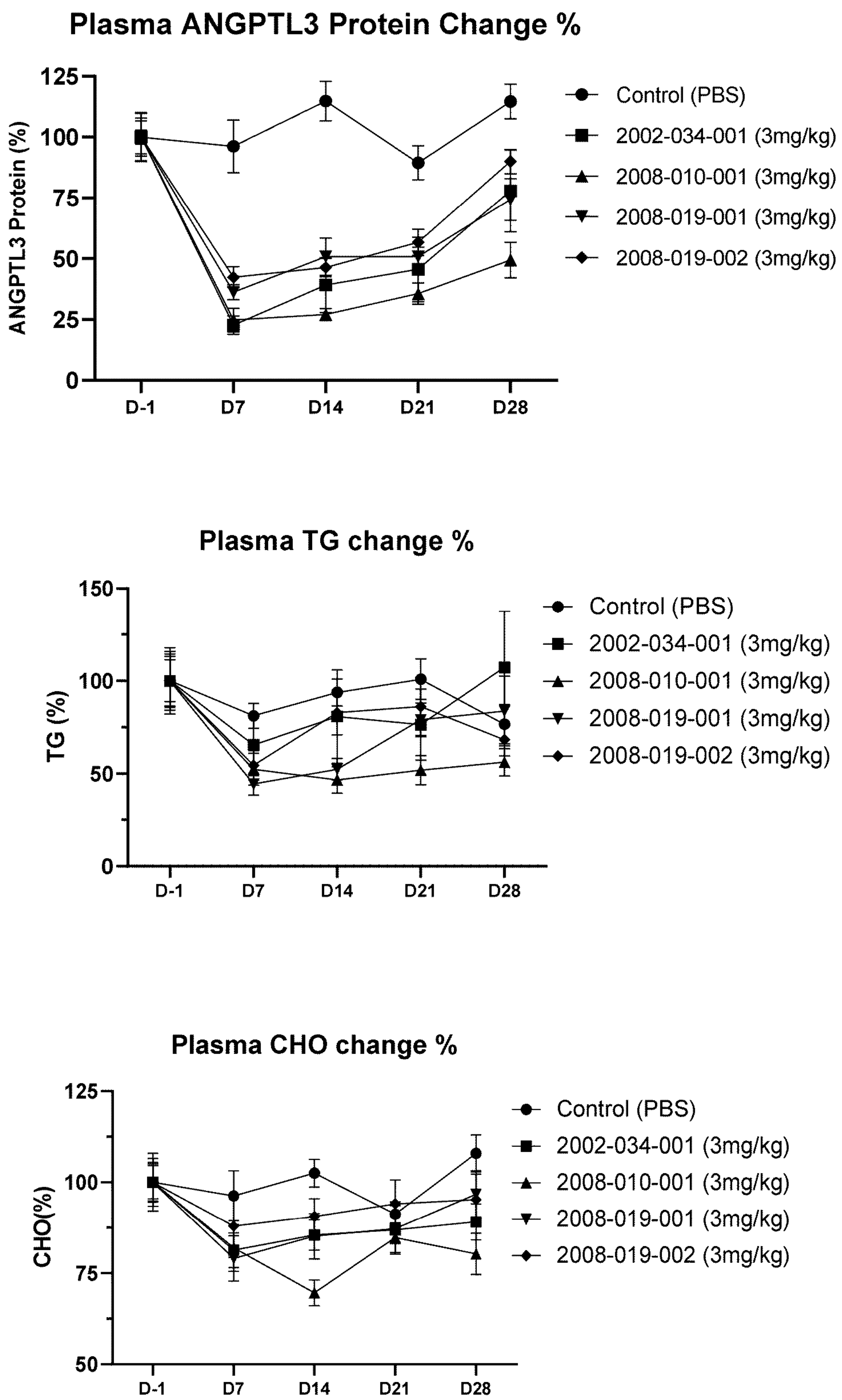
Plasma ANGPTL3 Protein Change %
ANGPTL3 Protein (%)
125
100
75
50
25
0
D-1
D7
D14
D21
D28
Control (PBS)
2002-034-001 (3mg/kg)
2008-010-001 (3mg/kg)
2008-019-001 (3mg/kg)
2008-019-002 (3mg/kg)
Plasma TG change %
TG (%)
150
100
50
0
D-1
D7
D14
D21
D28
Control (PBS)
2002-034-001 (3mg/kg)
2008-010-001 (3mg/kg)
2008-019-001 (3mg/kg)
2008-019-002 (3mg/kg)
Plasma CHO change %
CHO(%)
125
100
75
50
D-1
D7
D14
D21
D28
Control (PBS)
2002-034-001 (3mg/kg)
2008-010-001 (3mg/kg)
2008-019-001 (3mg/kg)
2008-019-002 (3mg/kg)

FIG. 1

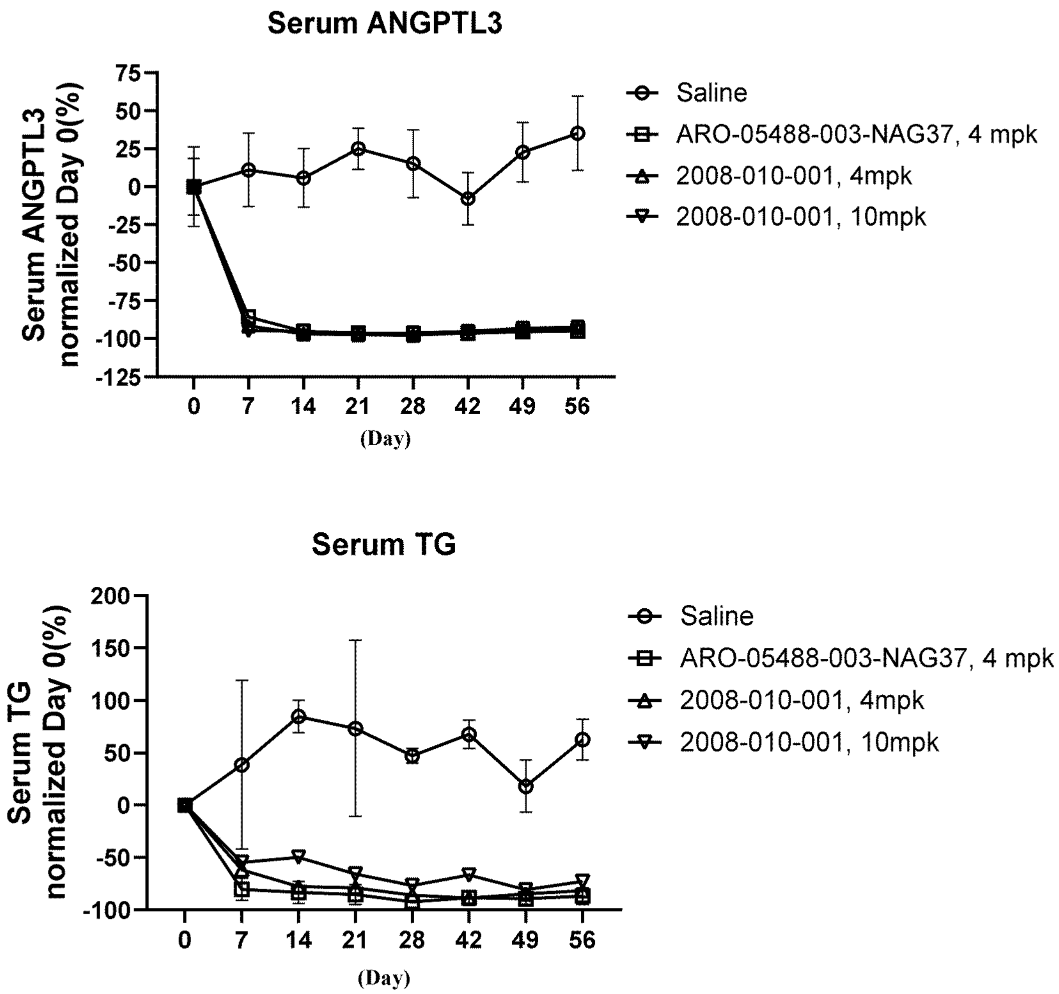
Serum ANGPTL3
Serum ANGPTL3 normalized Day 0(%)
75
50
25
0
-25
-50
-75
-100
-125
0 7 14 21 28 42 49 56
(Day)
Saline
ARO-05488-003-NAG37, 4 mpk
2008-010-001, 4mpk
2008-010-001, 10mpk
Serum TG
Serum TG normalized Day 0(%)
200
150
100
50
0
-50
-100
0 7 14 21 28 42 49 56
(Day)
Saline
ARO-05488-003-NAG37, 4 mpk
2008-010-001, 4mpk
2008-010-001, 10mpk

FIG. 2

## INTERNATIONAL SEARCH REPORT

International application No.
**PCT/CN2024/094888**

**A. CLASSIFICATION OF SUBJECT MATTER**

C12N15/113(2010.01)i； A61K47/54(2017.01)i； A61P19/06(2006.01)i； A61K31/713(2006.01)i； C07D211/58(2006.01)i； C07C229/16(2006.01)i； C07D211/28(2006.01)i

According to International Patent Classification (IPC) or to both national classification and IPC

**B. FIELDS SEARCHED**

Minimum documentation searched (classification system followed by classification symbols)

IPC:C12N,A61K,A61P,C07D,C07C

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, JPTXT, USTXT, EPTXT, 万方, WANFANG, CNKI, PubMed, GenBank, ISI web of knowledge, 中国专利序列数据库, China Patents Sequence Databas: 血管生成素样蛋白3, RNAi剂, 有义链, 反义链, 抑制, 表达, 2'-氟代修饰, 2'-甲氧基修饰, 硫代磷酸酯键, 平末端, angiopoietin-like 3, ANGPTL3, RNAi agent, sense strand, antisense strand, inhibition, expression, 2 '-fluoro modification, 2'-methoxy modification, phosphorothioate linkage, blunt end

**C. DOCUMENTS CONSIDERED TO BE RELEVANT**

| Category* | Citation of document, with indication, where appropriate, of the relevant passages | Relevant to claim No. |
|---|---|---|
| X | CN 111343994 A (ARROWHEAD PHARMACEUTICALS, INC.) 26 June 2020 (2020-06-26)<br>claims 1-60, and description, paragraphs 42-54 | 14-21 |
| A | CN 115572726 A (SHANGHAI JUNSHI BIOSCIENCES CO., LTD. et al.) 06 January 2023 (2023-01-06)<br>description, paragraphs 6-15 | 1-21 |
| A | CN 113614230 A (SUZHOU RIBO LIFE SCIENCE CO., LTD.) 05 November 2021 (2021-11-05)<br>claims 1-40, and description, paragraphs 197-202 | 1-21 |
| A | US 2022049252 A1 (AMGEN INC.) 17 February 2022 (2022-02-17)<br>claims 1-71 | 1-21 |
| A | US 2020108153 A1 (NOVARTIS AG) 09 April 2020 (2020-04-09)<br>claims 68-92 | 1-21 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 August 2024** | **16 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

INTERNATIONAL SEARCH REPORT

International application No.
**PCT/CN2024/094888**

**C. DOCUMENTS CONSIDERED TO BE RELEVANT**

| Category* | Citation of document, with indication, where appropriate, of the relevant passages | Relevant to claim No. |
|---|---|---|
| A | CN 115572725 A (SHANGHAI JUNSHI BIOSCIENCES CO., LTD. et al.) 06 January 2023 (2023-01-06) claims 1-27, and description, paragraphs 22-35 | 1-21 |
| A | CN 108368506 A (ARROWHEAD PHARMACEUTICALS, INC.) 03 August 2018 (2018-08-03) claims 1-31 | 1-21 |

Form PCT/ISA/210 (second sheet) (July 2022)

**INTERNATIONAL SEARCH REPORT**

International application No.
**PCT/CN2024/094888**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/094888**

| Patent document cited in search report | Publication date (day/month/year) | Patent family member(s) | Publication date (day/month/year) |
|---|---|---|---|
| CN 111343994 A | 26 June 2020 | BR 112020003126 A2 | 13 October 2020 |
| | | PH 12020500309 A1 | 25 January 2021 |
| | | JP 2023054368 A | 13 April 2023 |
| | | KR 20200044837 A | 29 April 2020 |
| | | TN 2020000038 A1 | 04 October 2021 |
| | | US 2021238602 A1 | 05 August 2021 |
| | | US 2019078089 A1 | 14 March 2019 |
| | | US 10995335 B2 | 04 May 2021 |
| | | ZA 202103669 B | 29 March 2023 |
| | | AU 2018331383 A1 | 23 April 2020 |
| | | SG 11201912188 RA | 30 January 2020 |
| | | JOP 20200050 A1 | 05 March 2020 |
| | | PE 20201287 A1 | 24 November 2020 |
| | | JP 2020534268 A | 26 November 2020 |
| | | JP 7281452 B2 | 25 May 2023 |
| | | EP 3681513 A1 | 22 July 2020 |
| | | EP 3681513 A4 | 22 September 2021 |
| | | WO 2019055633 A1 | 21 March 2019 |
| | | IL 273173 A | 30 April 2020 |
| | | CO 2020001740 A2 | 29 May 2020 |
| | | TW 201922264 A | 16 June 2019 |
| | | TWI 823866 B | 01 December 2023 |
| | | CA 3074320 A1 | 21 March 2019 |
| | | CL 2020000666 A1 | 14 August 2020 |
| | | MX 2020001912 A | 24 March 2020 |
| | | UY 37881 A | 29 March 2019 |
| | | ECSP 20018305 A | 30 June 2020 |
| CN 115572726 A | 06 January 2023 | None | |
| CN 113614230 A | 05 November 2021 | EP 3974533 A1 | 30 March 2022 |
| | | EP 3974533 A4 | 08 November 2023 |
| | | US 2024200076 A1 | 20 June 2024 |
| | | JP 2022533419 A | 22 July 2022 |
| | | TW 202111119 A | 16 March 2021 |
| | | WO 2020233651 A1 | 26 November 2020 |
| US 2022049252 A1 | 17 February 2022 | EA 202191630 A1 | 29 November 2021 |
| | | KR 20210102313 A | 19 August 2021 |
| | | AU 2019395341 A1 | 17 June 2021 |
| | | WO 2020123410 A1 | 18 June 2020 |
| | | EP 3894554 A1 | 20 October 2021 |
| | | IL 283550 A | 29 July 2021 |
| | | JP 2022511866 A | 01 February 2022 |
| | | CL 2021001490 A1 | 14 January 2022 |
| | | SG 11202105900 UA | 29 July 2021 |
| | | CA 3122393 A1 | 18 June 2020 |
| | | BR 112021011043 A2 | 31 August 2021 |
| | | MX 2021006745 A | 15 July 2021 |
| US 2020108153 A1 | 09 April 2020 | PE 20211646 A1 | 24 August 2021 |
| | | KR 20210066860 A | 07 June 2021 |
| | | WO 2020070675 A1 | 09 April 2020 |
| | | TW 202027794 A | 01 August 2020 |

Form PCT/ISA/210 (patent family annex) (July 2022)

**INTERNATIONAL SEARCH REPORT**
**Information on patent family members**

International application No.
**PCT/CN2024/094888**

| Patent document cited in search report | Publication date (day/month/year) | Patent family member(s) | Publication date (day/month/year) |
|---|---|---|---|
| | | PH 12021550675 A1 | 03 January 2022 |
| | | MX 2021003839 A | 27 May 2021 |
| | | JOP 20210061 A1 | 30 January 2023 |
| | | CR 20210156 A | 28 April 2021 |
| | | AR 116566 A1 | 19 May 2021 |
| | | CL 2021000819 A1 | 12 November 2021 |
| | | EP 3860657 A1 | 11 August 2021 |
| | | JP 2023145478 A | 11 October 2023 |
| | | CO 2021004012 A2 | 19 April 2021 |
| | | DOP 2021000053 A | 30 April 2021 |
| | | BR 112021005981 A2 | 29 June 2021 |
| | | CA 3112251 A1 | 09 April 2020 |
| | | EA 202190650 A1 | 21 July 2021 |
| | | US 2022323595 A1 | 13 October 2022 |
| | | CU 20210022 A7 | 12 October 2021 |
| | | JP 2022501406 A | 06 January 2022 |
| | | JP 7336513 B2 | 31 August 2023 |
| | | IL 281227 A | 29 April 2021 |
| | | ZA 202101419 B | 27 July 2022 |
| | | AU 2019353174 A1 | 01 April 2021 |
| | | SG 11202102076 TA | 29 April 2021 |
| | | ECSP 21022120 A | 29 April 2021 |
| | | US 11389541 B2 | 19 July 2022 |
| CN 115572725 A | 06 January 2023 | CA 3224904 A1 | 29 December 2022 |
| | | WO 2022268054 A1 | 29 December 2022 |
| | | JP 2024523458 A | 28 June 2024 |
| | | AU 2022298603 A1 | 01 February 2024 |
| | | EP 4361266 A1 | 01 May 2024 |
| | | KR 20240023635 A | 22 February 2024 |
| | | BR 112023026971 A2 | 12 March 2024 |
| CN 108368506 A | 03 August 2018 | MX 2018003833 A | 18 June 2018 |
| | | HK 1259063 A1 | 22 November 2019 |
| | | MY 195796 A | 21 February 2023 |
| | | TN 2018000094 A1 | 08 July 2019 |
| | | HUE 055942 T2 | 28 January 2022 |
| | | UY 36926 A | 28 April 2017 |
| | | ZA 202106265 B | 30 August 2023 |
| | | CY 1125263 T1 | 24 March 2023 |
| | | CA 3000397 A1 | 06 April 2017 |
| | | EA 201890864 A1 | 28 September 2018 |
| | | EA 038478 B1 | 03 September 2021 |
| | | IL 300438 A | 01 April 2023 |
| | | TW 202332769 A | 16 August 2023 |
| | | TWI 836693 B | 21 March 2024 |
| | | PE 20181139 A1 | 17 July 2018 |
| | | CR 20180231 A | 31 May 2018 |
| | | ES 2896298 T3 | 24 February 2022 |
| | | IL 258333 A | 31 May 2018 |
| | | IL 258333 B | 01 March 2022 |
| | | US 2020263179 A1 | 20 August 2020 |

Form PCT/ISA/210 (patent family annex) (July 2022)

**INTERNATIONAL SEARCH REPORT**
**Information on patent family members**

International application No.
**PCT/CN2024/094888**

| Patent document cited in search report | Publication date (day/month/year) | Patent family member(s) | | | Publication date (day/month/year) |
|---|---|---|---|---|---|
| | | WO | 2017059223 | A2 | 06 April 2017 |
| | | WO | 2017059223 | A3 | 11 May 2017 |
| | | WO | 2017059223 | A9 | 17 August 2017 |
| | | HRP | 20211410 | T1 | 24 December 2021 |
| | | EP | 3356529 | A2 | 08 August 2018 |
| | | EP | 3356529 | A4 | 04 September 2019 |
| | | EP | 3356529 | B1 | 25 August 2021 |
| | | SG | 10202008530 | TA | 29 October 2020 |
| | | EP | 4029941 | A1 | 20 July 2022 |
| | | IL | 290566 | A | 01 April 2022 |
| | | IL | 290566 | B1 | 01 March 2023 |
| | | IL | 290566 | B2 | 01 July 2023 |
| | | RS | 62523 | B1 | 30 November 2021 |
| | | US | 2017096665 | A1 | 06 April 2017 |
| | | US | 9932586 | B2 | 03 April 2018 |
| | | US | 2018195070 | A1 | 12 July 2018 |
| | | US | 10662427 | B2 | 26 May 2020 |
| | | KR | 20180052703 | A | 18 May 2018 |
| | | LT | 3356529 | T | 27 December 2021 |
| | | TW | 201726918 | A | 01 August 2017 |
| | | TWI | 784934 | B | 01 December 2022 |
| | | MA | 43347 | A | 08 August 2018 |
| | | MA | 43347 | B1 | 30 November 2021 |
| | | JP | 2021087459 | A | 10 June 2021 |
| | | JP | 7116212 | B2 | 09 August 2022 |
| | | UA | 121998 | C2 | 25 August 2020 |
| | | BR | 112018006489 | A2 | 09 October 2018 |
| | | CO | 2018003678 | A2 | 30 November 2018 |
| | | JP | 2018529732 | A | 11 October 2018 |
| | | JP | 6991966 | B2 | 03 February 2022 |
| | | MX | 2022013010 | A | 09 November 2022 |
| | | PH | 12018500713 | A1 | 15 October 2018 |
| | | JP | 2024009262 | A | 19 January 2024 |
| | | AU | 2022283623 | A1 | 02 February 2023 |
| | | SI | 3356529 | T1 | 31 January 2022 |
| | | JP | 2022113835 | A | 04 August 2022 |
| | | JP | 7442574 | B2 | 04 March 2024 |
| | | DK | 3356529 | T3 | 08 November 2021 |
| | | JOP | 20210043 | A1 | 16 June 2017 |
| | | PL | 3356529 | T3 | 20 December 2021 |
| | | AU | 2016331084 | A1 | 19 April 2018 |
| | | AU | 2016331084 | B2 | 08 September 2022 |
| | | PT | 3356529 | T | 04 November 2021 |
| | | NZ | 741086 | A | 24 November 2023 |
| | | JOP | 20160211 | B1 | 17 August 2021 |
| | | CL | 2018000803 | A1 | 31 August 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017058944 A
- US 7491805 B
- US 8106022 B
- US 8877917 B
- US 20030130186 A
- WO 2013166155 A
- US 7723509 B
- US 8017762 B
- US 8828956 B
- US 9181551 B
- WO 2021037205 A
- WO 2015042447 A1
- US 20120035115 A
- WO 02021049504 A1
- WO 2021049504 A1
- WO 2020093053 A


### Non-patent literature cited in the description


- *J. Org. Chem.*, 2020, vol. 85 (10), 6593-6604
- *J. Med. Chem.*, 2016, vol. 59 (6), 2718-2733
- **FOSTER DJ** ; **BROWN CR** ; **SHAIKH S et al.** Advanced siRNA Designs Further Improve In Vivo Performance of GalNAc-siRNA Conjugates.. *Mol Ther.*, 2018, vol. 26 (3), 708-717